(19) Europäisches Patentamt / European Patent Office / Office européen des brevets

(11) **EP 3 821 848 A1**

(12) **EUROPEAN PATENT APPLICATION**

(43) Date of publication:
19.05.2021   Bulletin 2021/20

(51) Int Cl.:
*A61F 2/00* (2006.01)   *A61B 17/12* (2006.01)
*A61F 7/02* (2006.01)   *A61N 1/05* (2006.01)
*A61N 1/36* (2006.01)

(21) Application number: 20211627.3

(22) Date of filing: 10.10.2008

(84) Designated Contracting States:
AT BE BG CH CY CZ DE DK EE ES FI FR GB GR HR HU IE IS IT LI LT LU LV MC MT NL NO PL PT RO SE SI SK TR

(30) Priority: 11.10.2007   US 960715 P

(62) Document number(s) of the earlier application(s) in accordance with Art. 76 EPC:
08838038.1 / 2 211 771

(71) Applicant: **Implantica Patent Ltd.**
**223 70 Lund (SE)**

(72) Inventor: **Forsell, Peter**
**223 70 Lund (SE)**

Remarks:
This application was filed on 03-12-2020 as a divisional application to the application mentioned under INID code 62.

(54) **APPARATUS FOR THE TREATMENT OF GALLSTONES**

(57)   An apparatus for influencing a flow in the biliary duct is disclosed. The apparatus comprises an implantable stimulation device for stimulating muscle tissue of a wall portion of the biliary duct of the patient, comprising an electrical stimulation element configured to engage the muscle tissue, and a control device for controlling said stimulation device to transmit electrical pulses to the muscle tissue causing contraction of the muscle tissue

Fig.1C

**EP 3 821 848 A1**

**Description**

FIELD OF THE INVENTION

The present invention relates to treatment of gallstones

BACKGROUND OF THE INVENTION

Cholelithiasis (gallstones) is the presence or formation of gallstones in the biliary tract. It can cause intense pain and is potentially dangerous. It is a common medical problem, affecting 10 to 15 percent of the population.

Bile is formed in the gallbladder and consists of water, cholesterol, fats, bile salts, proteins, and bilirubin. The main function is to secrete bile salts that emulsify dietary fats and to secrete bilirubin, which is a waste product. Bile is produced by hepatocytes of the liver and transported to the gall bladder were it is stored for release into the duodenum. Bile is transported through a system of ducts. The ducts include the hepatic ducts, which carry bile out of the liver, the cystic duct, which takes bile to and from the gallbladder, and the common bile duct, which takes bile from the cystic and hepatic ducts to the small intestine. These three ducts together with the sphincters that control them are referred to herein as the "biliary duct" or the "biliary ducts". Biliary ducts has smooth muscle tissue, that enables the ducts to contract.

Gallstones may form when cholesterol or bilirubin precipitates into hard aggregates. Gallstones can block the normal flow of bile if they move from the gallbladder and lodge in any of the ducts that carry bile from the liver to the small intestine Symptoms of blocked bile ducts include intense pain and are often referred to as a gallbladder "attack" because they occur suddenly. If any of the biliary ducts remain blocked by gallstones for a significant period of time, severe damage or infection can occur in the gallbladder, liver, or pancreas. Left untreated, the condition can be fatal.

The most common treatment for gallstones is the complete removal of the gallbladder (cholecystectomy). Present data suggest that the gallbladder is a nonessential organ and that patient can live a normal life without the gallbladder, as bile can instead reach the intestines via direct flow from the liver through the hepatic ducts into the common bile duct and into the small intestine, instead of being stored in the gallbladder.

Removal of the call bladder is usually performed using laparascopical procedures. However, open surgery is necessary in about 5 percent of gallbladder operations. Recovery from open surgery usually requires 3 to 5 days in the hospital and several weeks at home.

A serious disadvantage with the current treatment is the riskt for serious damage to the the bile duct during surgery. This is a serious problem and requries additional surgery.

Another disadvantage is that a high percentage of patients suffer from diahorrea permanently or for a long time after removal of the gall bladder.

BRIEF SUMMARY OF THE INVENTION

The object of the present invention is to provide an apparatus for enhancing the movement of gallstones from the bile ducts to the duodenum, so as to at least substantially or even completely eliminate the blockage and pain associated with gallstones. One major purpose of the invention is to enable the transport of gallstones to the duodenum as this relieves the symptoms. Gallstones that reach the duodenum are secreted together with faeces.

Herein is also disclosed how flow in the biliary duct can be restricted by simultaneously using a restriction device and stimulation of the tissue of the wall of the biliary duct. It may be of importance to be able to completely close the biliary duct. One such occasion is when surgery on the biliary duct is to be performed.

Furthermore, the restriction device will be of use when a gallstone is lodged and cannot move downstream. It is then possible to use the desribed restriction device in order to release the lodged gallstone.

In accordance with this object of the present invention, there is provided an apparatus for enhancing the movement of gallstones in a patient suffering from gallstone trouble, the apparatus comprises an implantable stimulation device for stimulating a portion of the tissue wall of the biliary ducts, and a control device for controlling said stimulation device to progressively stimulate the tissue wall portion to cause progressive contraction of the tissue wall portion to move one or more gallstones in the biliary duct in the direction towards the duodenum.

In addition, there is provided an apparatus for controlling the flow of bile and/or gallstones in biliary ducts, the apparatus comprising an implantable constriction device for gently constricting a portion of the tissue wall to influence the movement of bile and/or gallstones in the biliary duct, a stimulation device for stimulating the wall portion of the tissue wall, and a control device for controlling the stimulation device to stimulate the wall portion as the constriction device constricts the wall portion to cause contraction of the wall portion to further influence the movement of bile and/or gallstones in the biliary duct.

The present invention provides an advantageous combination of constriction and stimulation devices, which results in a two-stage influence on the movement of gallstones in the lumen of the biliary ducts. Thus, the constriction device may gently constrict the tissue wall by applying a relatively weak force against the wall portion, and the stimulation device may stimulate the constricted wall portion to achieve the desired final influence on the movement of bile and/or gallstones in the biliary duct. The phrase "gently constricting a portion of the tissue wall" is to be understood as constricting the wall portion without substantially hampering the blood circulation in the tissue wall.

Preferably, the stimulation device is adapted to stimulate different areas of the wall portion as the constriction device constricts the wall portion, and the control device controls the stimulation device to intermittently and individually stimulate the areas of the wall portion. This intermittent and individual stimulation of different areas of the wall portion of the biliary duct allows tissue of the wall portion to maintain substantially normal blood circulation during the operation of the apparatus of the invention.

It will be advantageous to be able to calibrate the apparatus. Therefore, in a preferred embodiment of the invention, the constriction device is adjustable to enable adjustment of the constriction of the wall portion as desired, wherein the control device controls the constriction device to adjust the constriction of the wall portion. The control device may control the constriction and stimulation devices independently of each other, and simultaneously. Optionally, the control device may control the stimulation device to stimulate, or to not stimulate the wall portion while the control device controls the constriction device to change the constriction of the wall portion.

Initially, the constriction device may be calibrated by using the control device to control the stimulation device to stimulate the wall portion, while controlling the constriction device to adjust the constriction of the biliary ducts until the desired restriction of the movement of bile and/or gallstones in the biliary duct is obtained.

The apparatus can be placed so that it can stimulate movement of gallstones in any of the bilary ducts.

Flow restriction

As mentioned above, there may be of importance to be able to restrict flow of bile or even completely close the biliary duct. For example when surgery on the biliary duct is to be performed.The apparatus of the present invention is well suited for such a situation. Thus, in a principal embodiment of the invention, the constriction device is adapted to constrict the wall portion to at least restrict the movement of bile and/or gallstones in the biliary duct, and the control device controls the stimulation device to cause contraction of the constricted wall portion, so that the movement of bile and/or gallstones in the biliary duct is at least further restricted. Specifically, the constriction device is adapted to constrict the wall portion to a constricted state in which the blood circulation in the constricted wall portion is substantially unrestricted and the movement of bile and/or gallstones in the biliary duct is at least restricted, and the control device controls the stimulation device to cause contraction of the wall portion, so that the movement of bile and/or gallstones in the biliary duct is at least further restricted when the wall portion is kept by the constriction device in the constricted state.

The constriction and stimulation devices may be controlled to constrict and stimulate, respectively, to an extent that depends on the flow restriction that is desired to be achieved in a specific application of the apparatus of the invention. Thus, in accordance with a first flow restriction option, the control device controls the constriction device to constrict the wall portion, such that movement of bile and/or gallstones in the biliary duct is restricted but not stopped, and controls the stimulation device to stimulate the constricted wall portion to cause contraction thereof, such that movement of bile and/or gallstones in the biliary duct is further restricted but not stopped. More precisely, the control device may control the stimulation device in a first mode to stimulate the constricted wall portion to further restrict but not stop the movement of bile and/or gallstones in the biliary duct and to:

a) control the stimulation device in a second mode to cease the stimulation of the wall portion to increase the movement of bile and/or gallstones in the biliary duct; or
b) control the stimulation and constriction devices in the second mode to cease the stimulation of the wall portion and release the wall portion to restore the movement of bile and/or gallstones in the biliary duct.

In accordance with a second flow restriction option, the control device controls the constriction device to constrict the wall portion, such that movement of bile and/or gallstones in the biliary duct is restricted but not stopped, and controls the stimulation device to stimulate the constricted wall portion to cause contraction thereof, such that movement of bile and/or gallstones in the biliary duct is stopped. More precisely, the control device may control the stimulation device in a first mode to stimulate the constricted wall portion to further restrict but not stop the movement of bile and/or gallstones in the biliary duct and to:

a) control the stimulation device in a second mode to cease the stimulation of the wall portion to allow movement of bile and/or gallstones in the biliary duct; or
b) control the stimulation and constriction devices in the second mode to cease the stimulation of the wall portion and release the wall portion to restore the movement of bile and/or gallstones in the biliary duct.

In accordance with a third flow restriction option, the control device controls the constriction device to constrict the wall portion, such that the movement of bile and/or gallstones in the biliary duct is substantially stopped, and controls the stimulation device to stimulate the constricted wall portion to cause contraction thereof, such that the movement of bile and/or gallstones in the biliary duct is completely stopped. More precisely, the control device may control the stimulation device in a first mode to stimulate the constricted wall portion to completely stop the movement of bile and/or gallstones in the biliary duct and to:

a) control the stimulation device in a second mode to cease the stimulation of the wall portion to allow movement of bile and/or gallstones in the biliary duct; or

b) control the stimulation and constriction devices in the second mode to cease the stimulation of the wall portion and release the wall portion to restore the movement of bile and/or gallstones in the biliary duct.

Where the stimulation device stimulates the constricted wall portion to contract, such that the movement of bile and/or gallstones in the biliary duct is stopped, the control device suitably controls the stimulation device to simultaneously and cyclically stimulate a first length of the constricted wall portion and a second length of the constricted wall portion, which is located downstream of the first length, wherein the control device controls the stimulation device to progressively stimulate the first length in the upstream direction of the lumen and to progressively stimulate the second length in the downstream direction of the lumen.

The control device may control the stimulation device to change the stimulation of the wall portion in response to a sensed physical parameter of the patient or functional parameter of the apparatus. For example, the control device may control the stimulation device to increase the intensity of the stimulation of the wall portion in response to a sensed pressure increase in the lumen, such that the movement of bile and/or gallstones in the biliary duct remains stopped. Any sensor for sensing a physical parameter of the patient, such as a pressure in the patient's body that relates to the pressure in the lumen may be provided, wherein the control device controls the stimulation device in response to signals from the sensor. Such a sensor may for example sense the pressure in the patient's abdomen, the pressure against the implanted constriction device or the pressure on the tissue wall of the biliary duct.

In accordance with a fourth flow restriction option, the control device controls the constriction device to constrict the wall portion, such that the movement of bile and/or gallstones in the biliary duct is stopped. More precisely, the control device may control the constriction device in a first mode to constrict the constricted wall portion to stop the movement of bile and/or gallstones in the biliary duct and in a second mode to cease the constriction of the wall portion to restore movement of bile and/or gallstones in the biliary duct. In this case, the control device only controls the stimulation device to stimulate the wall portion when needed. A sensor for sensing a physical parameter of the patient's body that relates to the pressure in the lumen may be provided, wherein the control device controls the stimulation device in response to signals from the sensor. Such a physical parameter may be a pressure in the patient's abdomen and the sensor may be a pressure sensor.

In some applications of the invention, the implanted constriction device may be designed to normally keep the patient's wall portion of the biliary duct in the constricted state. In this case, the control device may be used when needed, conveniently by the patient, to control the stimulation device to stimulate the constricted tissue wall portion, preferably while adjusting the stimulation intensity, to cause contraction of the wall portion, such that the movement of bile and/or gallstones in the biliary duct is at least further restricted or stopped, and to control the stimulation device to cease the stimulation. More precisely, the control device may:

a) control the stimulation device in a first mode to stimulate the constricted wall portion to further restrict the movement of bile and/or gallstones in the biliary duct, and control the stimulation device in a second mode to cease the stimulation of the wall portion to increase the movement of bile and/or gallstones in the biliary duct; or

b) control the stimulation device in a first mode to stimulate the constricted wall portion to stop the movement of bile and/or gallstones in the biliary duct, and control the stimulation device in a second mode to cease the stimulation of the wall portion to allow movement of bile and/or gallstones in the biliary duct.

Either the first mode or the second mode may be temporary.

The constriction device may include a plurality of separate constriction elements adapted to constrict any wall portions of a series of wall portions of the biliary duct's tissue wall, respectively. The control device may control the constriction device to activate the constriction elements in random or in accordance with a predetermined sequence. In this case, the stimulation device includes stimulation elements positioned on the constriction elements, wherein the control device controls the stimulation device to activate the stimulation elements to stimulate any wall portions of the series of wall portions constricted by said constriction elements to contract the biliary duct to close the biliary duct's lumen.

Alternatively, the control device controls the constriction device to activate the constriction elements to constrict all of the wall portions of the series of wall portions, and controls the stimulation device to activate the stimulation elements to stimulate any constricted wall portions in random or in accordance with a predetermined sequence to close the biliary duct. The design of the constriction device in the form of a plurality of separate constriction elements makes possible to counteract growth of hard fibrosis where the constriction device is implanted.

Movement of bile and/or gallstones in the bilary duct

Importantly, the apparatus of the invention is specifically suited for actively moving gallstones in the biliary ducts, as described in the embodiments of the invention listed below.

1) The control device controls the constriction device to close the biliary ducts, at an upstream end of the wall portion, and then controls the constriction device to constrict the remaining part of the wall portion to move the gallstones in the biliary duct.
1a) In accordance with a first alternative of the above noted embodiment (1), the control device controls the stimulation device to stimulate the wall portion as the constriction device constricts the remaining part of the wall portion.
1b) In accordance with a second alternative, the constriction device is adapted to constrict the wall portion to restrict but not stop the flow in the biliary duct. The control device controls the stimulation device to stimulate the wall portion constricted by the constriction device to close the biliary duct, either at an upstream end or a downstream end of the wall portion, and simultaneously controls the constriction device to increase the constriction of the wall portion to move the gallstones in the biliary duct.
2) The constriction device is adapted to constrict the wall portion to restrict or vary the movement of bile and/or gallstones in the biliary duct, and the control device controls the stimulation device to progressively stimulate the constricted wall portion, in the downstream or upstream direction of the lumen, to cause progressive contraction of the wall portion to move the bile and/or gallstones in the biliary duct.
3) The control device controls the constriction device to vary the constriction of the different areas of the wall portion, such that the wall portion is progressively constricted in the downstream or upstream direction of the lumen to move the gallstones in the biliary duct. The constriction device may include at least one elongated constriction element that extends along the wall portion, wherein the control device controls the elongated constriction element to progressively constrict the wall portion in the downstream or upstream direction of the biliary duct.
3a) In accordance with a preferred alternative of the above noted embodiment (3), the control device controls the stimulation device to progressively stimulate the constricted wall portion to cause progressive contraction thereof in harmony with the progressive constriction of the wall portion performed by the constriction device. Where the constriction device includes at least one elongated constriction element the control device controls the elongated constriction element to progressively constrict the wall portion in the downstream or upstream direction of the biliary duct. Suitably, the elongated constriction element comprises contact surfaces dimensioned to contact a length of the wall portion, when the constriction device constricts the wall portion, and the stimulation device comprises a plurality of stimulation elements distributed along the contact surfaces, such that the stimulation elements stimulate the different areas of the wall portion along the length of the wall portion, when the control device controls the stimulation device to stimulate the wall portion.
4) The constriction device is adapted to constrict any one of a series of wall portions of the tissue wall to at least restrict the movement of bile and/or gallstones in the biliary duct. The control device controls the constriction device to successively constrict the wall portions of the series of wall portions to move gallstones in the biliary duct in a peristaltic manner.
4a) In accordance with a first alternative of embodiment (4), the constriction device includes a plurality of constriction elements adapted to constrict the wall portions of the tissue wall, respectively. The control device controls the constriction device to activate the constriction elements one after the other, so that the wall portions of the series of wall portions are successively constricted along the biliary duct, whereby the gallstones are moved.
4b) In accordance with a second alternative of embodiment (4), the constriction device includes at least one constriction element that is moveable along the wall of the biliary duct to successively constrict the wall portions of the series of wall portions, wherein the control device controls the constriction device to cyclically move the constriction element along the wall portions of the series of wall portions. Preferably, the constriction device comprises a plurality of constriction elements, each of which is moveable along the wall of the biliary duct to successively constrict the wall portions of the series of wall portions, wherein the control device controls the constriction device to cyclically move the constriction elements one after the other along the wall portions of the series of wall portions. Specifically, the constriction device includes a rotor carrying the constriction elements, and the control device controls the rotor to rotate, such that each constriction element cyclically constricts the wall portions of the series of wall portions. Each constriction element suitably comprises a roller for rolling on the wall of the biliary duct to constrict the latter.
4c) In accordance with a preferred alternative of the above noted embodiment (4), the stimulation device stimulates any of the wall portions of the series of wall portions constricted by the constriction device, to close the lumen. Where the constriction device includes at least one constriction element, the stimulation device suitably includes at least one stimulation element positioned on the constriction element for stimulating the wall portion constricted by the constriction element to close the biliary duct.

Where the constriction device includes a plurality of constriction elements, the stimulation device suitably includes stimulation elements positioned on the constriction elements for stimulating the wall portions constricted by the constriction elements to close the biliary duct.

5) The constriction device is adapted to constrict any one of a series of wall portions of the tissue wall to restrict the movement of bile and/or gallstones in the biliary duct, wherein the constriction device includes a plurality of constriction elements adapted to constrict the wall portions of the tissue wall, respectively, and the stimulation device includes stimulation elements positioned on the constriction elements for stimulating the wall portions constricted by the constriction elements to close the lumen. The control device controls the constriction device to activate the constriction elements to constrict the wall portions of the series of wall portions without completely closing the lumen of the biliary duct, and controls the stimulation device to activate the stimulation elements to stimulate the wall portions one after the other, so that the wall portions of the series of wall portions are successively contracted along the biliary duct to move the bile and/or gallstones in the lumen of the biliary duct.

6) The constriction device comprises a first constriction element for constricting the wall portion at an upstream end thereof, a second constriction element for constricting the wall portion at a downstream end thereof, and a third constriction element for constricting the wall portion between the upstream and downstream ends thereof. The control device controls the first, second and third constriction elements to constrict and release the wall portion independently of one another. More specifically, the control device controls the first or second constriction element to constrict the wall portion at the upstream or downstream end thereof to close the lumen, and controls the third constriction element to constrict the wall portion between the upstream and downstream ends thereof, whereby the bile and/or gallstones contained in the wall portion between the upstream and downstream ends thereof is moved downstream or upstream in the lumen. Optionally, the control device controls the stimulation device to stimulate the wall portion between the upstream and downstream ends thereof, when the third constriction element constricts the wall portion.

6a) In accordance with a first alternative, the control device controls the first constriction element to constrict the wall portion at the upstream end thereof to restrict the movement of bile and/or gallstones in the biliary duct and controls the stimulation device to stimulate the constricted wall portion at the upstream end to close the lumen. With the lumen closed at the upstream end of the constricted wall portion, the control device controls the third constriction element to constrict the wall portion between the upstream and downstream ends thereof, and optionally controls the stimulation device to simultaneously stimulate the wall portion as the latter is constricted by the third constriction element. As a result, the gallstones contained in the wall portion between the upstream and downstream ends thereof is moved downstream in the lumen.

6b) In accordance with a second alternative, the control device controls the second constriction element to constrict the wall portion at the downstream end thereof to restrict the movement of bile and/or gallstones in the biliary duct and controls the stimulation device to stimulate the constricted wall portion at the downstream end to close the lumen. With the lumen closed at the downstream end of the constricted wall portion, the control device controls the third constriction element to constrict the wall portion between the upstream and downstream ends thereof, and optionally controls the stimulation device to simultaneously stimulate the wall portion as the latter is constricted by the third constriction element. As a result, the gallstones contained in the wall portion between the upstream and downstream ends thereof is moved upstream in the lumen. This can be used to release gallstones that are stuck in the biliary ducts. These gallstones are subsequently moved downstream, towards the duodenum.

In any of the above noted embodiments (1) to (6b), the stimulation device may stimulate the wall portion with electric pulses.

The biliary duct may be surgically prepared to extend in zigzag with adjacent walls stitched together by two parallel rows of stitches and with the adjacent walls cut through between the two rows of stitches. As a result, the lumen of this long wall portion of the biliary duct can be significantly expanded. In this case, the constriction device of the apparatus of the invention is able to move a considerably larger volume of fluid each time it constricts the long wall portion of the biliary duct.

The various solutions described above under the headline: "Flow restriction" to stop the flow in the lumen of the biliary duct may also be used in any of the above noted embodiments (1a), (1b), (4a), (5), (6), (6a) and (6b).

Importantly, in any of the embodiments above, movement of bile and/or gallstones may be carried out with electric stimulation only. Thus, in such an embodiment, there is no need for a constriction device.

Stimulation

When stimulating neural or muscular tissue there is a risk of injuring or deteriorating the tissue over time, if the stimulation is not properly performed. The apparatus of the present invention is designed to reduce or even eliminate that risk. Thus, in accordance with the present invention, the control device controls the stimulation device to intermittently stimulate different areas of the wall portion of the biliary duct, such that at least two of the areas are stimulated at different points of time that is, the stimulation is shifted from one area to another area over time. In addition, the control device controls the stimulation device, such that an area of the different areas that currently is not stimulated has time to restore substantially normal blood circulation before the stimulation device stimulates the area again. Furthermore, the control device controls the stimulation device to stimulate each area during successive time periods, wherein each time period is short enough to maintain satisfactory blood circulation in the area until the lapse of the time period. This gives the advantage that the apparatus of the present invention enables continuous stimulation of the wall portion of the biliary duct to achieve the desired flow control, while essentially maintaining over time the natural physical properties of the biliary duct without risking injuring the biliary duct.

Also, by physically changing the places of stimulation on the biliary duct over time as described above it is possible to create an advantageous changing stimulation pattern on the biliary duct, in order to achieve a desired flow control.

The control device may control the stimulation device to stimulate one or more of the areas of the wall portion at a time, for example by sequentially stimulating the different areas. Furthermore, the control device may control the stimulation device to cyclically propagate the stimulation of the areas along the wall portion, preferably in accordance with a determined stimulation pattern. To achieve the desired reaction of the tissue wall during the stimulation thereof, the control device may control the stimulation device to, preferably cyclically, vary the intensity of the stimulation of the wall portion.

In a preferred embodiment of the invention, the control device controls the stimulation device to intermittently stimulate the areas of the wall portion with pulses that preferably form pulse trains. At least a first area and a second area of the areas of the wall portion may be repeatedly stimulated with a first pulse train and a second pulse train, respectively, such that the first and second pulse trains over time are shifted relative to each other. For example, the first area may be stimulated with the first pulse train, while the second area is not stimulated with said second pulse train, and vice versa. Alternatively, the first and second pulse trains may be shifted relative to each other, such that the first and second pulse trains at least partially overlap each other.

The pulse trains can be configured in many different ways. Thus, the control device may control the stimulation device to vary the amplitudes of the pulses of the pulse trains, the duty cycle of the individual pulses of each pulse train, the width of each pulse of the pulse trains, the length of each pulse train, the repetition frequency of the pulses of the pulse trains, the repetition frequency of the pulse trains, the number of pulses of each pulse train, and/or the off time periods between the pulse trains. Several pulse trains of different configurations may be employed to achieve the desired effect.

In case the control device controls the stimulation device to vary the off time periods between pulse trains that stimulate the respective area of the wall portion, it is also possible to control each off time period between pulse trains to last long enough to restore substantially normal blood circulation in the area when the latter is not stimulated during the off time periods.

Electric Stimulation

In accordance with a preferred embodiment of the invention, the stimulation device is an electrically powered stimulation device that electrically stimulates the tissue wall portion of the patient's biliary duct, preferably with electric pulses. This embodiment is particularly suited for applications in which the wall portion includes muscle fibers that react to electrical stimula. In this embodiment, the control device controls the stimulation device to stimulate the wall portion with electric pulses preferably in the form of electric pulse trains, when the wall portion is in the constricted state, to cause contraction of the wall portion. Of course, the configuration of the electric pulse trains may be similar to the above described pulse trains and the control device may control the stimulation device to electrically stimulate the different areas of the wall of the biliary duct in the same manner as described above.

The electric stimulation device suitably comprises at least one, preferably a plurality of electrical elements, such as electrodes, for engaging and stimulating the wall portion with electric pulses. Optionally, the electrical elements may be placed in a fixed orientation relative to one another. The control device controls the electric stimulation device to electrically energize the electrical elements, one at a time, or groups of electrical elements at a time. Preferably, the control device controls the electric stimulation device to cyclically energize each element with electric pulses. Optionally, the control device may control the stimulation device to energize the electrical elements, such that the electrical elements are energized one at a time in sequence, or such that a number or groups of the electrical elements are energized at the same time. Also, groups of electrical elements may be sequentially energized, either randomly or in accordance with a predetermined pattern.

The electrical elements may form any pattern of electrical elements. Preferably, the electrical elements form an elongate pattern of electrical elements, wherein the electrical elements are applicable on the patient's wall of the biliary duct, such that the elongate pattern of electrical elements extends lengthwise along the wall of the biliary duct, and the elements abut the respective areas of the wall portion. The elongate pattern of electrical elements may include one or more rows of electrical elements extending lengthwise along the wall of the biliary duct. Each row of electrical elements may form a straight, helical or zig-zag path of electrical elements, or any form of path. The control device may control the stimulation device to successively energize the electrical elements longitudinally along the elongate pattern of electrical elements in a direction opposite to, or in the same direction as that of, the flow in the patient's biliary duct.

Optionally, the control device may control the stimulation device to successively energize the electrical elements from a position substantially at the center of the constricted wall portion towards both ends of the elongate pattern of electrical elements. Where the biliary duct is to be kept closed for a relatively long time, the control device may control the stimulation device to energize the electrical elements, such that energized electrical elements form two waves of energized electrical elements that simultaneously advance from the center of the constricted wall portion in two opposite directions towards both ends of the elongate pattern of electrical elements. Such waves of energized electrical elements can be repeated over and over again without harming the biliary duct and without moving matter in any direction in the lumen of the biliary duct.

The control device suitably controls the stimulation device to energize the electrical elements, such that the electrical elements currently energized form at least one group of adjacent energized electrical elements. In accordance with a first alternative, the elements in the group of energized electrical elements form one path of energized electrical elements. The path of energized electrical elements may extend at least in part around the patient's biliary duct. In a second alternative, the elements of the group of energized electrical elements may form two paths of energized electrical elements extending on mutual sides of the patient's biliary duct, preferably substantially transverse to the flow direction in the lumen of the biliary duct. In a third alternative, the elements of the group of energized electrical elements may form more than two paths of energized electrical elements extending on different sides of the biliary duct, preferably substantially transverse to the flow direction in the lumen of the biliary duct.

In accordance with a preferred embodiment of the invention, the electrical elements form a plurality of groups of elements, wherein the groups form a series of groups extending along the biliary duct in the flow direction in the patient's lumen. The electrical elements of each group of electrical elements may form a path of elements extending at least in part around the biliary duct. In a first alternative, the electrical elements of each group of electrical elements may form more than two paths of elements extending on different sides of the bilary duct, preferably substantially transverse to the flow direction in the patient's lumen. The control device may control the stimulation device to energize the groups of electrical elements in the series of groups in random, or in accordance with a predetermined pattern. Alternatively, the control device may control the stimulation device to successively energize the groups of electrical elements in the series of groups in a direction opposite to, or in the same direction as that of, the flow in the patient's bilary duct, or in both said directions starting from a position substantially at the center of the constricted wall portion. For example, groups of energized electrical elements may form advancing waves of energized electrical elements, as described above; that is, the control device may control the stimulation device to energize the groups of electrical elements, such that energized electrical elements form two waves of energized electrical elements that simultaneously advance from the center of the constricted wall portion in two opposite directions towards both ends of the elongate pattern of electrical elements.

A structure may be provided for holding the electrical elements in a fixed orientation. Although the structure may be separate from the constriction device, it is preferable that the structure is integrated in the constriction device, which is a practical design and facilitates implantation of the constriction and stimulation devices. Where the electrical elements form an elongate pattern of electrical elements, the structure may be applicable on the patient's biliary duct such that the elongate pattern of electrical elements extends along the biliary duct in the same direction as that of the flow in the bilary duct and the elements abut the respective areas of the wall portion of the biliary duct.

Thermal stimulation

In another embodiment of the invention, the stimulation device thermally stimulates the wall portion of the biliary duct. Thus, the control device may control the stimulation device to cool the wall portion, when the wall portion is constricted, to cause contraction of the wall portion. For example, the constriction device may constrict the wall portion to at least restrict the movement of bile and/or gallstones in the biliary duct, and the control device may control the stimulation device to cool the constricted wall portion to cause contraction thereof, such that the movement of bile and/or gallstones in the biliary duct is at least further restricted, or further restricted but not stopped, or stopped. Alternatively, the control device may control the stimulation device to heat the wall portion, when the wall portion is constricted and contracted, to cause expansion of the wall portion. Where applicable, thermal stimulation may be practised in any of the embodiments of the present invention, and the thermal stimulation may be controlled in response to various sensors, for example strain, motion or pressure sensors.

Sensor Controlled Constriction and/or Stimulation Device

As mentioned above, the apparatus may comprise at least one implantable sensor, wherein the control device controls the constriction device and/or the stimulation device in response to signals from the sensor. Generally, the sensor directly or indirectly senses at least one physical parameter of the patient, or at least one functional parameter of the apparatus, or at least one functional parameter of a medical implant in the patient.

Many different kinds of sensor for sensing physical parameters may be used. For example motion sensors for sensing biliary duct motion, *i.e.* natural contractions, such contractions, pressure sensors for sensing pressure in the biliary duct, strain sensors for sensing strain of the biliary duct, flow sensors for sensing fluid movement of bile and/or gallstones in the biliary duct of the biliary duct, spectro-photometrical sensors, Ph-sensors for sensing acidity or alkalinity of the fluid in the lumen of the biliary duct, oxygen-sensors sensors for sensing the oxygen content of the fluid in the lumen of the biliary duct, or sensors for sensing the distribution of the stimulation on the stimulated biliary duct. Any conceivable sensors for sensing any other kind of useful physical parameter may be used.

Many different kinds of sensors that sense functional parameters of the apparatus may also be used for the control of the constriction device and/or the stimulation device. For example sensors for sensing electric parameters of implanted electric components of the apparatus, or sensors for sensing the performance of implanted motors of the apparatus.

The sensor may comprise a pressure sensor for sensing as the physical parameter a pressure in the patient's body that relates to the pressure in the lumen of the biliary duct, wherein the control device controls the constriction device and/or stimulation device to change the constriction of the patient's wall portion in response to the pressure sensor sensing a predetermined value of measured pressure.

Alternatively, or in combination with the pressure sensor, a position sensor may be provided for sensing as the physical parameter the orientation of the patient with respect to the horizontal. The position sensor may be a biocompatible version of what is shown in U.S. patents 4 942 668 and 5 900 909. For example, the control device may control the constriction device and/or stimulation device to change the constriction of the patient's wall portion in response to the position sensor sensing that the patient has assumed a substantially horizontal orientation, *i.e.* that the patient is lying down.

The above described sensors may be used in any of the embodiments of the invention, where applicable.

The control device may control the constriction device and/or stimulation device to change the constriction of the patient's wall portion in response to the time of day. For that purpose the control device may include a clock mechanism for controlling the constriction device and/or stimulation device to change the constriction of the patient's wall portion to increase or decrease the influence on the movement of bile and/or gallstones in the biliary duct during different time periods of the day. In case a sensor of any of the above-described types for sensing a physical or functional parameter is provided, either the clock mechanism is used for controlling the constriction device and/or stimulation device provided that the parameter sensed by the sensor does not override the clock mechanism, or the sensor is used for controlling the constriction device and/or stimulation device provided that the clock mechanism does not override the sensor. Suitably, the control device produces an indication, such as a sound signal or displayed information, in response to signals from the sensor.

The control device may comprise an implantable internal control unit that directly controls the constriction device and/or stimulation device in response to signals from the sensor. The control device may further comprise a wireless remote control adapted to set control parameters of the internal control unit from outside the patient without mechanically penetrating the patient. At least one of the control parameters, which are settable by the wireless remote control, is the physical or functional parameter. Suitably, the internal control unit includes the above mentioned clock mechanism, wherein the wireless remote control also is adapted to set the clock mechanism.

Alternatively, the control device may comprise an external control unit outside the patient's body for controlling the constriction device and/or stimulation device in response to signals from the sensor.

Adjustable Constriction Device

In several alternative embodiments of the invention, the constriction device is adjustable. In these embodiments, there is an operation device for operating the adjustable constriction device to change the constriction of the patient's tissue wall portion, and the constriction and stimulation devices form a constriction/stimulation unit. Preferably, the constriction and stimulation devices of the constriction/stimulation unit are integrated in a single piece suitable for implantation. The constriction device of the unit comprises contact surfaces dimensioned to contact a length of a tissue wall portion of a patient's biliary duct, and the stimulation device of the unit comprises a plurality of stimulation elements provided on and distributed along the contact surfaces. When the control device controls the stimulation device to stimulate the wall portion, the stimulation elements stimulate different areas of the wall portion along the length of the wall portion. The stimulation elements preferably comprise electric elements, as described above, for stimulating the wall portion with electric pulses. However, in most applications of the present invention, other kinds of stimulations, such as thermal stimulation, could be suitable to employ.

The operation device operates the adjustable constriction device of the constriction/stimulation unit in a manner that depends on the design of the constriction device, as will be explained by the following examples of embodiments.

1) The constriction device comprises at least two elongated clamping elements having the contact surfaces and extending along the wall portion on different sides of the biliary duct, and the operation device operates the clamping elements to clamp the wall portion between the clamping elements to constrict the wall portion of the biliary duct.

2) The constriction device comprises one elongate clamping element having the contact surfaces and extending along the wall portion on one side of the biliary duct, and the operation device operates the clamping element to clamp the wall portion between the clamping element and the bone or tissue of the patient to constrict the wall portion.

3) The constriction device comprises at least two engagement elements having the contact surfaces and positioned on different sides of the biliary duct, and the operation device rotates the engagement elements, such that the engagement elements engage and constrict the wall portion of the biliary duct.

4) The constriction device comprises at least two articulated clamping elements having the contact surfaces and positioned on different sides of the biliary duct, and the operation device moves the clamping elements towards each other to clamp the wall portion of the biliary duct between the clamping elements, to constrict the wall portion.

5) The constriction device comprises at least two separate clamping elements having the contact surfaces, at least one of the clamping elements being pivoted, such that it may turn in a plane in which the loop of the constriction member extends, and the operation device turns the pivoted clamping element to change the size of the constriction opening.

6) The constriction device comprises at least one elongated constriction member having the contact surfaces, and forming means for forming the constriction member into at least a substantially closed loop around the biliary duct, wherein the loop defines a constriction opening. The operation device operates the constriction member in the loop to change the size of the constriction opening.

6a) The elongated constriction member comprises a belt having the contact surfaces, and the operation device operates the belt to change the longitudinal extension of the belt in the loop to change the size of the constriction opening. The forming means may form the constriction member or belt into a loop having at least one predetermined size.

6b) The elongated constriction member is operable to change the size of the constriction opening, such that the outer circumferential confinement surface of the constriction device is changed, or, alternatively, is unchanged.

6c) The elongated constriction member is elastic and varies in thickness as seen in a cross-section there through, and is operable to turn around the longitudinal extension of the constriction member.

6d) The elongated constriction member comprises two substantially or partly semi-circular frame elements having the contact surfaces and hinged together, such that the semi-circular elements are swingable relative to each other from a fully open state in which they substantially or partly form a circle to a fully folded state in which they substantially form a semi-circle.

7) The constriction device is adapted to bend the wall portion of the biliary duct to constrict the latter.

In the above noted embodiments (1) to (7), it is important that the constriction device is designed to constrict said length of the tissue wall portion of the biliary duct. For this purpose, the constriction device may include two or more of the described constriction elements/members to be applied in a row along said length of the wall portion, wherein said row extends in the direction of movement of bile and/or gallstones in the biliary duct of the biliary duct. Preferably, such constriction elements/members are non-inflatable and mechanically operable or adjustable.

In the above noted embodiments (1) to (7), the operation device may either mechanically or hydraulically adjust the constriction device of the constriction/stimulation unit. Also, the operation device may comprise an electrically powered operation device for operating the constriction device. For many applications of the present invention, the operation device suitably operates the constriction device, such that the through-flow area of the lumen assumes a size in the constricted state that enables the stimulation device to contract the wall portion such that the movement of bile and/or gallstones in the biliary duct is stopped.

Mechanical operation

Where the operation device mechanically operates the constriction device of the constriction/stimulation unit, it may be non-inflatable. Furthermore, the operation device may comprise a servo system, which may include a gearbox.

The term "servo system" encompasses the normal definition of a servo mechanism, *i.e.,* an automatic device that controls large amounts of power by means of very small amounts of power, but may alternatively or additionally encompass the definition of a mechanism that transfers a weak force acting on a moving element having a long stroke into a strong force acting on another moving element having a short stroke. Preferably, the operation device operates the constriction device in a non-magnetic and/or non-manual manner. A motor may be operatively connected to the operation device. The operation device may be operable to perform at least one reversible function and the motor may be capable of reversing the function.

Hydraulic Operation

Where the operation device hydraulically operates the constriction device of the constriction/stimulation unit, it includes hydraulic means for adjusting the constriction device.

In an embodiment of the invention, the hydraulic means comprises a reservoir and an expandable/contractible cavity in the constriction device, wherein the operation device distributes hydraulic fluid from the reservoir to expand the cavity, and distributes hydraulic fluid from the cavity to the reservoir to contract the cavity. The cavity may be defined by a balloon of the constriction device that abuts the tissue wall portion of the patient's biliary duct, so that the patient's wall portion is constricted upon expansion of the cavity and released upon contraction of the cavity.

Alternatively, the cavity may be defined by a bellows that displaces a relatively large contraction element of the constriction device, for example a large balloon that abuts the wall portion, so that the patient's wall portion is constricted upon contraction of the bellows and released upon expansion of the bellows. Thus, a relatively small addition of hydraulic fluid to the bellows causes a relatively large increase in the constriction of the wall portion. Such a bellows may also be replaced by a suitably designed piston/cylinder mechanism.

Where the hydraulic means comprises a cavity in the constriction device, the apparatus of the invention can be designed in accordance with the options listed below.

1) The reservoir comprises first and second wall portions, and the operation device displaces the first and second wall portions relative to each other to change the volume of the reservoir, such that fluid is distributed from the reservoir to the cavity, or from the cavity to the reservoir.

1a) The first and second wall portions of the reservoir are displaceable relative to each other by at least one of a magnetic device, a hydraulic device or an electric control device.

2) The operation device comprises a pump for pumping fluid between the reservoir and the cavity.

2a) The pump comprises a first activation member for activating the pump to pump fluid from the reservoir to the cavity and a second activation member for activating the pump to pump fluid from the cavity to the reservoir.

2a1) The first and second activation members are operable by manual manipulation thereof.

2a2) At least one of the activation members operates when subjected to an external predetermined pressure.

2a3) At least one of the first and second activating members is operable by magnetic means, hydraulic means, or electric control means.

2b) The apparatus comprises a fluid conduit between the pump and the cavity, wherein the reservoir forms part of the conduit. The conduit and pump are devoid of any non-return valve. The reservoir forms a fluid chamber with a variable volume, and the pump distributes fluid from the chamber to the cavity by a reduction in the volume of the chamber and withdraws fluid from the cavity by an expansion of the volume of the chamber. The apparatus further comprises a motor for driving the pump, wherein the pump comprises a movable wall of the reservoir for changing the volume of the chamber.

In all of the above noted embodiments 1 to 2b where the hydraulic means comprises an expandable cavity in the constriction device, the cavity can be exchanged by a cylinder/piston mechanism for adjusting the constriction device. In this case, the operation device distributes hydraulic fluid between the reservoir and the cylinder/piston mechanism to adjust the constriction device.

In a special embodiment of the invention, the operation device comprises a reverse servo operatively connected to the hydraulic means. The term "reverse servo" is to be understood as a mechanism that transfers a strong force acting on a moving element having a short stroke into a weak force acting on another moving element having a long stroke; *i.e.,* the reverse function of a normal servo mechanism. Thus, minor changes in the amount of fluid in a smaller reservoir could be transferred by the reverse servo into major changes in the amount of fluid in a larger reservoir. The reverse servo is particularly suited for manual operation thereof.

Preferably, the reverse servo comprises an expandable servo reservoir containing servo fluid and a fluid supply reservoir hydraulically connected to the servo reservoir to form a closed conduit system for the servo fluid. The expandable servo reservoir has first and second wall portions, which are displaceable relative to each other in response to a change in the volume of the expandable servo reservoir.

In accordance with a first alternative, the first and second wall portions of the servo reservoir are operatively connected to the hydraulic means. The reverse servo distributes fluid between the fluid supply reservoir and the expandable servo reservoir to change the volume of the servo reservoir, whereby the hydraulic means is operated to adjust the constriction device.

In accordance with a second alternative, there is provided an implantable main reservoir containing a predetermined amount of hydraulic fluid, wherein the reverse servo is operable to distribute hydraulic fluid between the main reservoir and the hydraulic means to adjust the constriction device. More specifically, the main reservoir is provided with first and second wall portions operatively connected to the first and second wall portions of the expandable servo reservoir, such that the volume of the main reservoir is changed when the volume of the expandable servo reservoir is changed. Thus, when the reverse servo distributes servo fluid between the fluid supply reservoir and the expandable servo reservoir to change the volume of the main reservoir, hydraulic fluid is distributed from the main reservoir to the hydraulic means, or from the hydraulic means to the main reservoir. Advantageously, the servo and main reservoirs are dimensioned, such that when the volume of the servo reservoir is changed by a relatively small amount of servo fluid, the volume of the main reservoir is changed by a relatively large amount of hydraulic fluid.

In both of the above-described alternatives, the fluid supply reservoir may have first and second wall portions, which are displaceable relative to each other to change the volume of the fluid supply reservoir to distribute servo fluid between the fluid supply reservoir and the expandable servo reservoir. The first and second wall portions of the fluid supply reservoir may be displaceable relative to each other by manual manipulation, a magnetic device, a hydraulic device, or an electric control device to change the volume of the fluid supply reservoir to distribute servo fluid between the fluid supply reservoir and the expandable servo reservoir.

In all of the above noted embodiments 1 to 2b where the hydraulic means comprises an expandable cavity in the constriction device, or in embodiments where the hydraulic means comprises a hydraulically operable mechanical construction, the operation device may include the reverse servo described above. In a further embodiment of the invention, the hydraulic means include first and second hydraulically interconnected expandable/contractible reservoirs. The first reservoir is operatively connected to the constriction device, such that the constriction device changes the constriction of the patient's wall portion upon expansion or contraction of the first reservoir. By changing the volume of the second reservoir hydraulic fluid is distributed between the two reservoirs, so that the first reservoir is either expanded or contracted. This embodiment requires no non-return valve in the fluid communication conduits between the two reservoirs, which is beneficial to long-term operation of the hydraulic means.

Alternatively, the hydraulic means may include first and second hydraulically interconnected piston/cylinder mechanisms instead of the first and second reservoirs described above. The first piston/cylinder mechanism is operatively connected to the constriction device, such that the constriction device changes the constriction of the patient's wall portion upon operation of the first piston/cylinder mechanism. By operating the second piston/cylinder mechanism hydraulic fluid is distributed between the two piston/cylinder mechanisms, so that the first piston/cylinder mechanism adjusts the constriction device.

Where the constriction device does not include an expandable/contractible cavity, the constriction device may comprise at least two elongated clamping elements having the above-mentioned contact surfaces and extending along the wall portion on different sides of the biliary duct. The hydraulic means, which may include the reverse servo described above, hydraulically moves the elongated clamping elements towards the wall portion to constrict the wall portion. For example, the constriction device may have hydraulic chambers in which the clamping elements slide back and forth, and the hydraulic means may also include a pump and an implantable reservoir containing hydraulic fluid. The pump distributes hydraulic fluid from the reservoir to the chambers to move the clamping elements against the wall portion, and distributes hydraulic fluid from the chambers to the reservoir to move the clamping elements away from the wall portion.

Design of control device

The control device suitably controls the constriction/stimulation unit from outside the patient's body. Preferably, the control device is operable by the patient. For example, the control device may comprise a manually operable switch for switching on and off the constriction/stimulation unit, wherein the switch is adapted for subcutaneous implantation in the patient to be manually or magnetically operated from outside the patient's body. Alternatively, the control device may comprise a hand-held wireless remote control, which is conveniently operable by the patient to switch on and off the constriction/stimulation unit. The wireless remote control may also be designed for application on the patient's body like a wristwatch. Such a wristwatch type of remote control may emit a control signal that follows the patient's body to implanted signal responsive means of the apparatus.

Where the control device wirelessly controls the constriction/stimulation unit from outside the patient's body, the wireless control function is preferably performed in a non-magnetic manner, *i.e.,* the control device controls the constriction device of the constriction/stimulation unit in a non-magnetic manner. The patient may use the remote control to control the constriction/stimulation unit to adjust the stimulation intensity and/or adjust the constriction of the wall portion. The wireless remote control may comprise at least one external signal transmitter or transceiver and at least one internal signal receiver or transceiver implantable in the patient.

The wireless remote control preferably transmits at least one wireless control signal for controlling the constriction/stimulation unit. The control signal may comprise a frequency, amplitude, phase modulated signal or a combination thereof, and may be an analogue or a digital signal, or a combination of an analogue and digital signal. The remote control may transmit an electromagnetic carrier wave signal for carrying the digital or analogue control signal. Also the carrier signal may comprise digital, analogue or a combination of digital and analogue signals.

Any of the above control signals may comprise wave signals, for example a sound wave signal, an ultrasound wave signal, an electromagnetic wave signal, an infrared light signal, a visible light signal, an ultra violet light signal, a laser light signal, a microwave signal, a radio wave signal, an x-ray radiation signal or a gamma radiation signal. Alternatively, the control signal may comprise an electric or magnetic field, or a combined electric and magnetic field.

As mentioned above, the control signal may follow the patient's body to implanted signal responsive means of the apparatus.

The control device may include a programmable internal control unit, such as a microprocessor, implantable in the patient for controlling the constriction/stimulation unit. The control device may further include an external control unit intended to be outside the patient's body, wherein the internal control unit is programmable by the external control unit. For example, the internal control unit may be programmable for controlling the constriction/stimulation unit over time, suitably in accordance with an activity schedule program. The apparatus of the invention may comprise an external data communicator and an implantable internal data communicator communicating with the external data communicator, wherein the internal communicator feeds data related to the constriction/stimulation unit back to the external data communicator or the external data communicator feeds data to the internal data communicator.

Source of Energy

The present invention also presents a solution for supplying energy for use in connection with the operation of the constriction/stimulation unit. Thus, in a broad sense, the present invention provides an apparatus for controlling a flow of bile and/or gallstones in a lumen formed by a tissue wall of a patient's biliary duct, wherein the apparatus comprises an implantable constriction device for gently constricting a portion of the tissue wall to influence the movement of bile and/or gallstones in the biliary duct, a stimulation device for intermittently and individually stimulating different areas of the wall portion, as the constriction device constricts the wall portion, to cause contraction of the wall portion to further influence the movement of bile and/or gallstones in the biliary duct, wherein the constriction and stimulation devices form an operable constriction/stimulation unit, a source of energy, and a control device operable from outside the patient's body to control the source of energy to release energy for use in connection with the operation of the constriction/stimulation unit. In a simple form of the invention, the source of energy, such as a battery or accumulator, is implantable in the patient's body.

Transmission of Wireless Energy

In a more sophisticated form of the invention, which is preferable, the source of energy is external to the patient's body and the control device controls the external source of energy to release wireless energy. In this sophisticated form of the invention, the apparatus comprises an energy-transmission device that transmits the released wireless energy from outside the patient's body to inside the patient's body. Among many things the wireless energy may comprise electromagnetic energy, an electric field, an electromagnetic field or a magnetic field, or a combination thereof, or electromagnetic waves. The energy-transmission device may transmit wireless energy for direct use in connection with the operation of the constriction/stimulation unit, as the wireless energy is being transmitted. For example, where an electric motor or pump operates the constriction device, wireless energy in the form of a magnetic or an electromagnetic field may be used for direct power of the motor or pump.

Thus, the motor or pump is running directly during transmission of the wireless energy. This may be achieved in two different ways: a) using a transforming device implanted in the patient to transform the wireless energy into energy of a different form, preferably electric energy, and powering the motor or pump with the transformed energy, or b) using the wirelessly transmitted energy to directly power the motor or pump. Preferably wireless energy in the form of an electromagnetic or magnetic field is used to directly influence specific components of the motor or pump to create kinetic energy for driving the motor or pump. Such components may include coils integrated in the motor or pump, or materials influenced by magnetic fields, or permanent magnets, wherein the magnetic or electromagnetic field influences the coils to generate a current for driving the motor or pump, or influences the material or permanent magnets to create kinetic energy for driving the motor or pump.

Preferably, the energy-transmission device transmits energy by at least one wireless signal, suitably a wave signal. The wave signal may comprise an electromagnetic wave signal including one of an infrared light signal, a visible light signal, an ultra violet light signal, a laser signal, a microwave signal, a radio wave signal, an x-ray radiation signal, and a gamma radiation signal. Alternatively, the wave signal may comprise a sound or ultrasound wave signal. The wireless signal may be a digital or analogue signal, or a combination of a digital and analogue signal.

Transforming Wireless Energy

In accordance with a particular embodiment of the invention, an implantable energy-transforming device is provided for transforming wireless energy of a first form transmitted by the energy-transmission device into energy of a second form, which typically is different from the energy of the first form. The constriction/stimulation unit is operable in response to the energy of the second form. For example, the wireless energy of the first form may comprise sound waves, whereas the energy of the second form may comprise electric energy. In this case, the energy-transforming device may include a piezo-electric element for transforming the sound waves into electric energy. Optionally, one of the energy of the first form and the energy of the second form may comprise magnetic energy, kinetic energy, sound energy, chemical energy, radiant energy, electromagnetic energy, photo energy, nuclear energy or thermal energy. Preferably, one of the energy of the first form and the energy of the second form is non-magnetic, non-kinetic, non-chemical, non-sonic, non-nuclear or non-thermal.

The energy-transforming device may function differently from or similar to the energy-transmission device. In a special embodiment, the energy-transforming device comprises at least one element, such as at least one semiconductor, having a positive region and a negative region, when exposed to the energy of the first form transmitted by the energy-transmission device, wherein the element is capable of creating an energy field between the positive and negative regions, and the energy field produces the energy of the second form. More specifically, the element may comprise an electrical junction element, which is capable of inducing an electric field between the positive and negative regions when exposed to the energy of the first form transmitted by the energy-transmission device, whereby the energy of the second form comprises electric energy.

The energy-transforming device may transform the energy of the first form directly or indirectly into the energy of the second form. An implantable motor or pump for operating the constriction device of the constriction/stimulation unit may be provided, wherein the motor or pump is powered by the energy of the second form. The constriction device may be operable to perform at least one reversible function and the motor may be capable of reversing the function. For example, the control device may shift polarity of the energy of the second form to reverse the motor.

The energy-transforming device may directly power the motor or pump with the transformed energy, as the energy of the second form is being transformed from the energy of the first form. Preferably, the energy-transforming device directly operates the constriction/stimulation unit with the energy of the second form in a non-magnetic, non-thermal or non-mechanical manner.

Normally, the constriction/stimulation unit comprises electric components that are energized with electrical energy. Other implantable electric components of the apparatus may be at least one voltage level guard or at least one constant current guard. Therefore, the energy-transforming device may transform the energy of the first form into a direct current or pulsating direct current, or a combination of a direct current and pulsating direct current. Alternatively, the energy-transforming device may transform the energy of the first form into an alternating current or a combination of a direct and alternating current.

The apparatus of the invention may comprise an internal source of energy implantable in the patient for supplying energy for the operation of the constriction/stimulation unit. The apparatus may further comprise an implantable switch operable to switch from an "off" mode, in which the internal source of energy is not in use, to an "on" mode, in which the internal source of energy supplies energy for the operation of the constriction/stimulation unit, and/or for energizing implanted electronic components of the apparatus. The switch may be operable by the energy of the first form transmitted by the energy-transmission device or by the energy of the second form supplied by the energy-transforming device. The described switch arrangement reduces power consumption of the apparatus between operations.

The internal source of energy may store the energy of the second form supplied by the energy-transforming device. In this case, the internal source of energy suitably comprises an accumulator, such as at least one capacitor or at least one rechargeable battery, or a combination of at least one capacitor and at least one rechargeable battery. Where the internal source of energy is a rechargeable battery it may be charged only at times convenient for the patient, for example when the patient is sleeping. Alternatively, the internal source of energy may supply energy for the operation of the constriction/stimulation unit but not be used for storing the energy of the second form. In this alternative, the internal source of energy may be a battery and the switch described above may or may not be provided.

Suitably, the apparatus of the invention comprises an implantable stabilizer for stabilizing the energy of the second form. Where the energy of the second form is electric energy the stabilizer suitably comprises at least one capacitor.

The energy-transforming device may be designed for implantation subcutaneously in the abdomen, thorax or cephalic region of the patient. Alternatively, it may be designed for implantation in an orifice of the patient's body and under the mucosa or intramuscularly outside the mucosa of the orifice.

Although the constriction/stimulation unit in the embodiments described above is designed as a single piece, which is most practical for implantation, it should be noted that as an alternative the constriction device and stimulation device could be designed as separate pieces. Any one of the constriction and stimulation units described above may alternatively be replaced by two or more separate constriction/stimulation elements, which are controlled independently of one another.

The above-described apparatus of the invention is suited for treating dysfunctions of a biliary duct, for example gallstones, of a human being or animal.

The apparatus may also comprise

The present invention also provides a method for using an apparatus as described above to control a flow of bile and/or gallstones in a lumen formed by a tissue wall of a patient's biliary duct, the method comprising:

- providing a wireless remote control adapted to control the constriction device and/or stimulation device from outside the patient's body, and
- operating the wireless remote control by the patient, when the patient wants to influence the flow of bile and/or gallstones in the lumen.

The present invention also provides a method for controlling a flow of bile and/or gallstones in a lumen formed by a tissue wall of a patient's biliary duct, the method comprising:

a) gently constricting at least one portion of the tissue wall to influence the movement of bile and/or gallstones in the biliary duct, and
b) stimulating the constricted wall portion to cause contraction of the wall portion to further influence the movement of bile and/or gallstones in the biliary duct.

BRIEF DESCRIPTION OF THE DRAWINGS

FIGURES 1A, 1B, 1C, 1D and 1E schematically illustrate different states of operation of a general embodiment of an apparatus according to the present invention.

FIGURES 1F, 1G and 1H illustrates different states of operation of a modification of the general embodiment.

FIGURES 1I, 1K and 1L illustrate an alternative mode of operation of the modification of the general embodiment.

FIGURE 2 is a longitudinal cross-section of a preferred embodiment of the apparatus according to the invention including a constriction device and an electric stimulation device.

FIGURE 3 is a cross-section along line III-III in FIGURE 2.

FIGURE 4 is the same cross-section shown in FIGURE 3, but with the apparatus in a different state of operation.

FIGURES 5A, 5B and 5C are cross-sections of the embodiment of FIGURE 2 showing different states of operations with the apparatus applied on a tissue wall of a patient's biliary duct.

FIGURES 6A, 6B and 6C are cross-sections of a modification of the embodiment of FIGURE 2 showing different states of operations with the apparatus applied on a tissue wall of a patient's biliary duct.

FIGURES 7A and 7B show different steps of an electric stimulation mode performed by the apparatus of FIGURE 2, while the apparatus is constricting a tissue wall of a patient's biliary duct.

FIGURE 8A is a pulse/time diagram showing electric stimulation pulses generated by the apparatus of the invention for stimulating a tissue wall of a patient's biliary duct.

FIGURE 8B is pulse/time diagram showing a modification of the electric stimulation shown in FIGURE 8A, in which pulses of mixed frequencies and/or amplitudes are employed.

FIGURES 9A and 9B show two pulse/time diagrams, respectively, representing electric stimulation of two different areas of the tissue wall with pulses forming pulse trains.

FIGURES 10A and 10B show the pulse/time diagrams of FIGURES 9A and 9B with modified pulse trains.

FIGURE 11A is a longitudinal cross-section of an embodiment of the apparatus of the invention including a thermal stimulation device, wherein the apparatus is constricting a tissue wall of a patient's biliary duct.

FIGURE 11B is the same embodiment of FIGURE 11A with the thermal stimulation device activated.

FIGURE 12A is a schematic view of hydraulic operation means suited for operating the constriction device of the embodiments of FIGURES 2-11.

FIGURE 12B shows the embodiment of FIGURE 12A with the constriction device constricting a tissue wall of a patient's biliary duct.

FIGURE 13A is a schematic view of mechanical operation means suited for operating the constriction device of the embodiments of FIGURES 2-11.

FIGURE 13B shows the embodiment of FIGURE 13A with the constriction device constricting a tissue wall of a patient's biliary duct.

FIGURE 13C shows a modification of the embodiment of FIGURE 13B.

FIGURE 14 illustrates the apparatus of the invention applied on the common bile duct of a gallstone patient.

FIGURE 15 is a schematic sectional view of a mechanically operable non-inflatable constriction device for use in accordance with the invention.

FIGURES 16 and 17 are cross-sectional views taken along the lines XVI-XVI and XVII-XVII, respectively, of FIGURE 15.

FIGURE 18 schematically shows an alternative design of the embodiment of FIGURE 15;

FIGURE 19 schematically illustrates a motor arrangement for the design according to FIGURE 18;

FIGURES 20 and 21 are schematic sectional views of two alternative designs of non-inflatable constriction devices of the invention.

FIGURES 22 and 23 illustrate a fully open and a reduced constriction opening, respectively, of the embodiment of FIGURE 21;

FIGURE 24 is a schematic view of a further alternative design of a non-inflatable constriction device of the invention.

FIGURES 25 and 26 illustrate a fully open and a reduced constriction opening, respectively, of the embodiment of FIGURE 24;

FIGURE 27 is a schematic view of another alternative design of a non-inflatable constriction device of the invention.

FIGURES 28 and 29 are schematic sectional views, respectively, of yet another alternative design of a non-inflatable constriction device of the invention.

FIGURE 30A is a schematic view of a hydraulically operable inflatable constriction device for use in accordance with the invention.

FIGURE 30B is the same embodiment shown in FIGURE 30A with the constriction device inflated.

FIGURES 31A, 31B, 31C and 31D are block diagrams illustrating four different principles for hydraulic operation of the constriction device shown in FIGURE 30A.

FIGURE 32 is a cross-sectional view of a reservoir having a variable volume controlled by a remote control motor.

FIGURES 33A and 33B are perspective views of a reverse servo in accordance with a particular embodiment of the hydraulic operation principle shown in FIGURE 31C.

FIGURE 34 is a schematic view of another hydraulically operable constriction device for use in accordance with the invention.

FIGURE 35A illustrates the constriction device of FIGURE 34 in a constricted state.

FIGURE 35B illustrates the constriction device of FIGURE 34 in a released state.

FIGURES 36A - 36E schematically illustrate different operation stages of an embodiment of the invention, in which a constriction device and a stimulation device co-operate to move bile and/or gallstones in the lumen of a patient's biliary duct.

FIGURE 37 is a schematic block diagram illustrating a general embodiment of the apparatus of the invention, in which energy is transferred to energy consuming components of the apparatus implanted in the patient.

FIGURES 38 to 49 are schematic block diagrams illustrating twelve embodiments, respectively, based on the general embodiment shown in FIGURE 37, wherein wireless energy is transmitted from outside a patient's body to energy consuming components of the apparatus implanted in the patient.

FIGURE 50 illustrates an energy-transforming device in the form of an electrical junction element for use in the apparatus of the present invention.

FIGURE 51 is a block diagram illustrating control components of an embodiment of the invention.

FIGURE 52 is a schematic view of exemplary circuitry of an embodiment of the invention, in which wireless energy is transformed into a current.

FIGURES 53A - 53C schematically illustrate different operation stages of another embodiment of the invention of the type shown in FIGURE 2, in which a constriction device and a stimulation device co-operate to move the bile and/or gallstones in the lumen of a patient's biliary duct.

FIGURES 54A - 54B schematically illustrate different operation stages of another embodiment of the invention of the type shown in FIGURES 36A - 36E, in which a constriction device and a stimulation device co-operate to move the bile and/or gallstones in the lumen of a patient's biliary duct.

FIGURE 55A is a schematic view of another mechanically operable non-inflatable constriction device for use in accordance with the invention.

FIGURE 55B shows the constriction device of FIGURE 55A in a constricted state.

FIGURE 55C is an end view of the embodiment of FIGURE 55B.

DETAILED DESCRIPTION OF THE INVENTION

Referring to the drawing figures, like reference numerals designate identical or corresponding elements throughout the several figures.

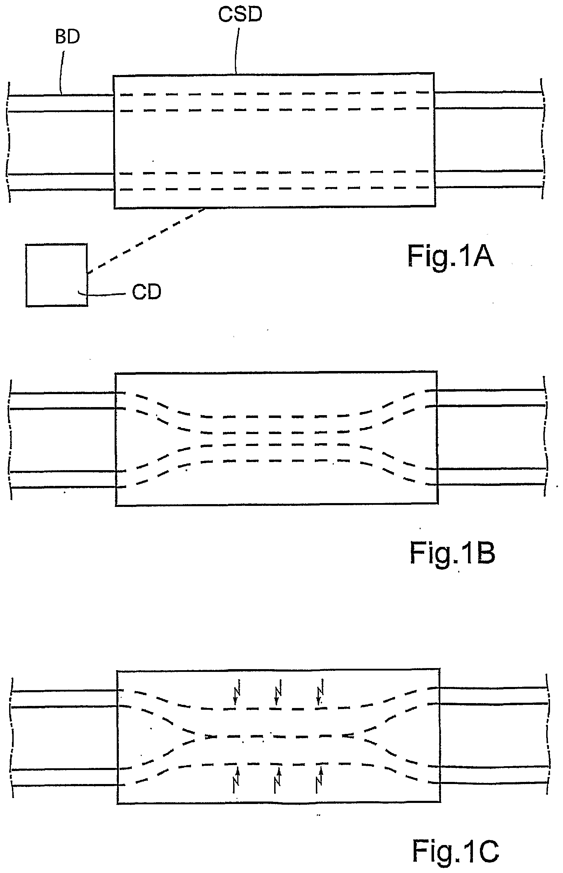

FIGURES 1A, 1B and 1C schematically illustrate different states of operation of a generally designed apparatus according to the present invention, when the apparatus is applied on a wall portion of a biliary duct designated BD. The apparatus includes a constriction device and a stimulation device, which are designated CSD, and a control device designated CD for controlling the constriction and stimulation devices CSD. FIGURE 1A shows the apparatus in an inactivation state, in which the constriction device does not constrict the biliary duct BD and the stimulation device does not stimulate the biliary duct BD. FIGURE 1B shows the apparatus in a constriction state, in which the control device CD controls the constriction device to gently constrict the wall portion of the biliary duct BD to a constricted state, in which the blood circulation in the constricted wall portion is substantially unrestricted and the movement of bile and/or gallstones in the biliary duct of the wall portion is restricted. FIGURE 1C shows the apparatus in a stimulation state, in which the control device CD controls the stimulation device to stimulate different areas of the constricted wall portion, so that almost the entire wall portion of the biliary duct BD contracts (thickens) and closes the lumen.

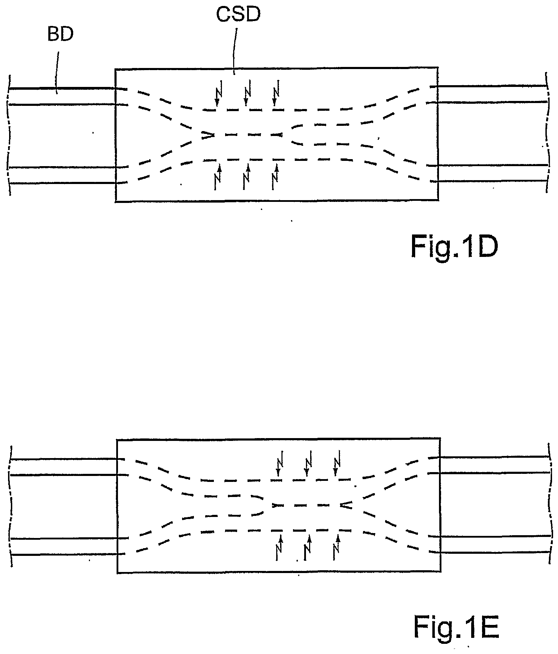

FIGURES 1D and 1E show how the stimulation of the constricted wall portion can be cyclically varied between a first stimulation mode, in which the left area of the wall portion (see FIGURE 1D) is stimulated, while the right area of the wall portion is not stimulated, and a second stimulation mode, in which the right area of the wall portion (see FIGURE 1E) is stimulated, while the left area of the wall portion is not stimulated, in order to maintain over time satisfactory blood circulation in the constricted wall portion. In addition, the figures 1D and 1E show how electric stimulation can be used to move bile and/or gallstones in the biliary duct.

It should be noted that the stimulation modes shown in FIGURES 1D and 1E only constitute a principle example of how the constricted wall portion of the biliary duct BD may be stimulated. Thus, more than two different areas of the constricted wall portion may be simultaneously stimulated in cycles or successively stimulated. Also, groups of different areas of the constricted wall portion may be successively stimulated.

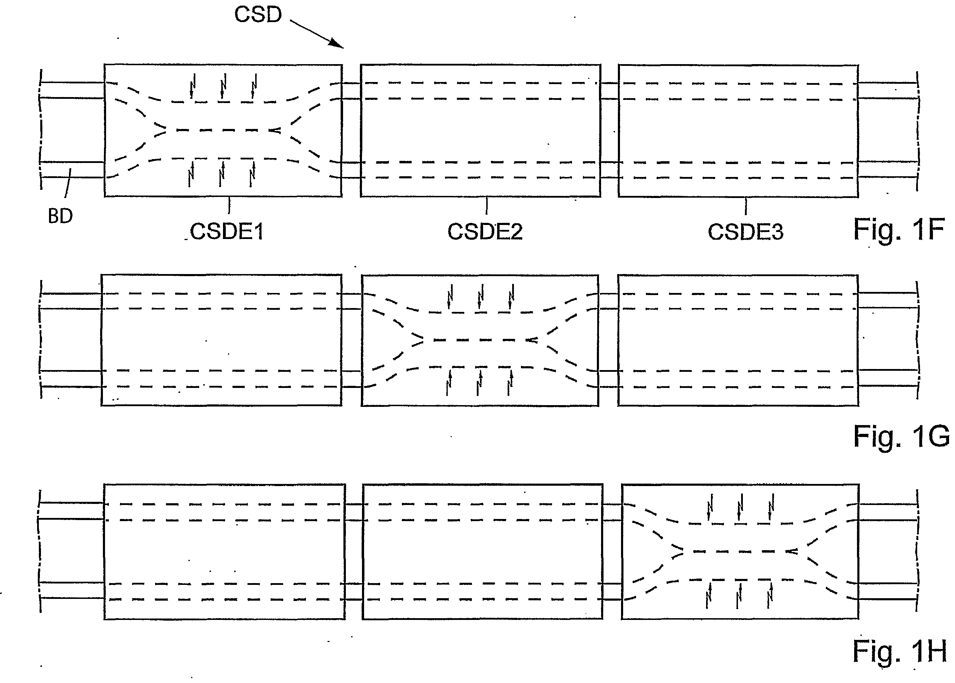

FIGURES 1F, 1G and 1H illustrate different states of operation of a modification of the general embodiment shown in FIGURES 1A-1E, wherein the constriction and stimulation devices CSD include several separate constriction/stimulation elements, here three elements CSDE1, CSDE2 and CSDE3. FIGURE 1F shows how the element CSDE1 in a first state of operation is activated to both constrict and stimulate the biliary duct BD, so that the lumen of the biliary duct BD is closed, whereas the other two elements CSDE2 and CSDE3 are inactivated. FIGURE 1G shows how the element CSDE2 in a second following state of operation is activated, so that the lumen of the biliary duct BD is closed, whereas the other two elements CSDE1 and CSDE3 are inactivated. FIGURE 1H shows how the element CSDE3 in a following third state of operation is activated, so that the lumen of the biliary duct BD is closed, whereas the other two elements CSDE1 and CSDE2 are inactivated. By shifting between the first, second and third states of operation, either randomly or in accordance with a predetermined sequence, different portions of the biliary duct can by temporarily constricted and stimulated while maintaining the lumen of the biliary duct closed, whereby the risk of injuring the biliary duct is minimized. It is also possible to activate the elements CSDE1-CSDE3 successively along the lumen of the biliary duct to move fluids and/or other bodily matter in the lumen.

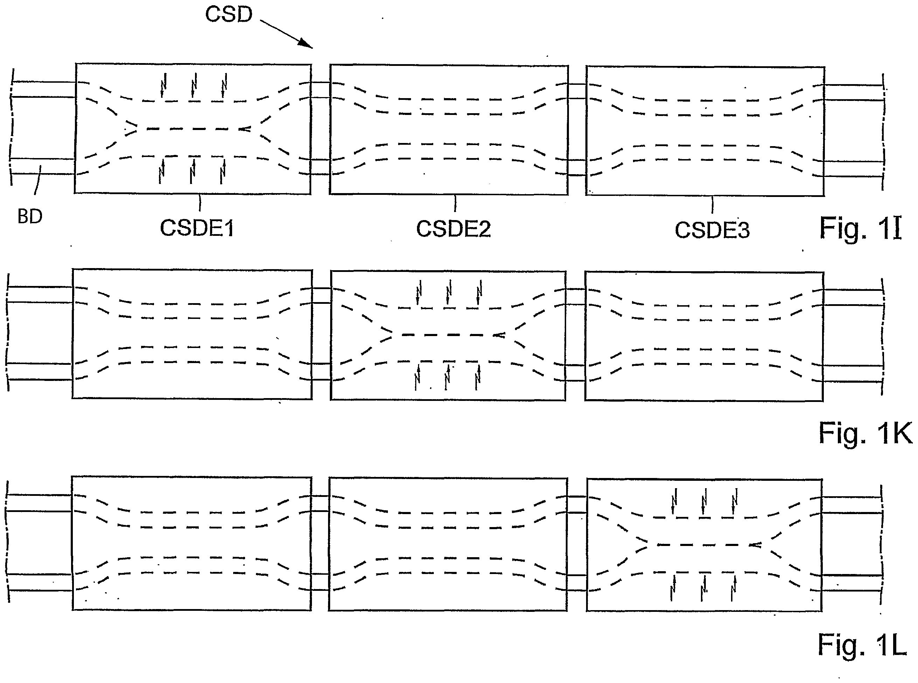

FIGURES 1I, 1K and 1L illustrate an alternative mode of operation of the modification of the general embodiment. Thus, FIGURE 1I shows how the element CSDE1 in a first state of operation is activated to both constrict and stimulate the biliary duct BD, so that the lumen of the biliary duct BD is closed, whereas the other two elements CSDE2 and CSDE3 are activated to constrict but not stimulate the biliary duct BD, so that the lumen of the biliary duct BD is not completely closed where the elements CSDE2 and CSDE3 engage the biliary duct BD. FIGURE 1K shows how the element CSDE2 in a second following state of operation is activated to both constrict and stimulate the biliary duct BD, so that the lumen of the biliary duct BD is closed, whereas the other two elements CSDE1 and CSDE3 are activated to constrict but not stimulate the biliary duct BD, so that the lumen of the biliary duct BD is not completely closed where the elements CSDE1 and CSDE3 engage the biliary duct BD. FIGURE 1L shows how the element CSDE3 in a following third state of operation is activated to both constrict and stimulate the biliary duct BD, so that the lumen of the biliary duct BD is closed, whereas the other two elements CSDE1 and CSDE2 are activated to constrict but not stimulate the biliary duct BD, so that the lumen of the biliary duct BD is not completely closed where the elements CSDE1 and CSDE2 engage the biliary duct BD. By shifting between the first, second and third states of operation, either randomly or in accordance with a predetermined sequence, different portions of the biliary duct can by temporarily stimulated while maintaining the lumen of the biliary duct closed, whereby the risk of injuring the biliary duct is reduced. It is also possible to activate the stimulation of the elements CSDE1-CSDE3 successively along the lumen of the biliary duct BD to move bile and/or gallstones in the biliary duct.

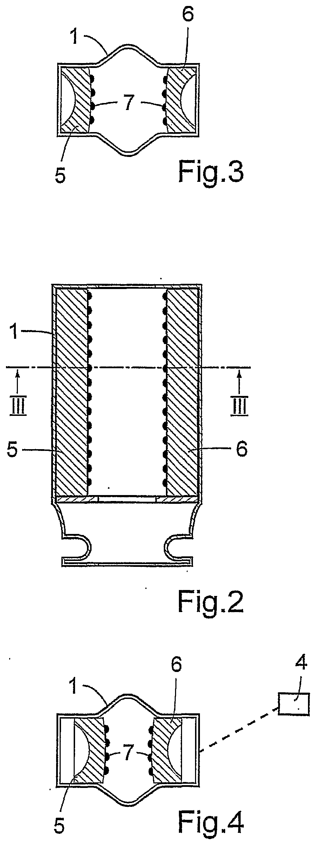

FIGURES 2-4 show basic components of an embodiment of the apparatus according to the invention for controlling a flow of bile and/or gallstones in a lumen formed by a tissue wall of a patient's biliary duct. The apparatus comprises a tubular housing 1 with open ends, a constriction device 2 arranged in the housing 1, a stimulation device 3 integrated in the constriction device 2, and a control device 4 (indicated in FIGURE 4) for controlling the constriction and stimulation devices 2 and 3. The constriction device 2 has two elongate clamping elements 5, 6, which are radially movable in the tubular housing 1 towards and away from each other between retracted positions, see FIGURE 3, and clamping positions, see FIGURE 4. The stimulation device 3 includes a multiplicity of electrical elements 7 positioned on the clamping elements 5, 6, so that the electrical elements 7 on one of the clamping elements 5, 6 face the electrical elements 7 on the other clamping element. Thus, in this embodiment the constriction and stimulation devices form a constriction/stimulation unit, in which the constriction and stimulation devices are integrated in a single piece.

The constriction and stimulation devices may also be separate from each other. In this case, a structure may be provided for holding the electrical elements 7 in a fixed orientation relative to one another. Alternatively, the electrical elements 7 may include electrodes that are separately attached to the wall portion of the patient's biliary duct.

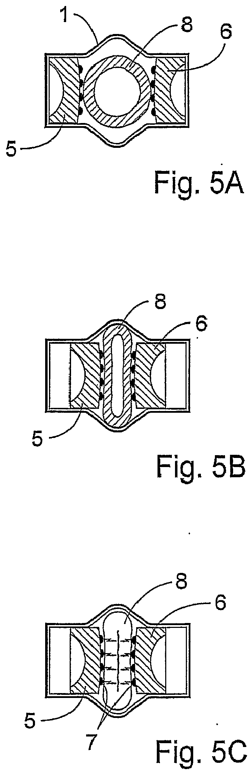

FIGURES 5A - 5C illustrate in principle the function of the apparatus of FIGURE 2 when the apparatus is applied on a portion 8 of a tubular tissue wall of a patient's biliary duct. Thus, FIGURE 5A shows the apparatus in a non-clamping state, in which the clamping elements 5, 6 are in their retracted positions and the wall portion 8 extends through the open ends of the housing 1 without being constricted by the clamping elements 5, 6. FIGURE 5B shows the apparatus in a clamping state, in which the clamping elements 5, 6 have been moved from their retracted positions to their clamping positions, in which the clamping elements 5, 6 gently constrict the wall portion 8 to a constricted state, in which the blood circulation in the constricted wall portion 8 is substantially unrestricted and the movement of bile and/or gallstones in the biliary duct of the wall portion 8 is restricted. FIGURE 5C shows the apparatus in a stimulation state, in which the clamping elements 5, 6 constrict the wall portion 8 and the electrical elements 7 of the stimulation device 3 electrically stimulate different areas of the wall portion 8, so that the wall portion 8 contracts (thickens) and closes the lumen.

When the apparatus is in its stimulation state, it is important to stimulate the different areas of the wall portion 8 in a manner so that they essentially maintains their natural physical properties over time to prevent the areas from being injured. Consequently, the control device 4 controls the stimulation device 3 to intermittently stimulate each area of the wall portion 8 during successive time periods, wherein each time period is short enough to maintain over time satisfactory blood circulation in the area. Furthermore, the control device 4 controls the stimulation of the areas of the wall portion 8, so that each area that currently is not stimulated restores substantially normal blood circulation before it is stimulated again. To maintain over time the effect of stimulation, *i.e.,* to keep the lumen closed by maintaining the wall portion 8 contracted, the control device 4 controls the stimulation device 3 to stimulate one or more of the areas at a time and to shift the stimulation from one area to another over time. The control device 4 may control the stimulation device 3 to cyclically propagate the stimulation of the areas along the tubular wall portion 8, for example, in accordance with a determined stimulation pattern. To achieve the desired reaction of the tissue wall during the stimulation thereof, the control device may control the stimulation device to, preferably cyclically, vary the intensity of the stimulation of the wall portion 8.

In the embodiment of FIGURES 2 - 4, the electrical elements 7 form a series of fourteen groups of electrical elements 7 extending longitudinally along each elongate clamping element 5 and 6, respectively, see FIGURE 2. The electrical elements 7 of each group of electrical elements 7 form a first path of four electrical elements 7 positioned in a row on clamping element 5 and extending tranverse thereto, and a second path of four electrical elements 7 positioned in a row on clamping element 6 and extending tranverse thereto. Thus, the two paths of electrical elements 7 extend on mutual sides of the patient's biliary duct. The control device 4 controls the stimulation device 3 to successively energize the groups of electrical elements 7 in the series of groups in a direction opposite to or, alternatively, in the same direction as that of the movement of bile and/or gallstones in the biliary duct of the patient's biliary duct. Of course, the number of electrical elements 7 of each path of electrical elements 7 can be greater or smaller than four, and several parallel rows electrical elements 7 can form each path of electrical elements 7.

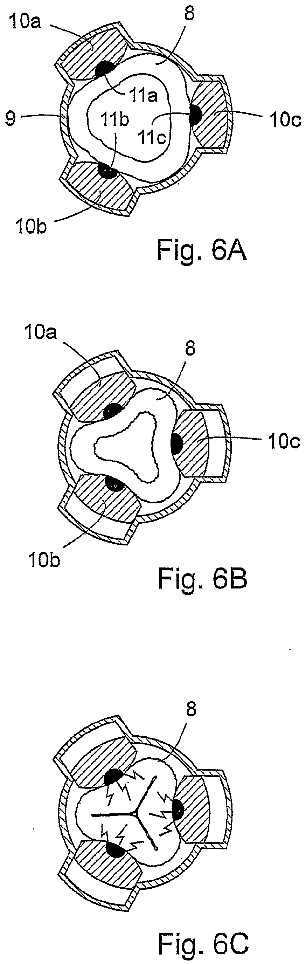

FIGURES 6A - 6C show another embodiment of the invention which includes a tubular housing 9 and three elongate clamping elements 10a, 10b, 10c, which are radially movable in the tubular housing 9 towards and away from a central axis thereof between retracted positions, see FIGURE 6A, and clamping positions, see FIGURE 6B. The three clamping elements 10a-10c are symmetrically disposed around the central axis of the housing 9. The stimulation device of this embodiment includes electrical elements 11a, 11 b, 11c that form a series of groups of elements extending longitudinally along the elongate clamping elements 10a-10c, wherein the electrical elements 11a - 11c of each group of electrical elements form a path of three electrical elements 11a, 11b and 11c extending circumferentially around the central axis of the housing 9. The three electrical elements 11a - 11c of each group are positioned on the three clamping elements 10a-10c, respectively. Thus, the path of three electrical elements 11a-11c extends around the patient's biliary duct. Of course, the number of electrical elements 11a-11c of each path of electrical elements can be greater than three, and several parallel rows electrical elements 11a-11c can form each path of electrical elements.

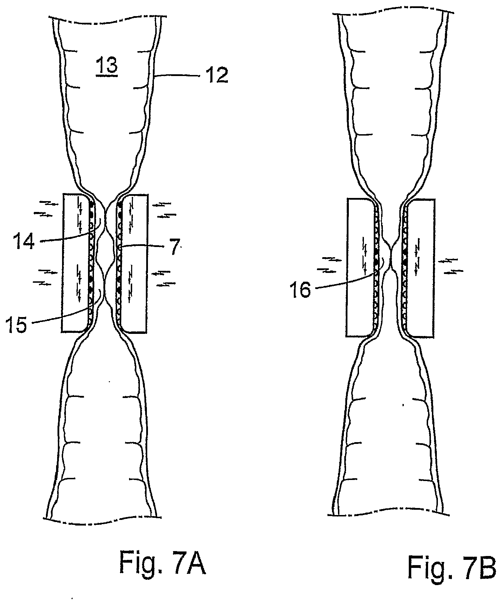

FIGURES 7A and 7B show different steps of an electric stimulation mode performed by the apparatus of FIGURE 2 while the clamping elements 5, 6 of the apparatus are constricting a portion of a tubular tissue wall of a patient's biliary duct 12 to restrict the movement of bile and/or gallstones in the biliary duct 13 of the biliary duct 12. For the sake of clarity only the clamping elements 5, 6 of the constriction device 2 are shown in FIGURES 7A, 7B. Thus, FIGURE 7A illustrates how energized electrical elements 7 of groups of electrical elements electrically stimulate a first portion 14 and a second portion 15 of the tubular wall to contract and close the lumen 13. FIGURE 7B illustrates how energized electrical elements 7 of other groups of electrical elements electrically stimulate a third portion 16 of the tubular wall different from the first and second portions to contract and close the lumen 13, while the electrical stimulation of the first and second portions 14, 15 of the tubular wall has been ceased, so that substantially normal blood circulation in the first and second portions is restored. In this manner, the electric stimulation of the constricted tubular wall is shifted over time from one portion of the tubular wall to another to insure recurrent restoration of blood circulation in the constricted tubular wall.

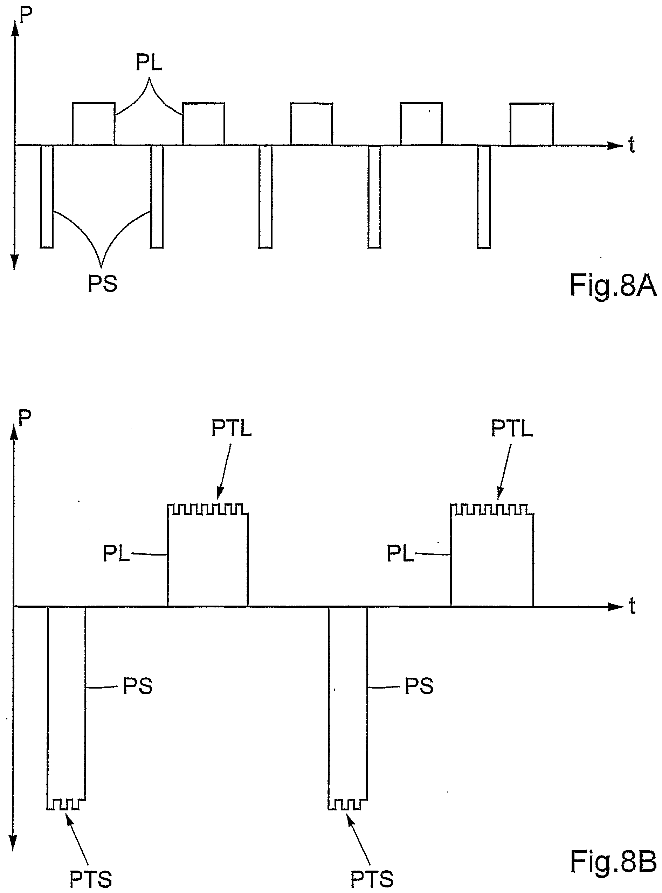

The control device 4 controls the stimulation device 3 to energize the electrical elements 7 with electric biphasic pulses, *i.e.,* combined positive and negative pulses. The desired stimulation effect is achieved by varying different pulse parameters. Thus, the control device 4 controls the stimulation device 3 to vary the pulse amplitude (voltage), the off time period between successive pulses, the pulse duration and the pulse repetition frequency. The pulse current should be between 1 to 30mA. For neural stimulation, a pulse current of about 5mA and pulse duration of about 300$\mu$s are suitable, whereas a pulse current of about 20mA and a pulse duration of about 30$\mu$s are suitable for muscular stimulation. The pulse repetition frequency suitably is about 10Hz. For example, as illustrated in the Pulse/time diagram P/t of FIGURE 8A, a pulse combination including a negative pulse PS of short duration and high amplitude (voltage), and a positive pulse PL of long duration and low amplitude following the negative pulse may be cyclically repeated to form a pulse train of such pulse combinations. The energy content of the negative pulse PS should be substantially equal to the energy content of the positive pulse PL.

FIGURE 8B is a pulse/time diagram showing a modification of the electric stimulation shown in FIGURE 8A. Thus, the pulse combination of FIGURE 8A is mixed with a pulse train combination having a first relatively long pulse train PTL of high frequency/low amplitude pulses, appearing simultaneously with the positive pulse PL of the pulse combination of FIGURE 8A, and a second relatively short pulse train PTS of high frequency/low amplitude appearing simultaneously with the negative pulse PS of the pulse combination shown in FIGURE 8A. As a result, the high frequency/low amplitudes pulse trains PTL and PTS are superimposed on the positive and negative pulses PL and PS of FIGURE 8A, as illustrated in FIGURE 8B. The pulse configuration of FIGURE 8B, and variations thereof, is beneficial to use in connection with the stimulation of particular human biliary ducts, in order to achieve the desired stimulation effect.

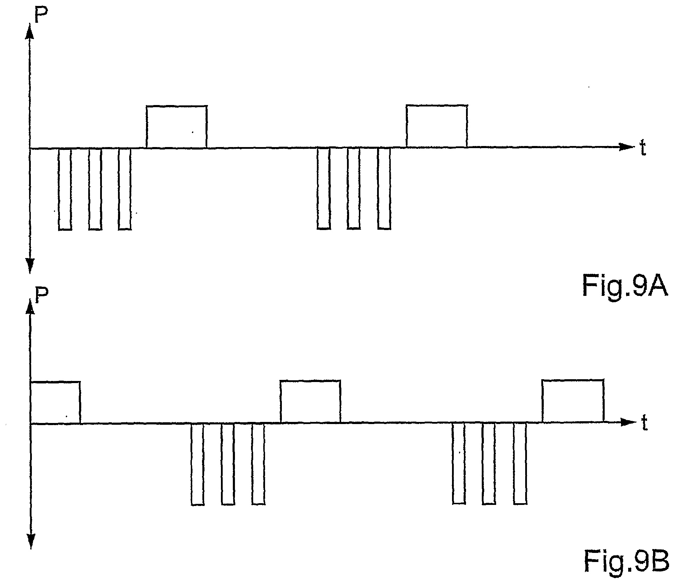

Preferably, the electric pulses form pulse trains, as illustrated in the Pulse/time diagrams P/t of FIGURES 9A, 9B, 9C and 9D. The Pulse/time diagram P/t of FIGURE 9A represents an individual area of the wall portion of the patient's biliary duct which is stimulated with a pulse train 18A. The pulse train 18A includes three initial negative pulses, each of which is of short duration and high amplitude (voltage), and one positive pulse of long duration and low amplitude following the negative pulses. After a delay to enable the area of the biliary duct to restore substantially normal blood circulation, the pulse train 18A is repeated.

The Pulse/time diagram P/t of FIGURE 9B represents another individual area of the wall portion, which is stimulated with a pulse train 18B having the same configuration as the pulse train 18A. The pulse trains 18A and 18B are shifted relative to each other, so that they partially overlap one another to ensure that the constricted wall portion always is stimulated to contract as desired.

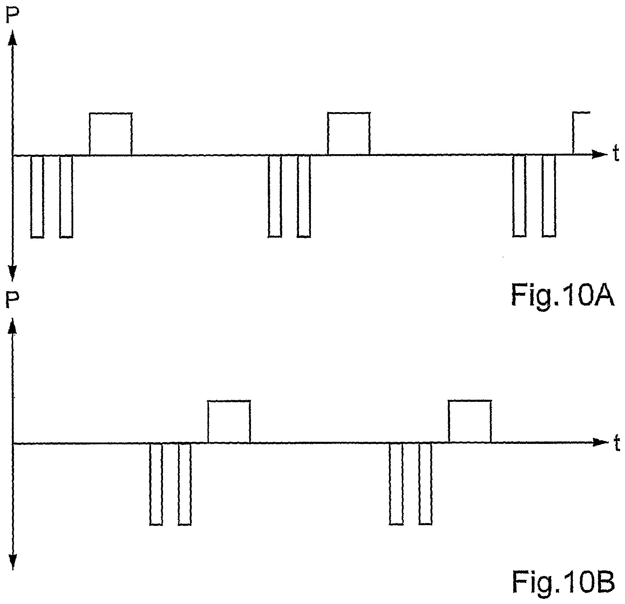

The pulse/time diagrams P/t of FIGURES 10A and 10B represent two different areas of the wall portion, which are stimulated with cyclically repeated pulse trains 18C and 18D, respectively, having the same configuration. Each pulse train 18C, 18D includes two initial negative pulses, each of which is of short duration and high amplitude (voltage), and one positive pulse of long duration and low amplitude following the two negative pulses. In this case, the pulse trains 18C and 18D are shifted relative to each other, so that they do not overlap each other. Thus, the off time period between adjacent pulse trains 18C is longer than the duration of pulse train 18D and the off time period between adjacent pulse trains 18D is longer than the duration of pulse train 18C.

The pulse trains 18A, 18B, 18C and 18D can be configured in many different ways. Thus, the control device 4 can control the stimulation device 2 to vary the length of each pulse train, the repetition frequency of the pulse trains, the number of pulses of each pulse train, and/or the off time periods between the pulse trains. Typically, the control device 4 controls each off time period between the pulse trains to last long enough to restore substantially normal blood circulation in the area that just has been stimulated before that area again is stimulated with electric pulses.

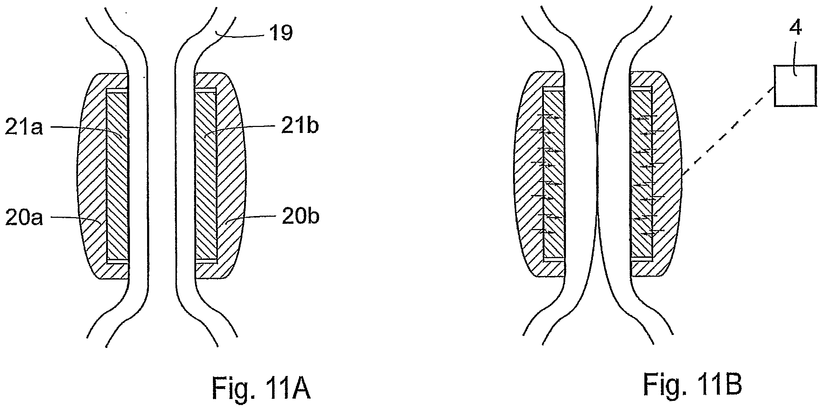

FIGURES 11A and 11B show another embodiment of the invention that controls flow in a biliary duct 19, comprising a constriction device with two clamping elements 20a and 20b, a stimulation device in the form of two thermal stimulation elements 21a and 21b integrated in the clamping elements 20a, 20b, respectively, and a control device 4 for controlling the clamping elements 20a, 20b and stimulation elements 21a, 21b. The clamping elements 20a and 20b are movable towards and away from each other in the same manner as described above in connection with the embodiment according to FIGURES 5A-5C. The thermal stimulation elements 21a and 21b, which may include Pertier elements, are positioned on the clamping elements 20a, 20b, so that the thermal elements 21a are facing the thermal elements 21b. FIGURE 11A shows how the clamping elements 20a, 20b constrict the biliary duct 19, so that the flow is restricted. FIGURE 11B shows how the control device 4 controls the thermal stimulation elements 21a, 21b to cool the wall of the biliary duct 19, so that the wall contracts and closes the biliary ducct 19. To release the biliary duct 19, the control device 4 controls the thermal stimulation elements 21a, 21b to heat the wall of the bilary duct 19, so that the wall expands.

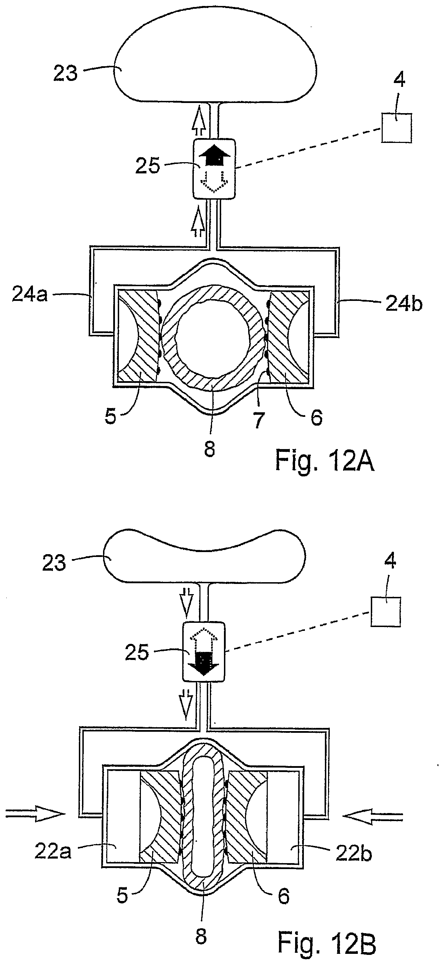

FIGURES 12A and 12B show hydraulic operation means suited for operating the constriction device of the embodiments described above. Specifically, FIGURES 12A and 12B show the apparatus of FIGURE 2 provided with such means for hydraulic operation of the constriction device 2. (The stimulation device is not shown.) Thus, the housing 1 forms two hydraulic chambers 22a and 22b, in which the two clamping elements 5, 6 are slidable back and forth relative to the tubular tissue wall portion 8 of a patient's biliary duct. The hydraulic operation means include an expandable reservoir 23, such as an elastic balloon, containing hydraulic fluid, conduits 24a and 24b between the reservoir 23 and the hydraulic chambers 22a, 22b, and a two-way pump 25 for pumping the hydraulic fluid in the conduits 24a, 24b. The control device 4 controls the pump 25 to pump hydraulic fluid from the reservoir 23 to the chambers 22a, 22b to move the clamping elements 5, 6 against the wall portion 8, whereby the tubular wall portion 8 is constricted, see FIGURE 12B, and to pump hydraulic fluid from the chambers 22a, 22b to the reservoir 23 to move the clamping elements 5, 6 away from the wall portion 8, whereby the tubular wall 8 is released, see FIGURE 12A.

Alternatively, the embodiment of FIGURES 12A and 12B may be manually operated by applying suitable manually operable hydraulic means for distributing the hydraulic fluid between the expandable reservoir 23 and the hydraulic chambers 22a, 22b. In this case the pump 25 is omitted.

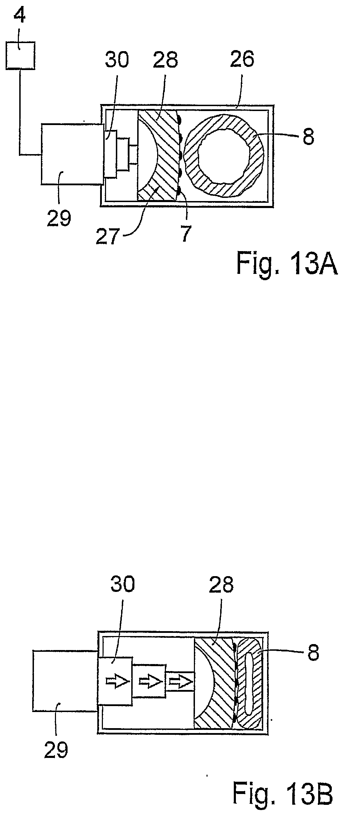

FIGURES 13A and 13B schematically show a mechanically operable embodiment of the invention, comprising an open ended tubular housing 26 applied on the tubular tissue wall portion 8 of a patient's biliary duct, a constriction device 27 arranged in the housing 26 and a control device 4 for controlling the constriction device 27. A stimulation device (not shown) as described above is also provided in the housing 26. The constriction device 27 includes a clamping element 28, which is radially movable in the tubular housing 26 towards and away from the tubular wall portion 8 between a retracted position, see FIGURE 13A, and a clamping position, see FIGURE 13B, in which the clamping element 28 gently constricts the tubular wall portion 8. Mechanical operation means for mechanically operating the clamping element 28 includes an electric motor 29 attached to the housing 26 and a telescopic device 30, which is driven by the motor 29 and operatively connected to the clamping element 28. The control device 4 controls the electric motor 29 to expand the telescopic device 30 to move the clamping element 28 against the wall portion 8, whereby the tubular wall portion 8 is constricted, see FIGURE 13B, and controls the motor 29 to retract the telescopic device 30 to move the clamping element 28 away from the wall portion 8, whereby the wall portion 8 is released, see FIGURE 13A.

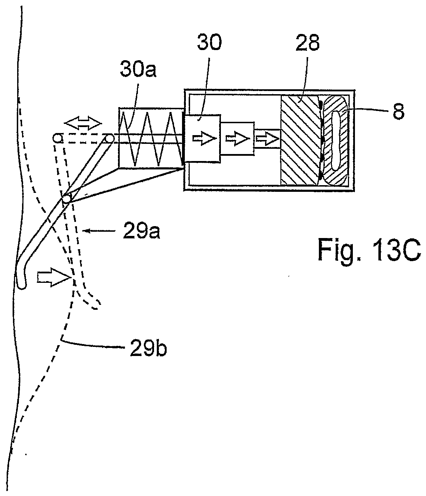

Alternatively, the motor 29 may be omitted and the telescopic device 30 be modified for manual operation, as shown in FIGURE 13C. Thus, a spring 30a may be provided acting to keep the telescopic device 30 expanded to force the clamping element 28 against the wall portion 8. The mechanical operation means may include a subcutaneously implanted lever mechanism 29a that is operatively connected to the telescopic device 30. The patient may push the lever mechanism 29a through the patient's skin 29b to pull the telescopic device 30 against the action of the spring 30a to the retracted position of the telescopic device 30, as indicated in phantom lines. When the patient releases the lever mechanism 29a, the spring 30a expands the telescopic device 30, whereby clamping element 28 is forced against the wall portion 8.

The mechanical operation means as described above in connection with FIGURES 13A, 13B and 13C may also be implemented in the embodiments according to FIGURES 1-11.

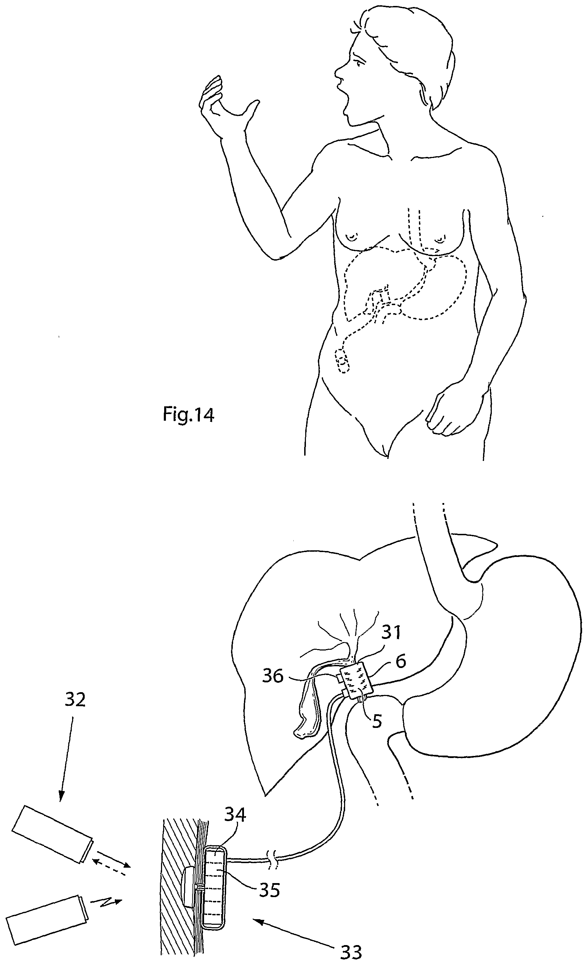

FIGURE 14 illustrates the embodiment of FIGURE 2 applied on the common bile duct 31 of a gallstone patient. The clamping elements 5, 6 of the constriction device 2 constrict the common bile duct 31 and the stimulation device 3 is energized to close the common bile duct. (For the sake of clarity, the housing is not shown and the clamping elements 5, 6 are exaggerated.) In this embodiment, a control device includes an external control unit in the form of a hand-held wireless remote control 32. The remote control 32 is operable by the patient to control the internal control unit 33 to switch on and off the constriction device and/or the stimulation device. Alternatively, however, the remote control 32 may be replaced by a subcutaneously implanted push button that is manually switched by the patient between "on" and"off". Such a manually operable push button may also be provided in combination with the remote control 32 as an emergency button to allow the patient to stop the operation of the apparatus in case of emergency or malfunction.

The internal control unit 33 controls an implanted operation device 34 to move the clamping elements 5, 6. An implanted source of energy 35, such as a rechargeable battery, powers the operation device 34. The internal control unit 33, which may be implanted subcutaneously or in the abdomen, also works as en energy receiver, *i.e.,* for transforming wireless energy into electric energy and charging the implanted source of energy 35 (rechargeable battery) with the electric energy.

An implanted sensor 36 senses a physical parameter of the patient, such as the pressure in the intestines, or a parameter that relates to the pressure in the common bile duct, wherein the internal control unit 33 controls the constriction device 2 and/or the stimulation device 3 in response to signals from the sensor 36. In this embodiment the sensor 36 is a pressure sensor, wherein the internal control unit 33 controls the constriction device and/or stimulation device to change the constriction of the patient's common bile duct 31 in response to the pressure sensor 36 sensing a predetermined value of measured pressure. For example, the control unit 33 may control the constriction device and/or stimulation device to increase the constriction of the patient's intestines 31 in response to the pressure sensor sensing an increased pressure. Alternatively or in combination, the remote control 32 controls the constriction device and/or stimulation device in response to signals from the sensor 36, in the same manner as the internal control unit 33.

The remote control 32 may be equipped with means for producing an indication, such as a sound signal or displayed information, in response to signals from the sensor 36.

Of course, the constriction device 2 shown in FIGURE 14 may be replaced by any one of the devices described in the various embodiments of the present invention, where applicable.

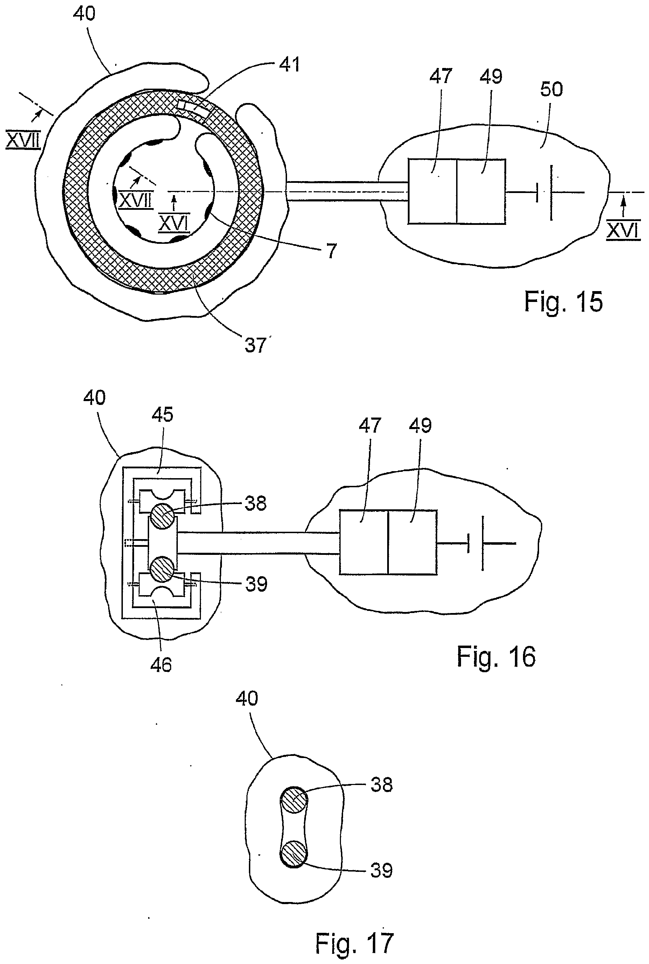

FIGURES 15-17 show a mechanically operable constriction device having an elongated constriction member in the form of a circular resilient core 37 with two overlapping end portions 38, 39. The core 37 defines a substantially circular restriction opening and is enclosed in an elastic soft hose 40 except at a releasable and lockable joint 41 of the core 37, which when released enables application of the core 37 with its hose 40 around a portion of a tubular tissue wall of a patient's biliary duct. The materials of all of these elements are bio-compatible so that the patient' body will not reject them. An operation device 42 for mechanically operating the longitudinal extension of the core 37 to change the size of the restriction opening comprises a drive wheel 43 in frictional engagement with the overlapping end portions 38, 39 of the core 37. The drive wheel 43 is journalled on a holder 44 placed in the hose 40 and provided with two counter pressure rollers 45, 46 pressing the respective end portions 38, 39 of the core 37 against the drive wheel 43 to increase the frictional engagement there between. An electric motor 47 of the operation device is connected to the drive wheel 43 via a long flexible drive shaft 48, and is moulded together with a remote controlled power supply unit 49 in a body 50 of silicone rubber. The length of the flexible drive shaft 48 is selected so that the body 50 can be placed in a desired position in the patient's body, suitably in the abdomen.

The power supply unit 49 can be controlled to power the electric motor 47 to turn the drive wheel 43 in one direction to reduce the diameter of the core 37, so that the wall portion is constricted, or to turn the drive wheel 43 in the opposite direction to increase the diameter of the core 37, so that the wall portion is released.

In accordance with a first alternative, a rack gear may be formed on one of the end portions 38, 39 of the core 37 and the drive wheel 43 may be replaced by a drive gear wheel connected to the other end portion of the core 37 and in mesh with the rack gear.

In accordance with a second alternative, the operation device 42 may be designed as a worm-driven hose clamp, *i. e.,* one of the end portions 38, 39 of the core 37 may be provided with threads and the other end portion of the core 37 may be provided with a worm, the threads of which interacts with the threads of said one end portion of the core 37. The threads of such a worm may also interact with threads provided on both end portions 38, 39 of the core 37. In this alternative, the electric motor 47 turns the worm in one direction to reduce the diameter of the core 37, so that the wall portion is constricted, or turn the worm in the opposite direction to increase the diameter of the core 37, so that the wall portion is released in one direction to reduce the diameter of the core 37, so that the wall portion is constricted, or turns the clamping screw in the opposite direction to increase the diameter of the core 37, so that the wall portion is released.

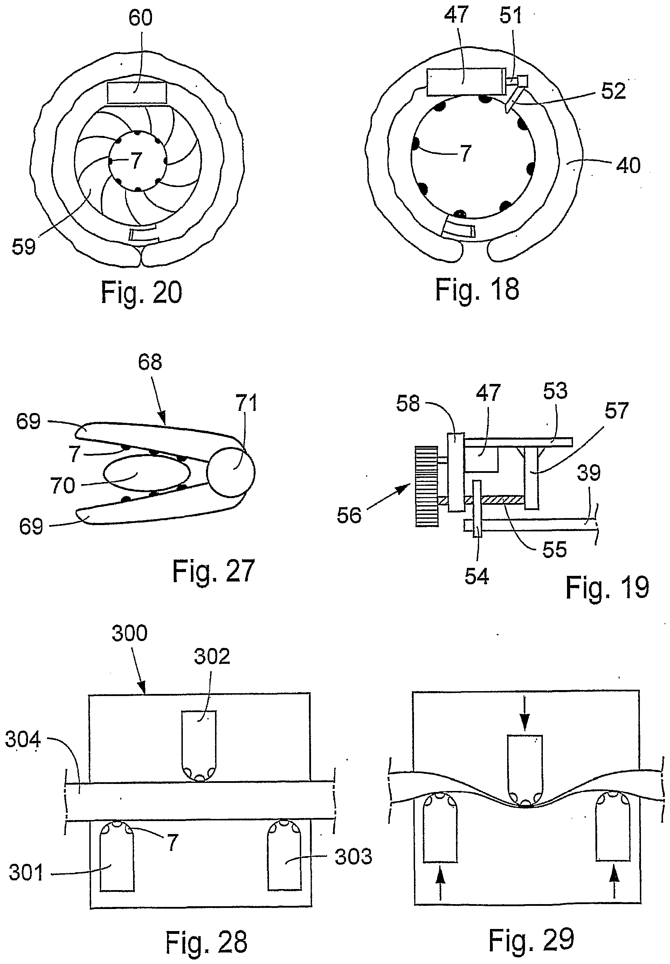

FIGURE 18 shows a constriction device which is identical to the embodiment of FIGURES 15-17, except that the motor 47 is encapsulated in the hose 40 so that it is fixed to the core 37 and has a short drive shaft 51, and that the motor 47 is positioned relative to the core 37, such that the drive shaft 51 extends substantially tangentially to the circular core 37. There is an angular gearing 52 connecting the drive shaft 51 to the drive wheel 43.

FIGURE 19 shows a suitable alternative arrangement for the motor 47 in the embodiment of FIGURE 18, comprising a first clamping member 53 secured to one end portion of the core 37 and a second clamping member 54 secured to the other end portion 39 of the core 37. The motor 47 is secured to the first clamping member 53 and is operatively connected to a worm gear 55 via a gear transmission 56. The worm gear 55 is journalled at its opposite ends on holders 57 and 58, which are rigidly secured to the clamping member 53 and the motor 47, respectively. The second clamping member 54 has a pinion in mesh with the worm gear 55. When the motor 47 is powered, the worm gear 55 rotates, and will thereby pull the end portion 39 of the core 37 in one or the opposite longitudinal direction, so that the diameter of the substantially circular core 37 is either increased or decreased. The motor 47, worm gear 55, gear transmission 56 and second clamping member 54 constitute a servo system of the type that transfers a weak force acting on a moving element having a long stroke into a strong force acting on another moving element having a short stroke.

FIGURE 20 shows a constriction device including a plurality of arcuate lamellae 59 arranged like the conventional adjustable aperture mechanism of a camera. A motor 60 operates the lamellae 59 to change the size of a restriction opening defined by the lamellae 59.

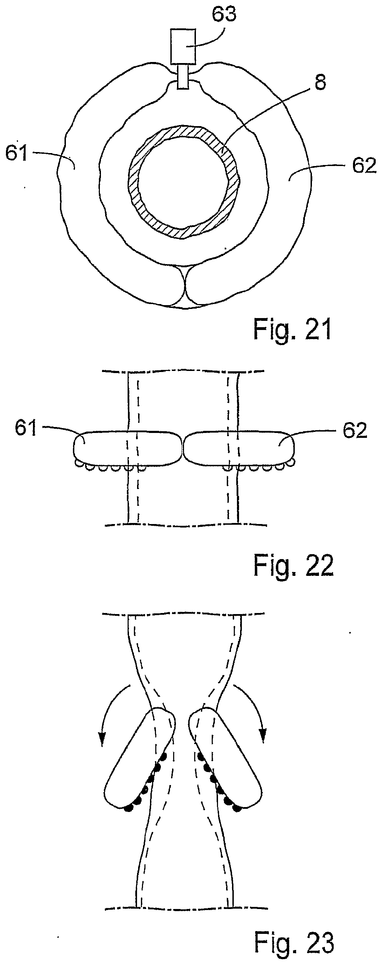

FIGURES 21-23 show a constriction device including two semi-circular elements 61 and 62, which are hinged together such that the semi-circular elements 61, 62 are swingable relative to each other between a fully open state in which they substantially form a circle, as illustrated in FIGURE 22, and an angular state, in which the size of the restriction opening defined by the semi-circular elements 61, 62 is reduced, as illustrated in FIGURE 23. A motor 63 operates the semi-circular elements 61, 62 to swing them relative to each other.

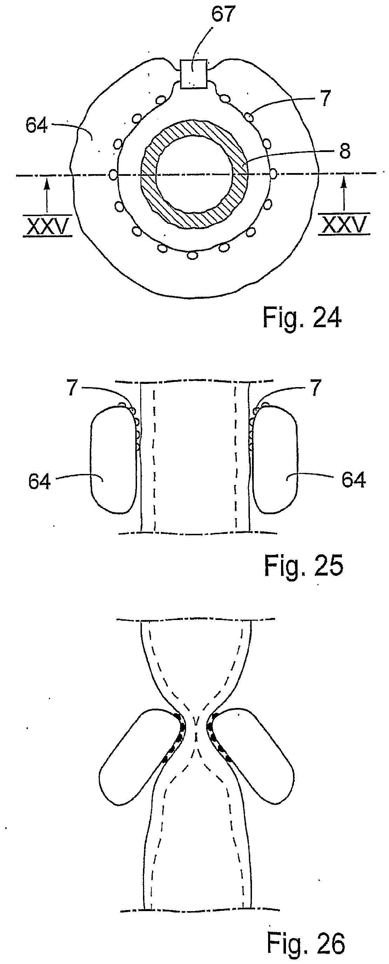

FIGURES 24-26 show a constriction device including an elastic belt 64 forming a circle and having a substantially oval cross-section. A motor 67 operates the belt 64 to turn around the longitudinal extension thereof between a fully open state, in which the inner broader side of the belt 64 forms a substantially cylindrical surface, as illustrated in FIGURE 25, and a reduced open state, in which the inner broader side of the belt 64 forms a substantially conical surface, as illustrated in FIGURE 26.

FIGURE 27 shows a constriction device 68 having two rigid articulated clamping elements 69 positioned on opposite sides of a portion of a tubular tissue wall 70 of a patient's biliary duct. An operation device 71 turns the clamping elements 69 toward each other to clamp the wall portion 70 between the clamping elements 69 to thereby contract the wall portion, and turns the clamping elements 69 away from each other to release the wall portion from the clamping elements 69.

FIGURES 28 and 29 show an embodiment of the apparatus of the invention comprising a constriction device 300 having three bending members 301, 302 and 303 displaced relative to one another in a row along a portion of a tubular tissue wall 304 of a patient's biliary duct and positioned alternately on opposite sides of the tubular wall 304. (Alternatively, each member 301, 302 and 303 may take the shape of an hour-glass.) An operation device (not shown) moves the two outer members 301, 303 laterally against the tubular wall 304 in one direction and the intermediate member 302 against the tubular wall 304 in the opposite direction to bend the tubular wall 304, to thereby constrict the tubular wall portion 304, as illustrated in FIGURE 29. To release the wall portion 304 the operation device moves the members 301-303 away from the tubular wall portion 304 to the position shown in FIGURE 28.

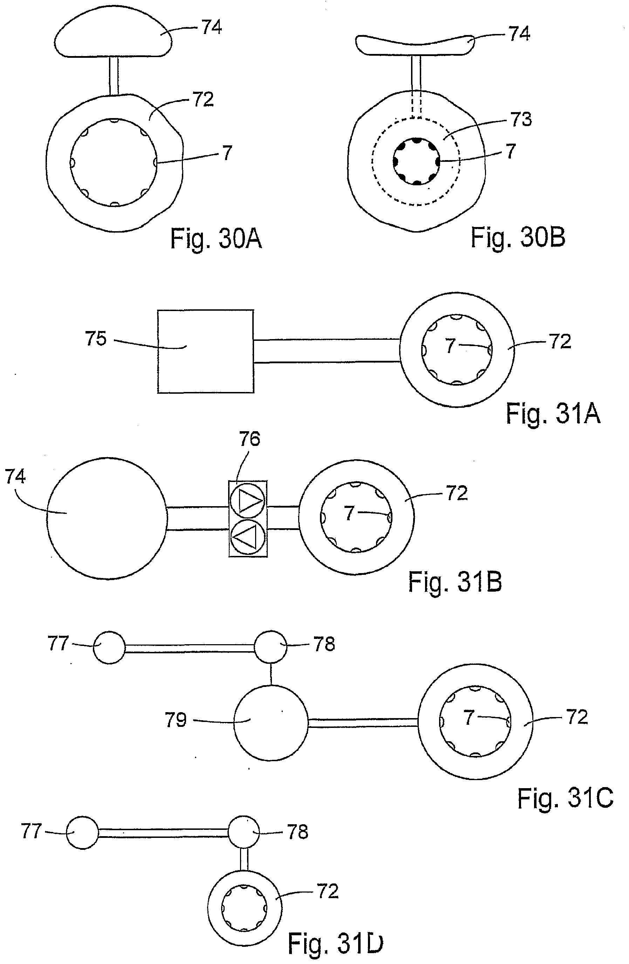

FIGURES 30A and 30B show a hydraulically operable elongated constriction device in the form of a band 72 having an expandable/contractible cavity 73, which is in fluid communication with an adjustable reservoir 74 containing hydraulic fluid. FIGURE 30A illustrates when the band is in a non-constriction state, whereas FIGURE 30B illustrates when the band is in a constriction state, in which the cavity 73 is expanded by hydraulic fluid supplied by the reservoir 74.

FIGURES 31A, 31B, 31C and 31D are block diagrams of four differently operated hydraulic constriction devices. FIGURE 31A shows the band 72 of FIGURE 30A, the cavity 73 of which is in fluid communication with a reservoir 75. FIGURE 31B shows the embodiment of FIGURE 30A, in which the cavity 73 of the band 72 is in fluid communication with the reservoir 74 via an operation device in the form of a two-way pump 76. FIGURE 31C shows an operation device in the form of a reverse servo system with a first closed system controlling a second system. The reverse servo system comprises an adjustable fluid supply reservoir 77 and an adjustable servo reservoir 78. The servo reservoir 78 controls a larger adjustable reservoir 79 which in connection with the band 72 applied around a portion of tubular tissue wall of a patient's biliary duct varies the volume of the cavity 73 of the band 72, which in turn varies the constriction of the wall portion. FIGURE 31D shows an embodiment identical to the embodiment of FIGURE 31C, except that the larger reservoir 79 is omitted. Instead, the servo reservoir 78 is in fluid communication with the cavity of the band 72.

In all of the above embodiments according to FIGURES 12A through 30B, stimulation devices may be provided to form constriction/stimulation units, in which the stimulation devices include a multiplicity of electrical elements 7 (indicated in FIGURES 12A - 15, 18, 20 - 23, 26 - 31B) positioned on the constriction devices.

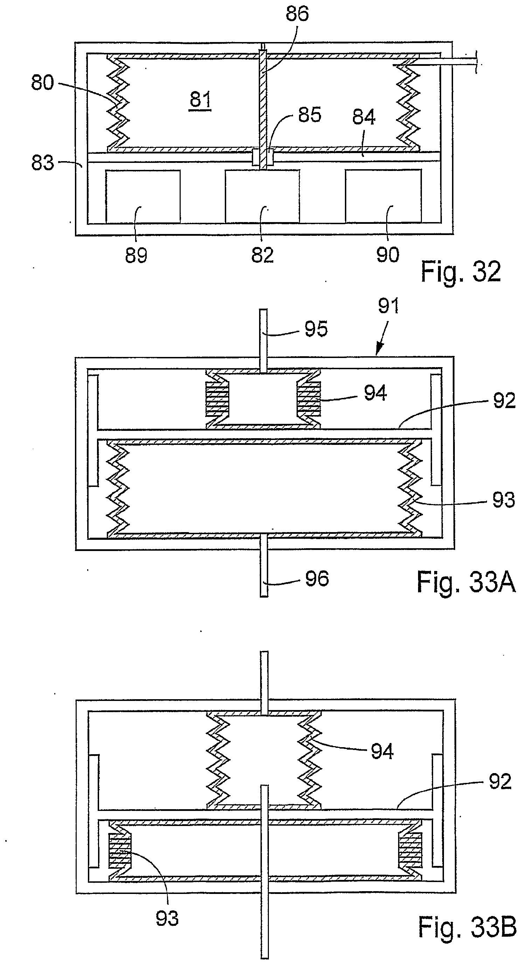

FIGURE 32 is a cross-sectional view of a fluid supply device including a bellows reservoir 80 defining a chamber 81, the size of which is variable by an operation device comprising a remote controlled electric motor 82. The reservoir 80 and the motor 82 are placed in a housing 83. Moving a large wall 84 varies the chamber 81. The wall 84 is secured to a nut 85, which is threaded on a rotatable spindle 86. The spindle 86 is rotated by the motor 82. A battery 89 placed in the housing 83 powers the motor 82. A signal receiver 90 for controlling the motor 82 is also placed in the housing 83. Alternatively, the battery 89 and the signal receiver 90 may be mounted in a separate place. The motor 82 may also be powered with energy transferred from transmitted signals.

Where applicable, the fluid supply device of FIGURE 32 may be used for supplying hydraulic fluid for the operation of the constriction devices described in this specification. For example, the fluid supply device of FIGURE 32 may be substituted for the reservoir 74 in the embodiment according to FIGURE 30A.

FIGURES 33A and 33B show a reverse servo including a rectangular housing 91 and an intermediate wall 92, which is movable in the housing 91. A relatively large, substantially cylindrical bellows reservoir 93 is arranged in the housing 91 and is joined to the movable intermediate wall 92. Another cylindrical bellows reservoir 94, which is substantially smaller than reservoir 93, is arranged in the housing 91 at the other side of the intermediate wall 92 and is also joined to the wall 92. The small bellows reservoir 94 has a fluid supply pipe 95 and the large bellows reservoir 93 has a fluid supply pipe 96.

Referring to FIGURE 33A, when a small amount of hydraulic fluid is conducted through the supply pipe 95 into the small bellows reservoir 94, the small bellows reservoir 94 expands and pushes the movable intermediate wall 92 towards the large bellows reservoir 93. As a result, the large bellows reservoir 93 is contracted by the intermediate wall 92, whereby a large amount of hydraulic fluid is forced out of the large bellows reservoir 93 through the supply pipe 96, as shown in FIGURE 33B.

For example, the reverse servo of FIGURES 33A and 33B may be used in the embodiment of FIGURE 31C, wherein the small bellows reservoir 94 corresponds to the small servo reservoir 78 and the large bellows reservoir 93 corresponds to the large reservoir 79. Also, the reverse servo of FIGURES 33A and 33B may be used in the embodiment of FIGURE 30A and 30B, wherein the small bellows reservoir 94 is connected to the adjustable reservoir 74, and the large bellows reservoir 93 is connected to the cavity 73 of the band 72.

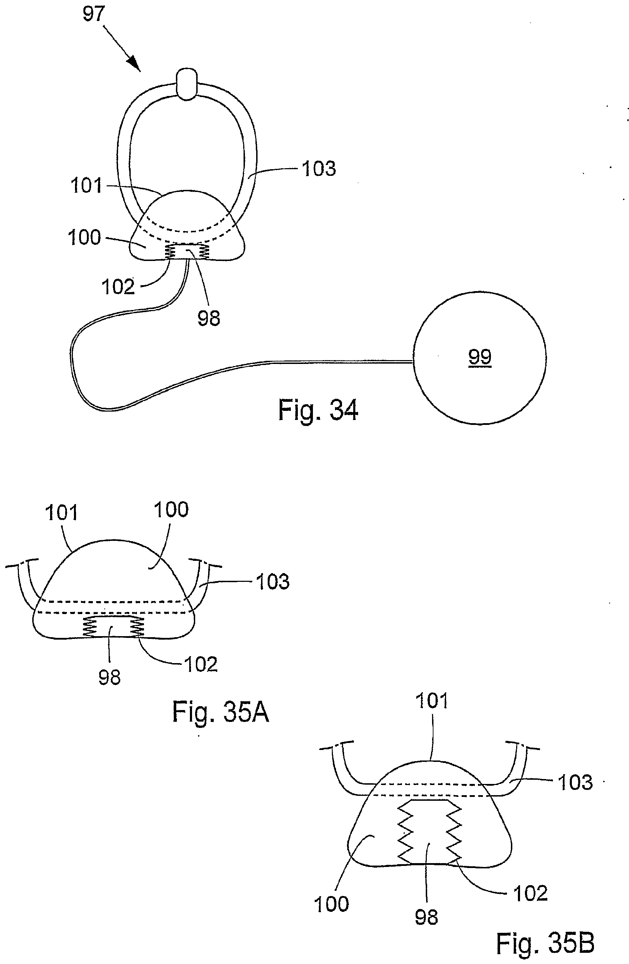

FIGURE 34 schematically shows a hydraulically operable constriction device 97 of the apparatus of the invention, which is similar to the embodiment shown in FIGURE 30A, except that the hydraulic system is designed differently. Thus, the constriction device 97 includes a relatively small inflatable cavity 98, which is in fluid communication with a reservoir 99 containing hydraulic fluid, and a relatively large cavity 100, which is displaceable by small cavity 98. Small cavity 98 is adapted to displace large cavity 100 to constrict the patient's tubular wall portion when small cavity 98 is inflated and to displace large cavity 100 to release the wall portion when small cavity 98 is deflated. Thus, a relatively small addition of hydraulic fluid from reservoir 99 to small cavity 98 causes a relatively large increase in the constriction of the wall portion.

Large cavity 100 is defined by a contraction element in the form of a big balloon 101, which may be connected to an injection port (not shown) for calibration of the volume of large cavity 100. Adding fluid to or withdrawing fluid from the injection port with the aid of a syringe calibrates the volume of balloon 101. Small cavity 98 is defined by a small bellows 102 attached to an annular frame 103 of constriction device 97 and at the opposite end is attached to balloon 101.

FIGURES 35A and 35B schematically illustrate the operation of constriction device 97, when annular frame 103 is applied around the tubular wall portion of the patient's biliary duct. Referring to FIGURE 35A, when small cavity 98 is deflated bellows 102 pulls balloon 101 inwardly into annular frame 103, so that constriction device 97 constricts the wall portion. Referring to FIGURE 35B, when small cavity 98 is inflated bellows 102 pulls balloon 101 out of annular frame 103, so that constriction device 97 releases the wall portion.

As mentioned above, the constriction device and stimulation device can co-operate to actively move the bile and/or gallstones in the lumen of a patient's biliary duct. This can be achieved using the constriction/stimulation unit shown in FIGURE 2. Thus, in accordance with a first cooperation option, the clamping elements 5, 6 of the constriction device constricts the wall portion 8 without completely closing the lumen, whereby the movement of bile and/or gallstones in the biliary duct is restricted, and the control device 4 controls the electrical elements 7 to progressively stimulate the constricted wall portion in the downstream or upstream direction of the lumen to cause progressive contraction of the wall portion 8 to move the bile and/or gallstones in the lumen.

In accordance with a second cooperation option, the constriction device constricts the wall portion so that the movement of bile and/or gallstones in the biliary duct is restricted, and the control device 4 controls a few electrical elements 7 at one end of the elongate clamping elements 5, 6 to stimulate the constricted wall portion 8 to close the lumen either at an upstream end or a downstream end of the wall portion 8. With the lumen closed in this manner, the control device 4 controls the constriction device to increase the constriction of the wall portion, whereby the bile and/or gallstones in the lumen is moved downstream or upstream of the wall portion 8.

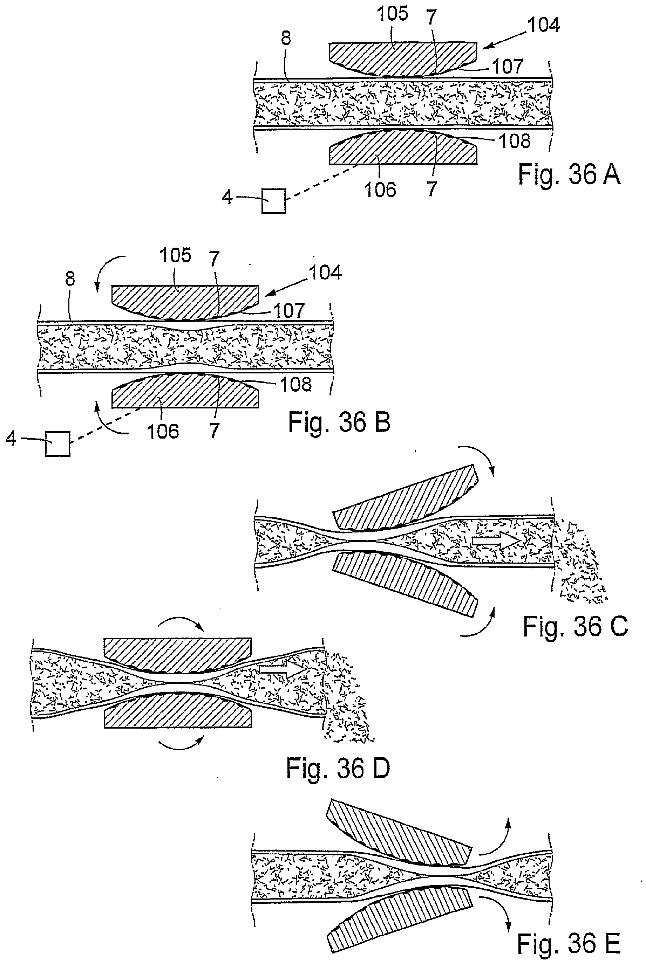

In another embodiment of the invention for performing the second cooperation option, the constriction device constricts the wall portion so that the movement of bile and/or gallstones in the biliary duct is restricted, and the control device 4 controls the stimulation device to stimulate the constricted wall portion while the constriction device varies the constriction of the different areas of the wall portion, such that the wall portion is progressively constricted in the downstream or upstream direction of the lumen. FIGURES 36A - 36E show different operation stages of such an alternative embodiment, which comprises a constriction device 104 including two elongate constriction elements 105, 106 having convex surfaces 107, 108 that abut a length of the wall portion 8 on mutual sides thereof, and a multiplicity of electrical elements 7 (such as electrodes) that are positioned on the convex surfaces 107, 108. The control device 4 controls the electrical elements 7 during operation of the constriction device 104 and controls the elongate constriction elements 105, 106 to move relative to the tubular wall portion 8 so that the constriction elements 105, 106 progressively constrict the wall portion 8, as appears from FIGURES 36A to 36D.

Thus, in an initial position of the constriction elements 105, 106 shown in FIGURE 36A, the wall portion is not constricted by the constriction elements 105, 106 and the electrical elements 7 are not energized. Starting from this initial position, the control device 4 controls the constriction elements 105, 106 to swing the left ends of the constriction elements 105, 106 toward the wall portion (indicated by arrows) to constrict the tubular wall portion 8, see FIGURE 36B, while energizing the electrical elements 7, so that the electrical elements 7 that contact the wall portion 8 contract the latter. FIGURE 36C shows how the lumen of the tubular wall portion 8 is completely closed by the thickened wall portion 8. Then, as shown in FIGURE 36C, the control device 4 controls the constriction elements 105, 106 to move so that their right ends are moving towards each other (indicated by arrows), while the convex surfaces 107, 108 of the constriction elements 105, 106 are rolling on each other with the contracted wall portion 8 between them, see FIGURE 36D. As a result, the bodily matter in the lumen of the biliary duct is forced to the right (indicated by a white arrow). When the constriction elements 105, 106 have rolled on each other to the position shown in FIGURE 36E, the control device 4 controls the right ends of the constriction elements 105, 106 to move away from each other (indicated by arrows in FIGURE 36E) to the initial position shown in FIGURE 36A. The operation stages described according to FIGURES 36A to 36E can be cyclically repeated a number of times until the desired amount of bodily matter has been moved in the lumen of the biliary duct in a peristaltic manner.

Alternatively, only one of the constriction elements 105, 106 can be provided with a convex surface, whereas the other constriction element has a plane surface that abuts the wall portion. It is also possible to use a single constriction element with a convex surface that presses the tubular portion 8 of the biliary duct against bone or other tissue of the patient.

In the embodiment according to FIGURES 36A to 36E, the control device 4 may control the electrical elements 7 to progressively stimulate the constricted wall portion 8 to cause progressive contraction thereof in harmony with the movement of the elongate constriction elements 105, 106, as the convex surfaces 107, 108 of the constriction elements 105, 106 are rolling on each other.

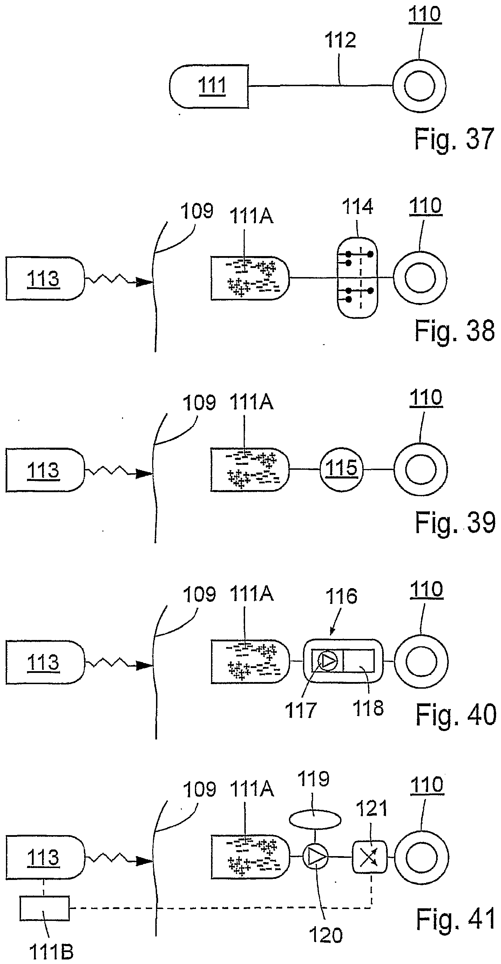

.FIGURE 37 schematically shows a general embodiment of the apparatus of the invention, in which energy is transferred to energy consuming components of the apparatus implanted in the patient. The apparatus of FIGURE 37 comprises an implanted constriction/stimulation unit 110, which is operable to gently constrict a portion of a tubular tissue wall of a patient's biliary duct and to stimulate different areas of the constricted portion to cause contraction of the wall portion. The constriction device of the constriction/stimulation unit 110 is capable of performing a reversible function, *i.e.,* to constrict and release the wall portion, so that the constriction/stimulation unit 110 works as an artificial sphincter.

A source of energy 111 is adapted to supply energy consuming components of the constriction/stimulation unit 110 with energy via a power supply line 112. A wireless remote control or a subcutaneously implanted switch operable by the patient to switch on or off the supply of energy from the source of energy may be provided. The source of energy may be an implantable permanent or rechargeable battery, or be included in an external energy-transmission device, which may be operable directly by the patient or be controlled by a remote control operable by the patient to transmit wireless energy to the energy consuming components of the constriction/stimulation unit. Alternatively, the source of energy may comprise a combination of an implantable rechargeable battery, an external energy-transmission device and an implantable energy-transforming device for transforming wireless energy transmitted by the external energy-transmission device into electric energy for the charge of the implantable rechargeable battery.

FIGURE 38 shows a special embodiment of the general embodiment of FIGURE 37 having some parts implanted in a patient and other parts located outside the patient's body. Thus, in FIGURE 38 all parts placed to the right of the patient's skin 109 are implanted and all parts placed to the left of the skin 109 are located outside the patient's body. An implanted energy-transforming device 111A of the apparatus is adapted to supply energy consuming components of the constriction/stimulation unit 110 with energy via the power supply line 112. An external energy-transmission device 113 of the apparatus includes a wireless remote control transmitting a wireless signal, which is received by a signal receiver incorporated in the implanted energy-transforming device 111A. The implanted energy-transforming device 111A transforms energy from the signal into electric energy, which is supplied via the power supply line 112 to the constriction/stimulation unit 110.

The apparatus of FIGURE 38 may also include an implanted rechargeable battery for energizing energy consuming implanted components of the apparatus. In this case, the implanted energy-transforming device 111A also charges the battery with electric energy, as the energy-transforming device transforms energy from the signal into the electric energy.

A reversing device in the form of an electric switch 114, such as a microprocessor, is implanted in the patient for reversing the constriction device of the constriction/stimulation unit 110. The wireless remote control of the external energy-transmission device 113 transmits a wireless signal that carries energy and the implanted energy-transforming device 111A transforms the wireless energy into a current for operating the switch 114. When the polarity of the current is shifted by the energy-transforming-device 111A the switch 114 reverses the function performed by the constriction device of the constriction/stimulation unit 110.

FIGURE 39 shows an embodiment of the invention including the energy-transforming device 111A, the constriction/stimulation unit 110 and an implanted operation device in the form of a motor 115 for operating the constriction device of the constriction/stimulation unit 110. The motor 115 is powered with energy from the energy-transforming device 111A, as the remote control of the external energy-transmission device113 transmits a wireless signal to the receiver of the energy-transforming device 111A.

FIGURE 40 shows an embodiment of the invention including the energy-transforming device 111A, the constriction/stimulation unit 110 and an implanted assembly 116 including a motor/pump unit 117 and a fluid reservoir 118. In this case the constriction device of the constriction/stimulation unit 110 is hydraulically operated, *i.e.,* hydraulic fluid is pumped by the motor/pump unit 117 from the reservoir 118 to the constriction/stimulation unit 110 to constrict the wall portion, and hydraulic fluid is pumped by the motor/pump unit 117 back from the constriction/stimulation unit 110 to the reservoir 118 to release the wall portion. The implanted energy-transforming device 111A transforms wireless energy into a current, for powering the motor/pump unit 117.

FIGURE 41 shows an embodiment of the invention comprising the external energy-transmission device 113.that controls the control unit 122 to reverse the motor 115 when needed, the constriction/stimulation unit 110, the constriction device of which is hydraulically operated, and the implanted energy-transforming device 111A, and further comprising an implanted hydraulic fluid reservoir 119, an implanted motor/pump unit 120, an implanted reversing device in the form of a hydraulic valve shifting device 121 and a separate external wireless remote control 111B. The motor of the motor/pump unit 120 is an electric motor. In response to a control signal from the wireless remote control of the external energy-transmission device 113, the implanted energy-transforming device 111A powers the motor/pump unit 120 with energy from the energy carried by the control signal, whereby the motor/pump unit 120 distributes hydraulic fluid between the reservoir 119 and the constriction device of the constriction/stimulation unit 110. The remote control 111B controls the shifting device 121 to shift the hydraulic fluid flow direction between one direction in which the fluid is pumped by the motor/pump unit 120 from the reservoir 119 to the constriction device of the constriction/stimulation unit 110 to constrict the wall portion, and another opposite direction in which the fluid is pumped by the motor/pump unit 120 back from the constriction device of the constriction/stimulation unit 110 to the reservoir 119 to release the wall portion.

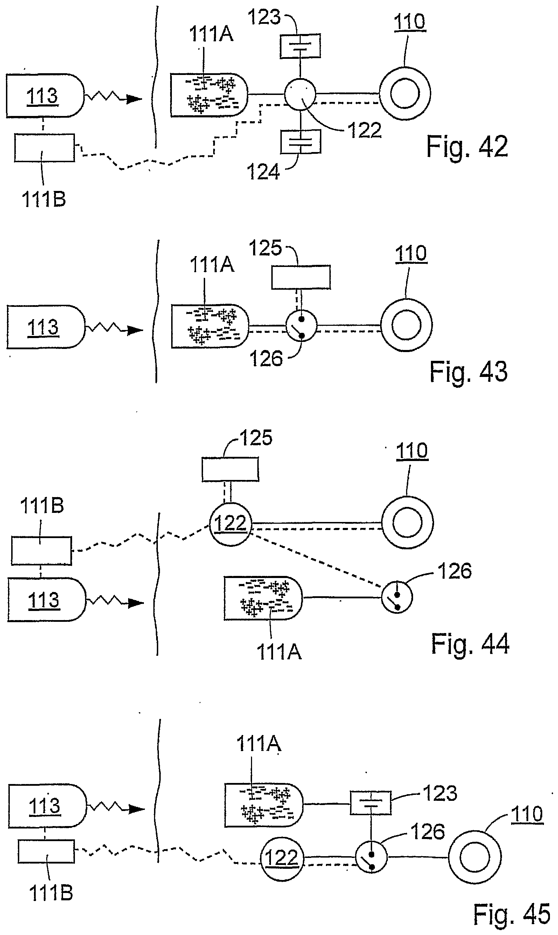

FIGURE 42 shows an embodiment of the invention including the energy-transforming device 111A and the constriction/stimulation unit 110. A control unit 122, an accumulator 123 and a capacitor 124 are also implanted in the patient. A separate external wireless remote control 111B controls the control unit 122. The control unit 122 controls the energy-transforming device 111A to store electric energy in the accumulator 123, which supplies energy to the constriction/stimulation unit 110. In response to a control signal from the wireless remote control 111B, the control unit 122 either releases electric energy from the accumulator 123 and transfers the released energy via power lines, or directly transfers electric energy from the energy-transforming device 111A via the capacitor 124, which stabilises the electric current, for the operation of the constriction/stimulation unit 110.

In accordance with one alternative, the capacitor 124 in the embodiment of FIGURE 42 may be omitted. In accordance with another alternative, the accumulator 123 in this embodiment may be omitted.

FIGURE 43 shows an embodiment of the invention including the energy-transforming device 111A, the constriction/stimulation unit 110. A battery 125 for supplying energy for the operation of the constriction/stimulation unit 110 and an electric switch 126 for switching the operation of the constriction/stimulation unit 110 are also implanted in the patient. The switch 126 is operated by the energy supplied by the energy-transforming device 111A to switch from an off mode, in which the battery 125 is not in use, to an on mode, in which the battery 125 supplies energy for the operation of the constriction/stimulation unit 110.

FIGURE 44 shows an embodiment of the invention identical to that of FIGURE 43, except that a control unit 122 also is implanted in the patient. A separate external wireless remote control 111B controls the control unit 122. In this case, the switch 126 is operated by the energy supplied by the energy-transforming device 111A to switch from an off mode, in which the wireless remote control 111B is prevented from controlling the control unit 122 and the battery 125 is not in use, to a standby mode, in which the remote control 111B is permitted to control the control unit 122 to release electric energy from the battery 125 for the operation of the constriction/stimulation unit 110.

FIGURE 45 shows an embodiment of the invention identical to that of FIGURE 44, except that the accumulator 123 is substituted for the battery 125 and the implanted components are interconnected differently. In this case, the accumulator 123 stores energy from the energy-transforming device 111A. In response to a control signal from the wireless remote control 111B, the implanted control unit 122 controls the switch 126 to switch from an off mode, in which the accumulator 123 is not in use, to an on mode, in which the accumulator 123 supplies energy for the operation of the constriction/stimulation unit 110.

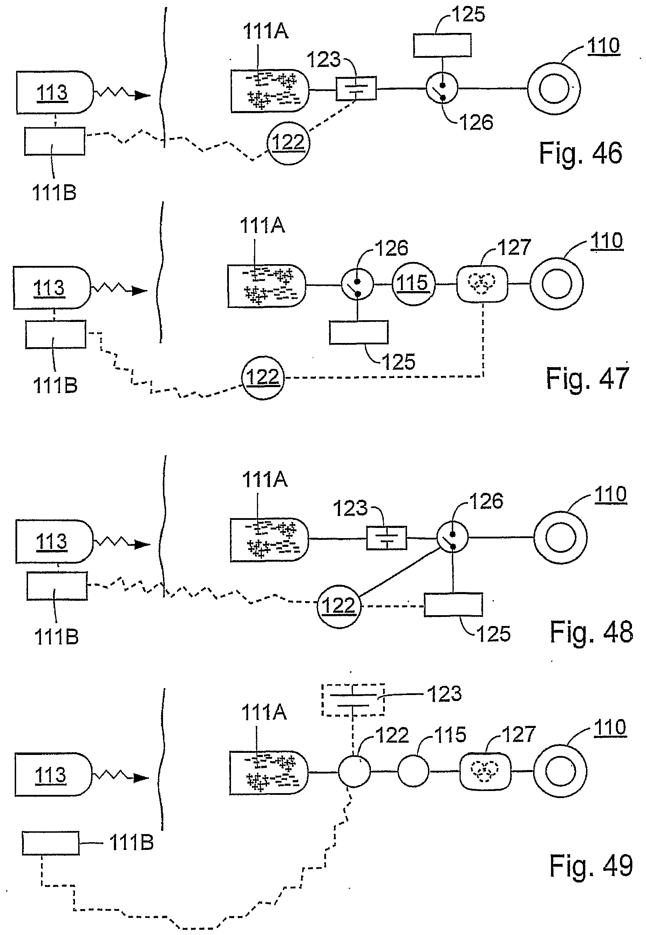

FIGURE 46 shows an embodiment of the invention identical to that of FIGURE 45, except that the battery 125 also is implanted in the patient, and the implanted components are interconnected differently. In response to a control signal from the wireless remote control 111B, the implanted control unit 122 controls the accumulator 123, which may be a capacitor, to deliver energy for operating the switch 126 to switch from an off mode, in which the battery 125 is not in use, to an on mode, in which the battery 125 supplies electric energy for the operation of the constriction/stimulation unit 110.

Alternatively, the switch 126 may be operated by energy supplied by the accumulator 123 to switch from an off mode, in which the wireless remote control 111B is prevented from controlling the battery 125 to supply electric energy and the battery 125 is not in use, to a standby mode, in which the wireless remote control 111B is permitted to control the battery 125 to supply electric energy for the operation of the constriction/stimulation unit 110.

FIGURE 47 shows an embodiment of the invention identical to that of FIGURE 43, except that a motor 115, a mechanical reversing device in the form of a gearbox 127 and a control unit 122 for controlling the gearbox 127 also are implanted in the patient. A separate external wireless remote control 111B controls the implanted control unit 122 to control the gearbox 127 to reverse the function performed by the constriction device (mechanically operated) of the constriction/stimulation unit 110.

FIGURE 48 shows an embodiment of the invention identical to that of FIGURE 46, except that the implanted components are interconnected differently. Thus, in this case, the battery 125 powers the control unit 122 when the accumulator 123, suitably a capacitor, activates the switch 126 to switch to an on mode. When the switch 126 is in its on mode the control unit 122 is permitted to control the battery 125 to supply, or not supply, energy for the operation of the constriction/stimulation unit 110.

FIGURE 49 shows an embodiment of the invention identical to that of FIGURE 39, except that a gearbox 127 that connects the motor 115 to the constriction/stimulation unit 110, and a control unit 122 that controls the energy-transforming device 111A to power the motor 115 also are implanted in the patient. There is a separate external wireless remote control 111B that controls the control unit 122 to reverse the motor 115 when needed.

Optionally, the accumulator 123 shown in FIGURE 42 may be provided in the embodiment of FIGURE 49, wherein the implanted control unit 122 controls the energy-transforming device 111A to store the transformed energy in the accumulator 123. In response to a control signal from the wireless remote control 111B, the control unit 122 controls the accumulator 123 to supply energy for the operation of the constriction/stimulation unit 110.

Those skilled in the art will realise that the above various embodiments according to FIGURES 38-49 could be combined in many different ways. For example, the energy operated switch 114 could be incorporated in any of the embodiments of FIGURES 39, 42-49, the hydraulic shifting device 121 could be incorporated in the embodiment of FIGURE 40, and the gearbox 127 could be incorporated in the embodiment of FIGURE 39. The switch 114 may be of a type that includes electronic components, for example a microprocessor, or a FGPA (Field Programmable Gate Array) designed for switching. Alternatively, however, the energy operated switch 114 may be replaced by a subcutaneously implanted push button that is manually switched by the patient between "on" and"off".

Alternatively, a permanent or rechargeable battery may be substituted for the energy-transforming devices 111A of the embodiments shown in FIGURES 38-49.

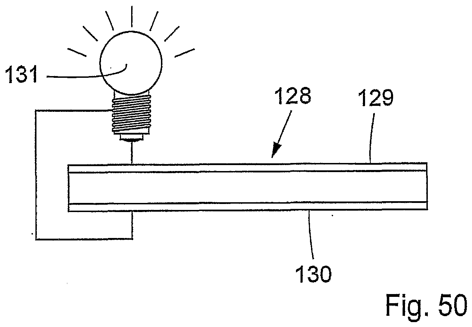

FIGURE 50 shows the energy-transforming device in the form of an electrical junction element 128 for use in any of the above embodiments according to FIGURES 37-49. The element 128 is a flat p-n junction element comprising a p-type semiconductor layer 129 and an n-type semiconductor layer 130 sandwiched together. A light bulb 131 is electrically connected to opposite sides of the element 128 to illustrate how the generated current is obtained. The output of current from such a p-n junction element 128 is correlated to the temperature. See the formula below.

$$I = I0\ (\exp(qV/kT)\text{-}1)$$

Where

I is the external current flow,
I0 is the reverse saturation current,
q is the fundamental electronic charge of $1.602 \times 10\text{-}19$ coulombs,
V is the applied voltage,
k is the Boltzmann constant, and
T is the absolute temperature.

Under large negative applied voltage (reverse bias), the exponential term becomes negligible compared to 1.0, and I is approximately -10. I0 is strongly dependent on the temperature of the junction and hence on the intrinsic-carrier concentration. I0 is larger for materials with smaller bandgaps than for those with larger bandgaps. The rectifier action of the diode, that is, its restriction of current flow to only one direction, is in this particular embodiment the key to the operation of the p-n junction element 128.

The alternative way to design a p-n junction element is to deposit a thin layer of semiconductor onto a supporting material which does not absorb the kind of energy utilised in the respective embodiments. For use with wirelessly transmitted energy in terms of light waves, glass could be a suitable material. Various materials may be used in the semiconductor layers, such as, but not limited to, cadmium telluride, copper-indium-diselenide and silicon. It is also possible to use a multilayer structure with several layers of p and n-type materials to improve efficiency.

The electric energy generated by the p-n junction element 128 could be of the same type as generated by solar cells, in which the negative and positive fields create a direct current. Alternatively, the negative and positive semiconductor layers may change polarity following the transmitted waves, thereby generating the alternating current.

The p-n junction element 128 is designed to make it suited for implantation. Thus, all the external surfaces of the element 128 in contact with the human body are made of a biocompatible material. The p-n junction semiconductors are designed to operate optimally at a body temperature of 37°C because the current output, which should be more than 1 $\mu$A, is significantly dependent upon such temperature, as shown above. Since both the skin and subcutis absorb energy, the relation between the sensitivity or working area of the element 128 and the intensity or strength of the wireless energy-transmission is considered. The p-n junction element 128 preferably is designed flat and small. Alternatively, if the element 128 is made in larger sizes it should be flexible, in order to adapt to the patient's body movements. The volume of the element 128 should be kept less than 2000 cm$^3$.

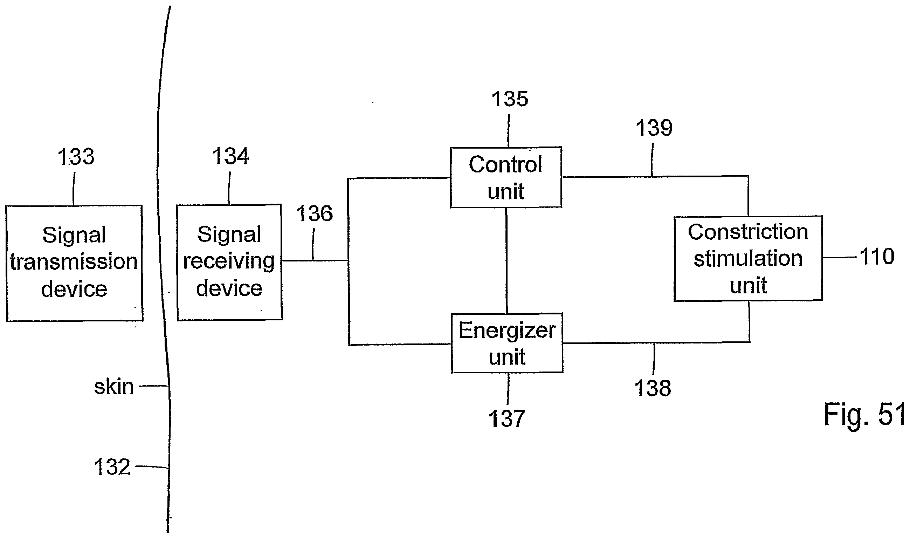

FIGURE 51 shows basic parts of a remote control of the apparatus of the invention for controlling the constriction/stimulation unit 110. In this case, the stimulation device of the constriction/stimulation unit stimulates the wall portion with electric pulses. The remote control is based on wireless transmission of electromagnetic wave signals, often of high frequencies in the order of 100 kHz - 1 gHz, through the skin 132 of the patient. IN FIGURE 51, all parts placed to the left of the skin 132 are located outside the patient's body and all parts placed to the right of the skin 132 are implanted.

An external signal-transmission device 133 is to be positioned close to a signal-receiving device 134 implanted close to the skin 132. As an alternative, the signal-receiving device 134 may be placed for example inside the abdomen of the patient. The signal-receiving device 134 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The signal transmission device 133 comprises a coil having about the same size as the coil of the signal-receiving device 134 but wound with a thick wire that can handle the larger currents that is necessary. The coil of the signal transmission device 133 is tuned to the same specific high frequency as the coil of the signal-receiving device 134.

The signal-transmission device 133 is adapted to send digital information via the power amplifier and signal-receiving device 134 to an implanted control unit 135. To avoid that accidental random high frequency fields trigger control commands, digital signal codes are used. A conventional keypad placed on the signal transmission device 133 is used to order the signal transmission device 133 to send digital signals for the control of the constriction/stimulation unit. The signal transmission device 133 starts a command by generating a high frequency signal. After a short time, when the signal has energized the implanted parts of the control system, commands are sent to operate the constriction device of the constriction/stimulation unit 110 in predefined steps. The commands are sent as digital packets in the form illustrated below.

| Start pattern, | Command, | Count, | Checksum, |
|---|---|---|---|
| 8 bits | 8 bits | 8 bits | 8 bits |

The commands are sent continuously during a rather long time period (e.g., about 30 seconds or more). When a new constriction or release step is desired, the Count byte is increased by one to allow the implanted control unit 135 to decode and understand that another step is demanded by the signal transmission device 133. If any part of the digital packet is erroneous, its content is simply ignored.

Through a line 136, an implanted energizer unit 137 draws energy from the high frequency electromagnetic wave signals received by the signal-receiving device 134. The energizer unit 137 stores the energy in a source of energy, such as a large capacitor, powers the control unit 135 and powers the constriction/stimulation unit 110 via a line 138.

The control unit 135 comprises a demodulator and a microprocessor. The demodulator demodulates digital signals sent from the signal transmission device 133. The microprocessor receives the digital packet, decodes it and sends a control signal via a signal line 139 to control the constriction device of the constriction/stimulation unit 110 to either constrict or release the wall portion of the patient's biliary duct depending on the received command code.

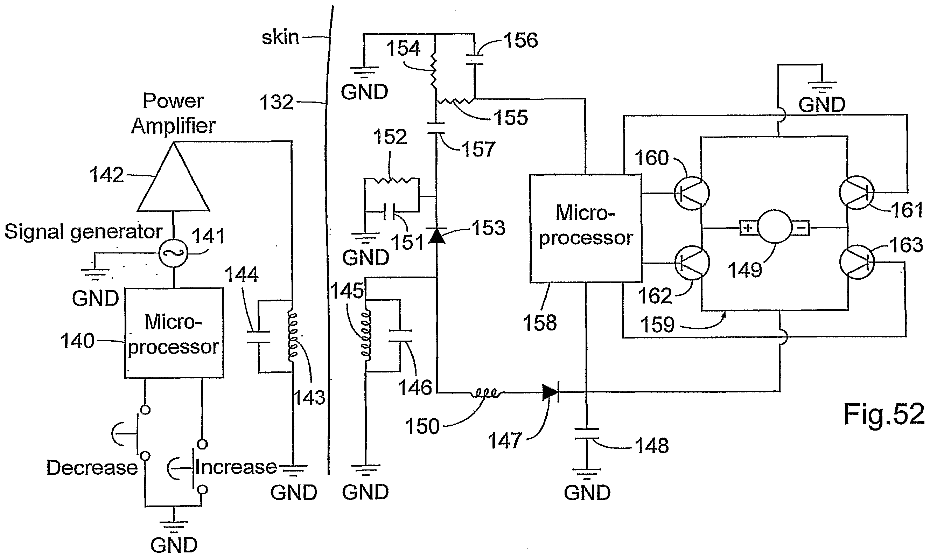

FIGURE 52 shows a circuitry of an embodiment of the invention, in which wireless energy is transformed into a current. External components of the circuitry include a microprocessor 140, a signal generator 141 and a power amplifier 142 connected thereto. The microprocessor 140 is adapted to switch the signal generator 141 on/off and to modulate signals generated by the signal generator 141 with digital commands. The power amplifier 142 amplifies the signals and sends them to an external signal-transmitting antenna coil 143. The antenna coil 143 is connected in parallel with a capacitor 144 to form a resonant circuit tuned to the frequency generated by the signal generator 141.

Implanted components of the circuitry include a signal receiving antenna coil 145 and a capacitor 146 forming together a resonant circuit that is tuned to the same frequency as the transmitting antenna coil 143. The signal receiving antenna coil 145 induces a current from the received high frequency electromagnetic waves and a rectifying diode 147 rectifies the induced current, which charges a storage capacitor 148. The storage capacitor 148 powers a motor 149 for driving the constriction device of the constriction/stimulation unit 110. A coil 150 connected between the antenna coil 145 and the diode 147 prevents the capacitor 148 and the diode 147 from loading the circuit of the signal-receiving antenna 145 at higher frequencies. Thus, the coil 150 makes it possible to charge the capacitor 148 and to transmit digital information using amplitude modulation.

A capacitor 151 and a resistor 152 connected in parallel and a diode 153 form a detector used to detect amplitude modulated digital information. A filter circuit is formed by a resistor 154 connected in series with a resistor 155 connected in series with a capacitor 156 connected in series with the resistor 154 via ground, and a capacitor 157, one terminal of which is connected between the resistors 154,155 and the other terminal of which is connected between the diode 153 and the circuit formed by the capacitor 151 and resistor 152. The filter circuit is used to filter out undesired low and high frequencies. The detected and filtered signals are fed to an implanted microprocessor 158 that decodes the digital information and controls the motor 149 via an H-bridge 159 comprising transistors 160, 161, 162 and 163. The motor 149 can be driven in two opposite directions by the H-bridge 159.

The microprocessor 158 also monitors the amount of stored energy in the storage capacitor 148. Before sending signals to activate the motor 149, the microprocessor 158 checks whether the energy stored in the storage capacitor 148 is enough. If the stored energy is not enough to perform the requested operation, the microprocessor 158 waits for the received signals to charge the storage capacitor 148 before activating the motor 149.

Alternatively, the energy stored in the storage capacitor 148 may only be used for powering a switch, and the energy for powering the motor 149 may be obtained from another implanted energy source of relatively high capacity, for example a battery. In this case the switch is adapted to connect the battery to the motor 149 in an on mode when the switch is powered by the storage capacitor 148 and to keep the battery disconnected from the motor 149 in a standby mode when the switch is not powered.

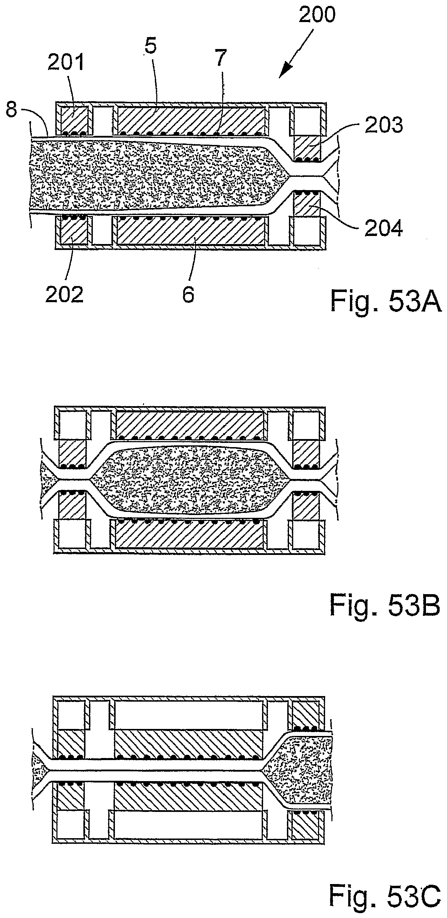

FIGURES 53A - 53C show an embodiment of the invention, which is similar to the embodiment of FIGURE 2, except that the constriction/stimulation unit, here denoted by reference numeral 200, is provided with additional clamping elements. The embodiment of FIGURES 53A - 53C is suited for actively moving the bile and/or gallstonesin the lumen of a patient's biliary duct. Thus, the constriction/stimulation unit 200 also includes a first pair of short clamping elements 201 and 202, and a second pair of short clamping elements 203 and 204, wherein the first and second pairs of clamping elements are positioned at mutual sides of the elongate clamping elements 5, 6. The two short clamping elements 201, 202 of the first pair are radially movable towards and away from each other between retracted positions (FIGURE 53A) and clamping positions (FIGURES 53B and 53C), and the two short clamping elements 203, 204 of the second pair are radially movable towards and away from each other between retracted positions (FIGURE 53C) and clamping positions (FIGURES 53A and 53B). The stimulation device 3 also includes electrical elements 7 positioned on the short clamping elements 201 - 204, so that the electrical elements 7 on one of the short clamping elements 201 and 203, respectively, of each pair of short elements face the electrical elements 7 on the other short clamping element 202 and 204, respectively, of each pair of short elements.

The constriction/stimulation unit 200 is applied on a wall portion 8 of a tubular tissue wall of a patient's biliary duct, so that the short clamping elements 201, 202 are positioned at an upstream end of the wall portion 8, whereas the short clamping elements 203, 204 202 are positioned at a downstream end of the wall portion 8. In FIGURES 53A to 53C the upstream end of the wall portion 8 is to the left and the downstream end of the wall portion 8 is to the right.

The control device 4 controls the pair of short clamping elements 201, 202, the pair of elongate clamping elements 5, 6 and the pair of short elements 203, 204 to constrict and release the wall portion 8 independently of one another. The control device also controls the electrical elements 7 on a clamping element that is constricting the wall portion to stimulate the constricted wall portion 8 with electric pulses to cause contraction of the wall portion 8, so that the lumen of the wall portion 8 is closed.

FIGURES 53A - 53C illustrate how the control device 4 controls the operation of the constriction/stimulation unit 200 to cyclically move bile and/or gallstones downstream in the lumen of the wall portion 8. Thus, in FIGURE 53A the short clamping elements 201, 202 and the elongate clamping elements 5, 6 are in their retracted positions, whereas the short clamping elements 203, 204 are in their clamping positions while the electrical elements 7 on elements 203, 204 electrically stimulate the wall portion 8. The electrical stimulation causes the wall portion 8 at the elements 203, 204 to thicken, whereby the lumen is closed. FIGURE 53B illustrates how also the short clamping elements 201, 202 have been moved radially inwardly to their clamping positions, while the electrical elements 7 on elements 201, 202 electrically stimulate the wall portion 8, whereby a volume of bile and/or gallstones are trapped in the lumen between the upstream and downstream ends of the wall portion 8. FIGURE 53C illustrates how initially the short clamping elements 203, 204 have been moved radially outwardly to their retracted positions, and then the elongate clamping elements 5, 6 have been moved radially inwardly to their clamping positions while the electrical elements 7 on elements 5, 6 electrically stimulate the wall portion 8. As a result, the bodily matter in the lumen between the upstream and downstream ends of the wall portion 8 has been moved downstream in the lumen Then, the control device 4 controls the constriction/stimulation unit 200 to assume the state shown in FIGURE 53A, whereby bodily matter may flow into and fill the lumen between the upstream and downstream ends of the wall portion 8, so that the cycle of the operation is completed.

Alternatively, the operation cycle of the constriction/stimulation unit 200 described above may be reversed, in order to move bodily matter upstream in the lumen. In this case, the control device 4 controls the short clamping elements 203, 204 to constrict the wall portion 8 at the downstream end thereof to restrict the flow in the lumen and controls the electric elements 7 to stimulate the constricted wall portion 8 with electric pulses at the downstream end to close the lumen. With the lumen closed at the downstream end of the constricted wall portion 8 and the short clamping elements 201, 202 in their retracted positions, as shown in FIGURE 53A, the control device 4 controls the elongate clamping elements 5, 6 to constrict the wall portion 8 between the upstream and downstream ends thereof. As a result, the fluid and/or other bodily matter contained in the wall portion 8 between the upstream and downstream ends thereof is moved upstream in the lumen.

Although FIGURES 53A - 53C disclose pairs of clamping elements, it should be noted that it is conceivable to design the constriction/stimulation unit 200 with only a single short clamping element 201, a single elongate clamping element 5 and a single short clamping element 203. In this case the bottom of the tubular wall portion 8 is supported by stationary elements of the constriction/stimulation unit 200 opposite to the clamping elements 201, 5, and 203.

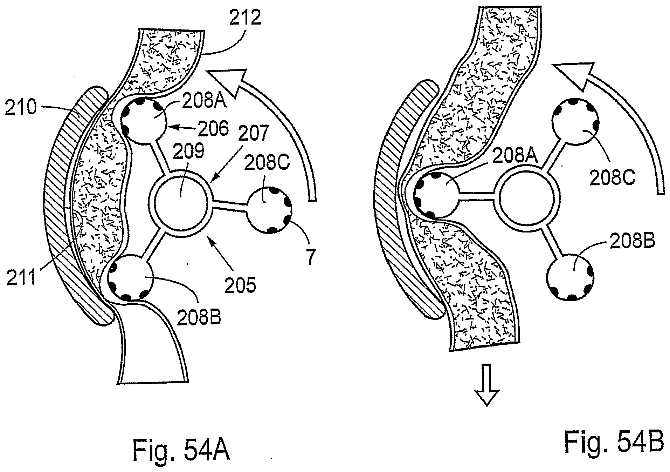

FIGURES 54A and 54B schematically show another embodiment of the invention, in which a constriction/stimulation unit 205 is designed for actively moving the bile and/or gallstones in the lumen of a patient's tubular biliary duct. The constriction device 206 of the constriction/stimulation unit 205 includes a rotor 207, which carries three cylindrical constriction elements 208A, 208B and 208C positioned equidistantly from the axis 209 of the rotor 207. The constriction elements 208A-208C may be designed as rollers. Each cylindrical element 208A-208C is provided with electrical elements 7. A stationary elongate support element 210 is positioned spaced from but close to the rotor 207 and has a part cylindrical surface 211 concentric with the axis 209 of the rotor 207. The constriction/stimulation unit 205 is applied on a patient's tubular biliary duct 212, so that the biliary duct 212 extends between the support element 210 and the rotor 207.

The control device 4 controls the rotor 207 of the constriction device to rotate, such that the constriction elements 208A-208C successively constrict wall portions of a series of wall portions of the tubular biliary duct 212 against the elongate support element 210. The electrical elements 7 of the constriction elements 208A-208C stimulate the constricted wall portions with electric pulses so that the wall portions thicken and close the lumen of the biliary duct 212. FIGURE 54A illustrates how the constriction element 208A has started to constrict the wall of the biliary duct 212 and how the lumen of the biliary duct 212 is closed with the aid of the electrical elements 7 on the constriction element 208A, whereas the constriction element 208B is about to release the biliary duct 212. FIGURE 54B illustrates how the constriction element 208A has advanced about halfway along the elongate support element 210 and moved the bodily matter in the lumen in a direction indicated by an arrow. The constriction element 208B has released the biliary duct 212, whereas the constriction element 208C is about to engage the biliary duct 212. Thus, the control device 4 controls the rotor 207 to cyclically move the constriction elements 208A-208C, one after the other, along the elongate support element 210, while constricting the wall portions of the biliary duct 212, so that the bodily matter in the biliary duct 212 is moved in a peristaltic manner.

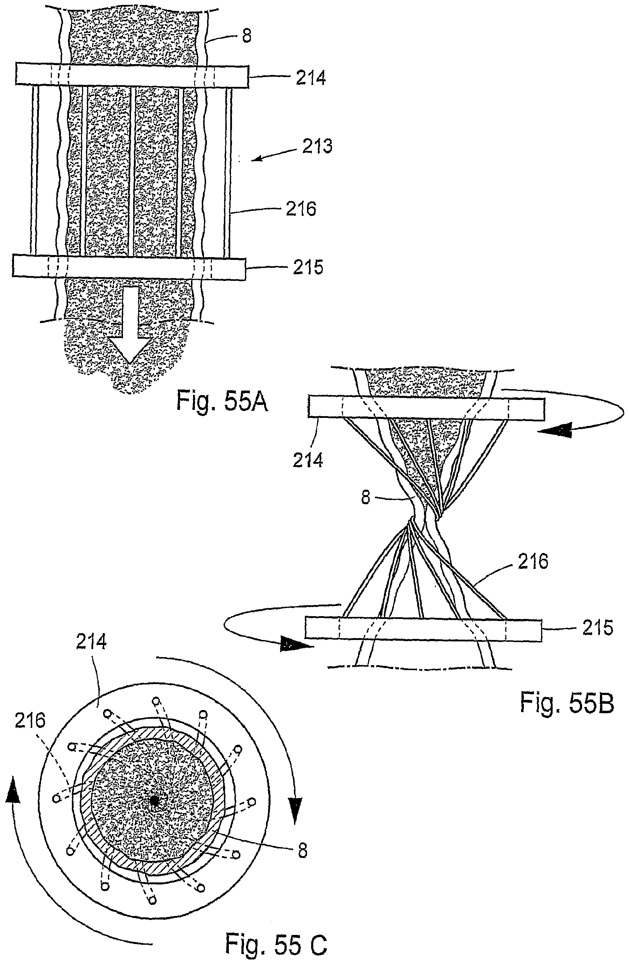

FIGURES 55A, 55B and 55C show another mechanically operable constriction device 213 for use in the apparatus of the invention. Referring to FIGURE 55A, the constriction device 213 includes a first ring-shaped holder 214 applied on a tubular biliary duct 8 of a patient and a second ring-shaped holder 215 also applied on the biliary duct 8 spaced apart from holder 214. There are elastic strings 216 (here twelve strings) that extend in parallel along the tubular biliary duct 8 and interconnect the two holders 213, 214 without contacting the biliary duct 8. FIGURE 55A illustrate an inactivated state of the constriction device 213 in which the biliary duct 8 is not constricted.

Referring to FIGURES 55B and 55C, when biliary duct 8 is to be constricted the ring-shaped holders 213 and 214 are rotated by an operation means (not shown) in opposite directions, whereby the elastic strings 216 constrict the biliary duct 8 in a manner that appears from FIGURES 55B and 55C. For the sake of clarity, only five strings 216 are shown in FIGURE 55B.

In accordance with the present invention, electrodes for electrically stimulating the biliary duct 8 to cause contraction of the wall of the biliary duct 8 are attached to the strings 216 (not shown in FIGURES 55A-55C).

While the invention has been described in connection with what is presently considered to be the most practical and preferred embodiment, it is to be understood that the invention is not to be limited to the disclosed embodiment, but on the contrary, is intended to cover various modifications and equivalent arrangements included within the spirit and scope of the appended claims.

ITEMIZED LIST OF EXAMPLES:

1. An apparatus for controlling the movement of bile and/or gallstones in the biliary ducts of a patient, comprising:

an implantable constriction device for gently constricting at least one portion of the tissue wall to influence the movement of bile and/or gallstones in the biliary duct,
a stimulation device for stimulating the wall portion of the tissue wall, and
a control device for controlling said stimulation device to stimulate the wall portion, as said constriction device constricts the wall portion, to cause contraction of the wall portion to further influence the movement of bile and/or gallstones in the biliary duct.

2. The apparatus according to example 1, wherein said constriction device is adapted to constrict the wall portion to at least restrict the movement of bile and/or gallstones in the biliary duct, and said control device controls said stimulation device to cause contraction of the constricted wall portion, so that the movement of bile and/or gallstones in the biliary duct is at least further restricted.

3. The apparatus according to example 2, wherein said constriction device is adapted to constrict the wall portion to a constricted state in which the blood circulation in the constricted wall portion is substantially unrestricted and the movement of bile and/or gallstones in the biliary duct is at least restricted, and said control device controls said stimulation device to cause contraction of the wall portion, so that the movement of bile and/or gallstones in the biliary duct is at least further restricted when the wall portion is kept by said constriction device in the constricted state.

4. The apparatus according to example 1, wherein said control device controls said constriction device to adjust the constriction of the patient's wall portion.

5. The apparatus according to example 4, wherein said control device controls said constriction and stimulation devices independently of each other.

6. The apparatus according to example 5, wherein said control device simultaneously controls said constriction device and said stimulation device.

7. The apparatus according to example 4, wherein said control device controls said stimulation device to stimulate the wall portion, while said control device controls said constriction device to change the constriction of the wall portion.

8. The apparatus according to example 4, wherein said control device is adapted to calibrate said constriction device by controlling said stimulation device to stimulate the wall portion while controlling said constriction device to adjust the constriction of the wall portion until the desired restriction of the movement of bile and/or gallstones in the biliary duct obtained.

9. The apparatus according to example 4, wherein said control device controls said stimulation device not to stimulate the wall portion while said control device controls said constriction device to change the constriction of the wall portion.

10. The apparatus according to example 4, wherein said control device controls said constriction device to constrict the wall portion, such that the movement of bile and/or gallstones in the biliary duct restricted but not stopped, and controls said stimulation device to stimulate the constricted wall portion to cause contraction thereof, such that the movement of bile and/or gallstones in the biliary duct is further restricted but not stopped.

11. The apparatus according to example 10, wherein said control device controls said stimulation device to adjust the intensity of the stimulation of the wall portion in response to a sensed physical parameter of the patient.

12. The apparatus according to example 10, wherein said control device controls said stimulation device to adjust the intensity of the stimulation of the wall portion in response to a sensed functional parameter of the apparatus.

13. The apparatus according to example 10, wherein said control device controls said stimulation device in a first mode to stimulate the constricted wall portion to further restrict but not stop the movement of bile and/or gallstones in the biliary duct and controls said stimulation device in a second mode to cease the stimulation of the wall portion to increase the movement of bile and/or gallstones in the biliary duct.

14. The apparatus according to example 13, wherein said control device in said second mode controls said stimulation device to cease the stimulation of the wall portion and controls said constriction device to release the wall portion to restore movement of bile and/or gallstones in the biliary duct.

15. The apparatus according to example 4, wherein said control device controls said constriction device to constrict the wall portion, such that the movement of bile and/or gallstones in the biliary duct is restricted but not stopped, and controls said stimulation device to stimulate the constricted wall portion to cause contraction thereof, such that the movement of bile and/or gallstones in the biliary duct is stopped.

16. The apparatus according to example 15, wherein said control device controls said stimulation device in a first mode to stimulate the constricted wall portion to stop the movement of bile and/or gallstones in the biliary duct and controls said stimulation device in a second mode to cease the stimulation of the wall portion to allow movement of bile and/or gallstones in the biliary duct.

17. The apparatus according to example 16, wherein said control device in said second mode controls said stimulation device to cease the stimulation of the wall portion and controls said constriction device to release the wall portion to restore the movement of bile and/or gallstones in the biliary duct.

18. The apparatus according to example 15, wherein said control device controls said stimulation device to adjust the intensity of the stimulation of the wall portion in response to a sensed physical parameter of the patient.

19. The apparatus according to example 15, wherein said control device controls said stimulation device to adjust the intensity of the stimulation of the wall portion in response to a sensed functional parameter of the apparatus.

20. The apparatus according to example 18, wherein said control device controls said stimulation device to increase the intensity of the stimulation of the wall portion, such that the movement of bile and/or gallstones in the biliary duct remains stopped when a pressure increase occurs in the biliary duct.

21. The apparatus according to example 20, further comprising a sensor for sensing a physical parameter of the patient's body that relates to the pressure in the lumen, wherein said control device controls said stimulation device in response to signals from said sensor.

22. The apparatus according to example 21, wherein the physical parameter is a pressure in the patient's body and the sensor is a pressure sensor.

23. The apparatus according to example 4, wherein said control device controls said constriction device to constrict the wall portion, such that the movement of bile and/or gallstones in the biliary duct is substantially stopped, and controls said stimulation device to stimulate the constricted wall portion to cause contraction thereof, such that the movement of bile and/or gallstones in the biliary duct is completely stopped.

24. The apparatus according to example 23, wherein said control device controls said stimulation device in a first mode to stimulate the constricted wall portion to completely stop the movement of bile and/or gallstones in the biliary duct and controls said stimulation device in a second mode to cease the stimulation of the wall portion to allow movement of bile and/or gallstones in the biliary duct.

25. The apparatus according to example 24, wherein said control device in said second mode controls said stimulation device to cease the stimulation of the wall portion and controls said constriction device to release the wall portion to restore the movement of bile and/or gallstones in the biliary duct.

26. The apparatus according to example 23, wherein said control device controls said stimulation device to adjust the intensity of the stimulation of the wall portion in response to a sensed physical parameter of the patient.

27. The apparatus according to example 23, wherein said control device controls said stimulation device to adjust the intensity of the stimulation of the wall portion in response to a sensed functional parameter of the apparatus

28. The apparatus according to example 26, wherein said control device controls said stimulation device to increase the intensity of the stimulation of the wall portion in response to a sensed pressure increase in the biliary duct of the biliary duct, such that the movement of bile and/or gallstones in the biliary duct remains stopped.

29. The apparatus according to example 28, further comprising a sensor for sensing a physical parameter of the patient's body that relates to the pressure in the biliary duct, wherein said control device controls said stimulation device in response to signals from said sensor.

30. The apparatus according to example 29, wherein the physical parameter is a pressure in the patient's body and the sensor is a pressure sensor.

31. The apparatus according to example 4, wherein said control device controls said constriction device to constrict the wall portion, such that the movement of bile and/or gallstones in the biliary duct is stopped.

32. The apparatus according to example 31, wherein said control device controls said constriction device in a first mode to constrict the constricted wall portion to stop the movement of bile and/or gallstones in the biliary duct and controls said constriction device in a second mode to cease the constriction of the wall portion to restore movement of bile and/or gallstones in the biliary duct.

33. The apparatus according to example 31, wherein said control device controls said stimulation device to stimulate the constricted wall portion to cause contraction thereof, such that the movement of bile and/or gallstones in the biliary duct remains stopped when a pressure increase occurs in the biliary duct.

34. The apparatus according to example 33, further comprising a sensor for sensing a physical parameter of the patient's body that relates to the pressure in the biliary duct, wherein said control device controls said stimulation device in response to signals from said sensor.

35. The apparatus according to example 34, wherein the physical parameter is a pressure in the patient's body and the sensor is a pressure sensor.

36. The apparatus according to example 4, wherein said control device controls said constriction device and/or said stimulation device from outside the patient's body.

37. The apparatus according to example 36, wherein said control device is operable by the patient.

38. The apparatus according to example 37, wherein said control device comprises a manually operable switch for switching on and off said constriction device and/or stimulation device, said switch being adapted for subcutaneous implantation in the patient to be manually operated from outside the patient's body.

39. The apparatus according to example 37, wherein said control device comprises a hand-held wireless remote control operable by the patient to switch on and off said constriction device and/or stimulation device.

40. The apparatus according to example 36, wherein said control device wirelessly controls said constriction device and/or stimulation device.

41. The apparatus according to example 40, wherein said control device wirelessly controls said constriction device in a non-magnetic manner.

42. The apparatus according to example 1, wherein said constriction device is designed to normally keep the patient's wall portion in a constricted state, in which the blood circulation in the constricted wall portion is substantially unrestricted and the movement of bile and/or gallstones in the biliary duct is at least restricted.

43. The apparatus according to example 42, wherein said control device controls said stimulation device to stimulate the constricted wall portion to cause contraction thereof, such that the movement of bile and/or gallstones in the biliary duct is further restricted but not stopped.

44. The apparatus according to example 43, wherein said control device controls said stimulation device to adjust the intensity of the stimulation of the wall portion in response to a sensed physical parameter of the patient or functional parameter of the apparatus.

45. The apparatus according to example 43, wherein said control device controls said stimulation device in a first mode to stimulate the constricted wall portion to further restrict the movement of bile and/or gallstones in the biliary duct and controls said stimulation device in a second mode to cease the stimulation of the wall portion to increase the movement of bile and/or gallstones in the biliary duct.

46. The apparatus according to example 42, wherein said control device controls said stimulation device to stimulate the constricted wall portion to cause contraction thereof, such that the movement of bile and/or gallstones in the biliary duct is stopped.

47. The apparatus according to example 46, wherein said control device controls said stimulation device to adjust the intensity of the stimulation of the wall portion in response to a sensed physical parameter of the patient or functional parameter of the apparatus.

48. The apparatus according to example 47, wherein said control device controls said stimulation device to increase the intensity of the stimulation, such that the movement of bile and/or gallstones in the biliary duct remains stopped when a pressure increase occurs in the biliary duct.

49. The apparatus according to example 48, further comprising a sensor for sensing a physical parameter of the patient that relates to the pressure in the biliary duct, wherein said control device controls said stimulation device in response to signals from said sensor.

50. The apparatus according to example 49, wherein the physical parameter is a pressure in the patient's body and the sensor is a pressure sensor.

51. The apparatus according to example 46, wherein said control device controls said stimulation device in a first mode to stimulate the constricted wall portion to stop the movement of bile and/or gallstones in the biliary duct and controls said stimulation device in a second mode to cease the stimulation of the wall portion to allow movement of bile and/or gallstones in the biliary duct.

52. The apparatus according to example 42, wherein said control device controls said stimulation device from outside the patient's body.

53. The apparatus according to example 52, wherein said control device is operable by the patient.

54. The apparatus according to example 53, wherein said control device comprises a manually operable switch for switching on and off said stimulation device, said switch being adapted for subcutaneous implantation in the patient to be manually operated from outside the patient's body.

55. The apparatus according to example 53, wherein said control device comprises a hand-held wireless remote control operable by the patient to switch on and off said stimulation device.

56. The apparatus according to example 52, wherein said control device wirelessly controls said stimulation device.

57. The apparatus according to example 1, wherein said control device controls said stimulation device to intermittently and individually stimulate different areas of the wall portion, such that at least two of the areas are stimulated at different points of time.

58. The apparatus according to example 57, wherein said control device controls said stimulation device to intermittently stimulate each area of the different areas of the wall portion during successive time periods, each time period being short enough to maintain over time satisfactory blood circulation in the area until the lapse of said time period.

59. The apparatus according to example 57, wherein said control device controls said stimulation device to intermittently stimulate the areas of the wall portion, such that an area of the wall portion that currently is not stimulated has time to restore substantially normal blood circulation before said stimulation device stimulates the area again.

60. The apparatus according to example 1, wherein said control device controls said stimulation device to stimulate one or more different areas of the wall portion at a time.

61. The apparatus according to example 60, wherein said control device controls said stimulation device to sequentially stimulate the different areas of the wall portion.

62. The apparatus according to example 60, wherein said control device controls said stimulation device to shift over time the stimulation from one area to another.

63. The apparatus according to example 62, wherein said control device controls said stimulation device to cyclically propagate the stimulation of the areas along the wall portion in the same or opposite direction of the flow in the patient's biliary duct.

64. The apparatus according to example 63, wherein said control device controls said stimulation device to propagate the stimulation of the areas in accordance with a determined stimulation pattern.

65. The apparatus according to example 1, wherein said control device controls said stimulation device to vary the intensity of the stimulation of the wall portion.

66. The apparatus according to example 65, wherein said control device controls said stimulation device to cyclically vary the intensity of the stimulation of said wall portion.

67. The apparatus according to example 1, wherein said control device controls said stimulation device to intermittently and individually stimulate different areas of the wall portion with pulses.

68. The apparatus according to example 67, wherein said control device controls said stimulation device to intermittently stimulate the areas with said pulses.

69. The apparatus according to example 67, wherein said pulses form pulse trains.

70. The apparatus according to example 69, wherein at least a first area and a second area of the areas of the wall portion are repeatedly stimulated with a first pulse train and a second pulse train, respectively, such that said first and second pulse trains over time are shifted relative to each other.

71. The apparatus according to example 70, wherein said first area is stimulated with said first pulse train while said second area is not stimulated with said second pulse train, and vice versa.

72. The apparatus according to example 70, wherein said first and second pulse trains are shifted relative to each other such that said first and second pulse trains at least partially overlap each other.

73. The apparatus according to example 69, wherein said control device controls said stimulation device to vary the amplitudes of the pulses of said pulse trains.

74. The apparatus according to example 69, wherein said control device controls said stimulation device to vary the off time periods between the individual pulses of each pulse train.

75. The apparatus according to example 69, wherein said control device controls said stimulation device to vary the width of each pulse of said pulse trains.

76. The apparatus according to example 69, wherein said control device controls said stimulation device to vary the frequency of the pulses of said pulse trains.

77. The apparatus according to example 69, wherein said control device controls said stimulation device to vary the off time periods between said pulse trains.

78. The apparatus according to example 77, wherein said control device controls said stimulation device to keep each off time period between said pulse trains long enough to restore substantially normal blood circulation in each area when the area is not stimulated during the off time periods.

79. The apparatus according to example 69, wherein said control device controls said stimulation device to vary the length of each pulse train.

80. The apparatus according to example 69, wherein said control device controls said stimulation device to vary the frequency of said pulse trains.

81. The apparatus according to example 69, wherein said control device controls said stimulation device to vary the number of pulses of each pulse train.

82. The apparatus according to example 1, wherein said stimulation device intermittently and individually electrically stimulates different areas of the patient's wall portion.

83. The apparatus according to example 82, wherein said stimulation device stimulates the areas of the patient's wall portion with electric pulses.

84. The apparatus according to example 83, wherein the wall portion includes muscle fibers and said stimulation device stimulates the wall portion including the muscle fibers with electric pulses, to cause contraction of the muscle fibres to contract the wall portion.

85. The apparatus according to example 82, wherein said stimulation device comprises at least one electrical element for engaging the wall portion and for stimulating the wall portion with electric pulses.

86. The apparatus according to example 85, wherein said stimulation device comprises a plurality of electrical elements.

87. The apparatus according to example 86, wherein said electrical elements are placed in a fixed orientation relative to one another.

88. The apparatus according to example 87, wherein said stimulation device comprises a structure holding said electrical elements in said fixed orientation.

89. The apparatus according to example 88, wherein said electrical elements form an elongate pattern of electrical elements, and said structure is applicable on the patient's biliary duct such that said elongate pattern of electrical elements extends along the wall portion of the biliary duct in the direction of the flow in the patient's biliary duct and the elements abut the respective areas of the wall portion.

90. The apparatus according to example 88, wherein said structure is integrated in said constriction device.

91. The apparatus according to example 88, wherein said structure is separate from said constriction device.

92. The apparatus according to example 86, wherein said control device controls said stimulation device to electrically energize said electrical elements.

93. The apparatus according to example 92, wherein said control device controls said stimulation device to cyclically energize each element with electric pulses.

94. The apparatus according to example 93, wherein said control device controls said stimulation device to energize said electrical elements, such that a number or groups of said electrical elements are energized at the same time.

95. The apparatus according to example 93, wherein said control device controls said stimulation device to energize said electrical elements, such that said electrical elements are energized one at a time in sequence or groups of said electrical elements are sequentially energized, either randomly or in accordance with a predetermined pattern.

96. The apparatus according to example 93, wherein said electrical elements form an elongate pattern of electrical elements, and said elements are applicable on the patient's wall such that said elongate pattern of electrical elements extends along the wall portion of the biliary duct in the direction of the flow in the patient's biliary duct and the elements abut the respective areas of the wall portion.

97. The apparatus according to example 96, wherein said control device controls said stimulation device to successively energize said electrical elements longitudinally along said elongate pattern of electrical elements.

98. The apparatus according to example 97, wherein said control device controls said stimulation device to successively energize said electrical elements along said elongate pattern of electrical elements in a direction opposite to, or in the same direction as, that of the flow in the patient's biliary duct, when said stimulation device is applied on the patient's biliary duct.

99. The apparatus according to example 97, wherein said control device controls said stimulation device to successively energize said electrical elements from a position substantially at the center of the constricted wall portion towards both ends of said elongate pattern of electrical elements, when said stimulation device is applied on the patient's biliary duct.

100. The apparatus according to example 97, wherein said control device controls said stimulation device to energize said electrical elements, such that electrical elements currently energized form at least one group of adjacent energized electrical elements.

101. The apparatus according to example 100, wherein said elements in said group of energized electrical elements form a path of energized electrical elements.

102. The apparatus according to example 101, wherein said path of energized electrical elements extends at least in part around the patient's biliary duct, when said stimulation device is applied on the biliary duct.

103. The apparatus according to example 102, wherein said path of energized electrical elements extends completely around the patient's biliary duct, when said stimulation device is applied on the biliary duct.

104. The apparatus according to example 100, wherein said elements in said group of energized electrical elements form two paths of energized electrical elements extending opposite to each other, when said stimulation device is applied on the patient's biliary duct.

105. The apparatus according to example 104, wherein said two paths of energized electrical elements extend on mutual sides of the patient's biliary duct and at least substantially transverse to the direction of flow in the patient's biliary duct, when said stimulation device is applied on the biliary duct.

106. The apparatus according to example 92, wherein said electrical elements form a plurality of groups of elements, the groups forming a series of groups extending along the patient's biliary duct in the direction of flow in the patient's biliary duct, when said stimulation device is applied on the biliary duct.

107. The apparatus according to example 106, wherein said control device controls said stimulation device to successively energize said groups of electrical elements in said series of groups in a direction opposite to, or in the same direction as, that of the flow in the patient's biliary duct, when said stimulation device is applied on the patient's biliary duct.

108. The apparatus according to example 106, wherein said control device controls said stimulation device to successively energize said groups of electrical elements in said series of groups from a position substantially at the center of the constricted wall portion in a direction opposite to, and in the same direction as, that of the flow in the patient's biliary duct, when said stimulation device is applied on the patient's biliary duct.

109. The apparatus according to example 106, wherein said electrical elements of each group of electrical elements form a path of elements extending at least in part around the patient's biliary duct, when said stimulation device is applied on the biliary duct.

110. The apparatus according to example 109, wherein said path of electrical elements of each group of elements extends completely around the patient's biliary duct, when said stimulation device is applied on the biliary duct.

111. The apparatus according to example 106, wherein said electrical elements of each group of electrical elements form two paths of elements extending on mutual sides of the patient's biliary duct, when said stimulation device is applied on the biliary duct.

112. The apparatus according to example 111, wherein said two paths of electrical elements of each group of elements extend at least substantially transverse to the direction of flow in the patient's biliary duct, when said stimulation device is applied on the patient's biliary duct.

113. The apparatus according to example 1, wherein said stimulation device thermally stimulates the wall portion.

114. The apparatus according to example 113, wherein said control device controls said stimulation device to cool the constricted wall portion to cause contraction of the wall portion.

115. The apparatus according to example 114, wherein said constriction device is adapted to constrict the wall portion to at least restrict the movement of bile and/or gallstones in the biliary duct, and said control device controls said stimulation device to cool the constricted wall portion to cause contraction thereof, such that the movement of bile and/or gallstones in the biliary duct is at least further restricted.

116. The apparatus according to example 115, wherein said control device controls said stimulation device to cool the wall portion to cause contraction thereof, such that the movement of bile and/or gallstones in the biliary duct is further restricted but not stopped.

117. The apparatus according to example 115, wherein said control device controls said stimulation device to cool the wall portion to cause contraction thereof, such that the movement of bile and/or gallstones in the biliary duct is stopped.

118. The apparatus according to example 113, wherein said control device controls said stimulation device to heat the wall portion, when the wall portion is constricted and contracted, to cause expansion of the wall portion.

119. The apparatus according to example 113, wherein the wall portion includes a blood vessel and said control device controls said stimulation device to cool the blood vessel to cause contraction thereof, or to heat the blood vessel to cause expansion thereof.

120. The apparatus according to example 113, wherein said control device controls said constriction device and/or said stimulation device from outside the patient's body.

121. The apparatus according to example 1, wherein said control device comprises an internal control unit implantable in the patient for controlling said constriction device and/or stimulation device.

122. The apparatus according to example 121, wherein said internal control unit is programmable.

123. The apparatus according to example 122, wherein said control device comprises an external control unit intended to be outside the patient's body for controlling said constriction device and/or stimulation device.

124. The apparatus according to example 123, wherein said internal control unit is programmable by said external control unit.

125. The apparatus according to example 122, wherein said internal control unit is programmable for controlling said constriction device and/or stimulation device over time.

126. The apparatus according to example 125, wherein said internal control unit controls said constriction device over time in accordance with an activity schedule program.

127. The apparatus according to example 122, wherein said internal control unit comprises a microprocessor.

128. The apparatus according to example 1, further comprising at least one implantable sensor, wherein said control device controls said constriction device and/or said stimulation device in response to signals from said sensor.

129. The apparatus according to example 128, wherein said sensor directly or indirectly senses at least one physical parameter of the patient.

130. The apparatus according to example 128, wherein said sensor directly or indirectly senses at least one functional parameter of a medical implant.

131. The apparatus according to example 128, wherein said sensor comprises a pressure sensor for sensing a pressure in the patient's body.

132. The apparatus according to example 131, wherein said control device controls said constriction device and/or stimulation device to change the constriction of the patient's wall portion in response to said pressure sensor sensing a predetermined value of measured pressure.

133. The apparatus according to example 128, wherein said control device comprises an implantable internal control unit directly controlling said constriction device and/or stimulation device in response to signals from said sensor.

134. The apparatus according to example 128, wherein said control device comprises an external control unit outside the patient's body for controlling said constriction device and/or stimulation device in response to signals from said sensor.

135. The apparatus according to example 128, wherein said control device produces an indication in response to said signals from said sensor.

136. The apparatus according to example 135, wherein said indication comprises a sound signal or displayed information.

137. The apparatus according to example 1, wherein said constriction device and stimulation device co-operate to move bile and/or gallstones in the biliary duct.

138. The apparatus according to example 137, wherein said constriction device is adapted to constrict the wall portion to restrict the movement of bile and/or gallstones in the biliary duct, and said control device controls said stimulation device to progressively stimulate the constricted wall portion to cause progressive contraction of the wall portion to move the bile and/or gallstones in the biliary duct.

139. The apparatus according to example 138, wherein said control device controls said stimulation device to progressively stimulate the constricted wall portion in the downstream or upstream direction of the biliary duct.

140. The apparatus according to example 137, wherein said constriction device is adapted to constrict the wall portion to restrict the movement of bile and/or gallstones in the biliary duct, and said control device controls said stimulation device to stimulate the constricted wall portion to close the biliary duct either at an upstream end or a downstream end of the wall portion and simultaneously controls said constriction device to increase the constriction of the wall portion to move the bile and/or gallstones in the biliary duct.

141. The apparatus according to example 137, wherein said control device controls said constriction device to vary the constriction of the wall portion and simultaneously controls said stimulation device to progressively stimulate the constricted wall portion to cause progressive contraction of the wall portion to move the bile and/or gallstonesin the biliary duct.

142. The apparatus according to example 141, wherein said control device controls said stimulation device to progressively stimulate the constricted wall portion in the downstream or upstream direction of the biliary duct.

143. The apparatus according to example 137, wherein said control device controls said stimulation device to stimulate the wall portion and simultaneously controls said constriction device to vary the constriction of different areas of the wall portion such that the wall portion is progressively constricted in the downstream or upstream direction of the biliary duct.

144. The apparatus according to example 143, wherein said control device controls said stimulation device to progressively stimulate the constricted wall portion to cause progressive contraction thereof in harmony with the progressive constriction of the wall portion performed by the constriction device.

145. The apparatus according to example 143, wherein said constriction device comprises at least one elongated constriction element extending along the wall portion, and said control device controls said elongated constriction element to progressively constrict the wall portion in the downstream or upstream direction of the biliary duct.

146. The apparatus according to example 145, wherein said elongated constriction element comprises contact surfaces dimensioned to contact a length of the wall portion, when said constriction device constricts the wall portion, and said stimulation device comprises a plurality of stimulation elements distributed along said contact surfaces, such that said stimulation elements stimulate the different areas of the wall portion along the length of the wall portion, when said control device controls said stimulation device to stimulate the wall portion.

147. The apparatus according to example 137, wherein said constriction device is adapted to constrict any one of a series of wall portions of the tissue wall to at least restrict the movement of bile and/or gallstones in the biliary duct, said stimulation device stimulates the wall portion constricted by said constriction device to close the biliary duct, and said control device controls said constriction device to successively constrict the wall portions of the series of wall portions to move the bile and/or gallstones in the biliary duct in a peristaltic manner.

148. The apparatus according to example 147, wherein said constriction device comprises at least one constriction element that is moveable along the biliary duct to successively constrict the wall portions of the series of wall portions of the biliary duct, and said stimulation device comprises at least one stimulation element positioned on said constriction element for stimulating the wall portion constricted by said constriction element to close the biliary duct.

149. The apparatus according to example 148, wherein said control device controls said constriction device to cyclically move said constriction element along the wall portions of the series of wall portions.

150. The apparatus according to example 147, wherein said constriction device comprises a plurality of constriction elements, each of which is moveable along the biliary duct to successively constrict the wall portions of the series of wall portions of the biliary duct, and said stimulation device comprises stimulation elements positioned on said constriction elements for stimulating the wall portions constricted by said constriction elements to close the biliary duct.

151. The apparatus according to example 150, wherein said control device controls said constriction device to cyclically move said constriction elements one after the other along the wall portions of the series of wall portions of the biliary duct.

152. The apparatus according to example 151, wherein said constriction device comprises a rotor carrying said constriction elements, and said control device controls said rotor to rotate such that each constriction element cyclically constricts the wall portions of the series of wall portions of the biliary duct.

153. The apparatus according to example 152, wherein each constriction element comprises a roller for rolling on the biliary duct to constrict the latter.

154. The apparatus according to example 137, wherein said constriction device comprises a first constriction element for constricting the wall portion of the biliary duct at an upstream end thereof, a second constriction element for constricting the wall portion at a downstream end thereof, and a third constriction element for constricting the wall portion between the upstream and downstream ends thereof, and said control device controls said first, second and third constriction elements to constrict and release the wall portions independently of one another.

155. The apparatus according to example 154, wherein said control device controls said first or second constriction element to constrict the wall portion at the upstream or downstream end thereof to close the biliary duct and controls said third constriction element to constrict the wall portion between the upstream and downstream ends thereof, whereby the fluid or other bodily matter contained in the wall portion between the upstream and downstream ends thereof is moved downstream or upstream in the biliary duct.

156. The apparatus according to example 155, wherein said control device controls said stimulation device to stimulate the wall portion between the upstream and downstream ends thereof, when said third constriction element constricts the wall portion.

157. The apparatus according to example 154, wherein said control device controls said first constriction element to constrict the wall portion at the upstream end thereof to restrict the movement of bile and/or gallstones in the biliary duct and controls said stimulation device to stimulate the constricted wall portion at the upstream end to close the biliary duct.

158. The apparatus according to example 157, wherein said control device controls said third constriction element to constrict the wall portion between the upstream and downstream ends thereof, whereby the bile and/or gallstones contained in the wall portion between the upstream and downstream ends thereof is moved downstream in the biliary duct.

159. The apparatus according to example 158, wherein said control device controls said second constriction element to constrict the wall portion at the downstream end thereof to restrict the movement of bile and/or gallstones in the biliary duct and controls said stimulation device to stimulate the constricted wall portion at the downstream end to close the biliary duct.

160. The apparatus according to example 159, wherein said control device controls said third constriction element to constrict the wall portion between the upstream and downstream ends thereof, whereby the bile and/or gallstones contained in the wall portion between the upstream and downstream ends thereof is moved upstream in the biliary duct.

161. The apparatus according to example 137, wherein said stimulation device is adapted to stimulate the wall portion with electric pulses.

162. The apparatus according to example 1, wherein said constriction device is adjustable, and further comprising an operation device for operating the adjustable constriction device to change the constriction of the patient's wall portion of the biliary duct.

163. The apparatus according to example 162, wherein said operation device mechanically operates said constriction device.

164. The apparatus according to example 162, wherein said operation device hydraulically operates said constriction device.

165. The apparatus according to example 163, wherein said operation device operates said constriction device in a non-magnetic and/or non-manual manner.

166. The apparatus according to example 163, wherein said operation device comprises an electrically powered operation device.

167. The apparatus according to example 162, wherein said operation device comprises a motor.

168. The apparatus according to example 162, wherein said operation device comprises a servo system.

169. The apparatus according to example 162, wherein said constriction device comprises at least two elongated clamping elements extending along different sides of the patient's biliary duct in the direction of flow in the biliary duct, and said operation device operates said clamping elements to clamp the wall portion between said clamping elements to constrict the wall portion.

170. The apparatus according to example 162, wherein said operation device comprises hydraulic means for hydraulically adjusting said constriction device and a reverse servo operatively connected to said hydraulic means.

171. The apparatus according to example 162, wherein said constriction device comprises a non-inflatable mechanical constriction device and said operation device comprises hydraulic means that hydraulically adjusts said mechanical constriction device.

172. A method for using an apparatus as recited in example 1 to control the movement of bile and/or gallstones in a patient's biliary duct, the method comprising:

- providing a wireless remote control adapted to control the constriction device and/or stimulation device from outside the patient's body, and
- operating the wireless remote control by the patient, when the patient wants to influence the flow of bile and/or gallstones in the lumen.

173. An apparatus for controlling the movement of bile and/or gall in the biliary duct, the apparatus comprising:

an implantable constriction device for gently constricting a portion of the tissue wall to influence the movement of bile and/or gallstones in the biliary duct,
a stimulation device for stimulating the wall portion, as said constriction device constricts the wall portion, to cause contraction of the wall portion to further influence the movement of bile and/or gallstones in the biliary duct, wherein said constriction and stimulation devices form an operable constriction/stimulation unit,
a source of energy, and
a control device operable from outside the patient's body to control said source of energy to release energy for use in connection with the operation of said constriction/stimulation unit.

174. The apparatus according to example 173, wherein said source of energy is implantable in the patient's body.

175. The apparatus according to example 174, wherein said source of energy comprises a battery.

176. The apparatus according to example 173, wherein said source of energy is external to the patient's body and said control device controls said external source of energy to release wireless energy, further comprising an energy-transmission device for transmitting said released wireless energy from outside the patient's body to inside the patient's body.

177. The apparatus according to example 176, wherein said energy-transmission device is adapted to transmit the wireless energy in pulses or digital pulses, or a combination of pulses and digital pulses.

178. The apparatus according to example 176, wherein the wireless energy comprises electromagnetic energy.

179. The apparatus according to example 176, wherein the wireless energy comprises an electric, an electromagnetic or a magnetic field, or a combination thereof, or electromagnetic waves.

180. The apparatus according to example 176, wherein said energy-transmission device transmits wireless energy for direct use in connection with the operation of said constriction/stimulation unit, as the wireless energy is being transmitted.

181. The apparatus according to example 180, further comprising an implantable electric motor or pump for operating the constriction device, wherein said motor or pump is directly powered by wireless energy in the form of a magnetic or an electromagnetic field.

182. The apparatus according to example 181, wherein said motor or pump comprises coils, and the magnetic or electromagnetic field influences said coils to generate a current for driving said motor or pump.

183. The apparatus according to example 181, wherein said motor or pump comprises materials influenced by magnetic fields, and the magnetic or electromagnetic field influences said materials to create kinetic energy for driving said motor or pump.

184. The apparatus according to example 181, wherein said motor or pump comprises permanent magnets, and the magnetic or electromagnetic field influences said magnets to create kinetic energy for driving said motor or pump.

185. The apparatus according to example 176, wherein said energy-transmission device transmits energy of a first form and said constriction/stimulation unit is operable in response to energy of a second form, and further comprising an energy-transforming device implantable in the patient for transforming the energy of the first form wirelessly transmitted by said energy-transmission device into the energy of the second form.

186. The apparatus according to example 185, wherein the energy of the second form is different from the energy of the first form.

187. The apparatus according to example 185, wherein said energy-transforming device comprises at least one element having a positive region and a negative region, when exposed to the energy of the first form transmitted by said energy-transmission device said element is capable of creating an energy field between said positive and negative regions, and said energy field produces the energy of the second form.

188. The apparatus according to example 187, wherein said element comprises an electrical junction element, and said electrical junction element is capable of inducing an electric field between said positive and negative regions when exposed to the energy of the first form transmitted by said energy-transmission device, whereby the energy of the second form comprises electric energy.

189. The apparatus according to example 185, wherein said energy-transforming device comprises at least one semiconductor.

190. The apparatus according to example 189, wherein said semiconductor comprises at least one element having a positive region and a negative region, when exposed to the energy of the first form transmitted by said energy-transmission device, said element is capable of creating an energy field between said positive and negative regions, and said energy field produces the energy of the second form.

191. The apparatus according to example 185, wherein said energy-transforming device transforms the energy of the first form directly or indirectly into the energy of the second form.

192. The apparatus according to example 191, further comprising an implantable motor or pump for operating said constriction device, wherein said motor or pump is powered by the energy of the second form.

193. The apparatus according to example 192, wherein said constriction device is operable to perform at least one reversible function and said motor is capable of reversing said function.

194. The apparatus according to example 192, wherein the control device shifts polarity of the energy of the second form to reverse said motor.

195. The apparatus according to example 192, wherein said energy-transforming device directly powers said motor or pump with the transformed energy, as the energy of the second form is being transformed from the energy of the first form.

196. The apparatus according to example 191, wherein the wireless energy of the first form comprises sound waves and the energy of the second form comprises electric energy.

197. The apparatus according to example 196, wherein said energy-transforming device comprises a piezo-electric element for transforming sound waves into electric energy.

198. The apparatus according to example 185, further comprising an internal source of energy implantable in the patient for supplying energy for the operation of said constriction/stimulation unit.

199. The apparatus according to example 198, wherein said internal source of energy comprises a battery.

200. The apparatus according to example 198, wherein said internal source of energy stores the energy of the second form supplied by said energy-transforming device.

201. The apparatus according to example 200, wherein said internal source of energy comprises an accumulator.

202. The apparatus according to example 201, wherein said accumulator comprises at least one capacitor or at least one rechargeable battery, or a combination of at least one capacitor and at least one rechargeable battery.

203. The apparatus according to example 198, further comprising an implantable switch operable to switch from an off mode, in which said internal source of energy is not in use, to an on mode, in which said internal source of energy supplies energy for the operation of said constriction/stimulation unit.

204. The apparatus according to example 203, wherein said switch is operable by the energy of the first form transmitted by said energy-transmission device.

205. The apparatus according to example 203, wherein said switch is operable by the energy of the second form supplied by said energy-transforming device.

206. The apparatus according to example 185, further comprising an implantable stabiliser for stabilising the energy of the second form.

207. The apparatus according to example 206, wherein the energy of the second form comprises electric current and said stabiliser comprises at least one capacitor.

208. The apparatus according to example 185, wherein said energy-transforming device directly operates said constriction/stimulation unit with the energy of the second form in a non-magnetic, non-thermal or non-mechanical manner.

209. The apparatus according to example 176, wherein said energy-transmission device transmits energy by at least one wireless signal.

210. The apparatus according to example 209, wherein said signal comprises a wave signal.

211. The apparatus according to example 210, wherein said wave signal comprises an electromagnetic wave signal including one of an infrared light signal, a visible light signal, an ultra violet light signal, a laser signal, a micro wave signal, a radio wave signal, an x-ray radiation signal, and a gamma radiation signal.

212. The apparatus according to example 210, wherein said wave signal comprises a sound or ultrasound wave signal.

213. The apparatus according to example 209, wherein said signal comprises a digital or analogue signal, or a combination of a digital and analogue signal.

214. The apparatus according to example 185, wherein said energy-transforming device transforms the energy of the first form into a direct current or pulsating direct current, or a combination of a direct current and pulsating direct current.

215. The apparatus according to example 185, wherein said energy-transforming device transforms the energy of the first form into an alternating current or a combination of a direct and alternating current.

216. The apparatus according to example 185, wherein one of the energy of the first form and the energy of the second form comprises magnetic energy, kinetic energy, sound energy, chemical energy, radiant energy, electromagnetic energy, photo energy, nuclear energy or thermal energy.

217. The apparatus according to example 185, wherein one of the energy of the first form and the energy of the second form is non-magnetic, non-kinetic, non-chemical, non-sonic, non-nuclear or non-thermal.

218. The apparatus according to example 173, further comprising implantable electrical components including at least one voltage level guard.

219. The apparatus according to example 173, further comprising implantable electrical components including at least one constant current guard.

220. The apparatus according to example 185, wherein said energy-transmission device functions different from said energy-transforming device.

221. The apparatus according to example 185, wherein said energy-transmission device functions similar to said energy-transforming device.

222. The apparatus according to example 185, wherein said energy-transforming device is designed to be implanted subcutaneously or in the abdomen, thorax or cephalic region of the patient.

223. The apparatus according to example 185, wherein said energy-transforming device is designed to be implanted in an orifice of the patient's body and under the mucosa or intramuscularly outside the mucosa of the orifice.

224. The apparatus according to example 173, wherein said control device controls said constriction/stimulation unit.

225. The apparatus according to example 224, wherein said control device comprises a microprocessor.

226. The apparatus according to example 173, wherein said control device is operable by the patient.

227. The apparatus according to example 173, wherein said control device comprises a manually or magnetically operable switch for switching on and off said constriction/stimulation unit, said switch being adapted for subcutaneous implantation in the patient.

228. The apparatus according to example 226, wherein said control device comprises a hand-held wireless remote control operable by the patient to control said constriction/stimulation unit to adjust the stimulation intensity and/or adjust the constriction of the wall portion.

229. The apparatus according to example 173, wherein said control device comprises a remote control for controlling said constriction/stimulation unit from outside the patient's body.

230. The apparatus according to example 229, wherein said remote control comprises a wireless remote control.

231. The apparatus according to example 230, wherein said wireless remote control comprises at least one external signal transmitter or transceiver and at least one internal signal receiver or transceiver implantable in the patient.

232. The apparatus according to example 230, wherein said wireless remote control is adapted to transmit at least one wireless control signal for controlling said constriction/stimulation unit.

233. The apparatus according to example 232, wherein said control signal comprises a frequency, amplitude, phase modulated signal or a combination thereof.

234. The apparatus according to example 232, wherein said control signal comprises an analogue or a digital signal, or a combination of an analogue and digital signal.

235. The apparatus according to example 232, wherein said remote control transmits a carrier signal for carrying said control signal.

236. The apparatus according to example 235, wherein said carrier signal comprises digital, analogue or a combination of digital and analogue signals.

237. The apparatus according to example 236, wherein said signals comprise wave signals.

238. The apparatus according to example 232, wherein said control signal comprises a wave signal comprising one of a sound wave signal, an ultrasound wave signal, an electromagnetic wave signal, an infrared light signal, a visible light signal, an ultra violet light signal, a laser light signal, a micro wave signal, a radio wave signal, an x-ray radiation signal and a gamma radiation signal.

239. The apparatus according to example 232, wherein said control signal comprises an electric or magnetic field, or a combined electric and magnetic field.

240. The apparatus according to example 234, wherein said remote control transmits an electromagnetic carrier wave signal for carrying said digital or analogue control signal.

241. The apparatus according to example 173, further comprising an external data communicator and an implantable internal data communicator communicating with said external data communicator, wherein said internal communicator feeds data related to said constriction/stimulation unit back to said external data communicator or said external data communicator feeds data to said internal data communicator.

242. The apparatus according to example 173, further comprising at least one implantable sensor, wherein said control device is adapted to control said constriction/stimulation unit in response to signals from said sensor.

243. The apparatus according to example 242, wherein said sensor is adapted to directly or indirectly sense at least one physical parameter of the patient.

244. The apparatus according to example 242, wherein said sensor is adapted to directly or indirectly sense at least one functional parameter of a medical implant.

245. The apparatus according to example 243, wherein said sensor comprises a pressure sensor for directly or indirectly sensing the pressure against said constriction device.

246. The apparatus according to example 245, wherein said control device is adapted to control said constriction/stimulation unit to change the constriction of the patient's wall portion in response to said pressure sensor sensing a predetermined value.

247. The apparatus according to example 242, wherein said control device comprises an internal control unit implanted in the patient and directly controlling said constriction/stimulation unit in response to signals from said sensor.

248. The apparatus according to example 242, wherein said control device comprises an external control unit outside the patient's body controlling said constriction/stimulation unit in response to signals from said sensor.

249. The apparatus according to example 242, wherein said control device comprises an implantable internal control unit that directly controls said constriction/stimulation unit in response to signals from said sensor.

250. The apparatus according to example 242, wherein said control device comprises an external control unit outside said patient's body for controlling said constriction/stimulation unit in response to signals from said sensor.

251. The apparatus according to example 243, wherein said control device is adapted to produce an indication in response to said sensor sensing said physical parameter.

252. The apparatus according to example 251, wherein said indication comprises a sound signal or displayed information.

253. The apparatus according to example 173, further comprising an implantable operation device adapted to operate said constriction/stimulation unit.

254. The apparatus according to example 253, further comprising a magnet for activating said operation device.

255. The apparatus according to example 254, wherein said magnet is adapted to activate said operation device from outside the patient's body.

256. The apparatus according to example 253, wherein said operation device comprises a motor.

257. The apparatus according to example 256, wherein said motor is powered by energy released from said source of energy.

258. The apparatus according to example 256, further comprising an implantable gearbox, wherein said motor is operatively connected to said constriction device of said constriction/stimulation unit via said gearbox.

259. The apparatus according to example 173, wherein said constriction device is operable to perform a reversible function.

260. The apparatus according to example 259, further comprising a reversing device implantable in the patient for reversing the function performed by said constriction device.

261. The apparatus according to example 260, wherein said control device controls said reversing device to reverse the function performed by said constriction device.

262. The apparatus according to example 260, wherein said reversing device comprises hydraulic means including a valve for shifting the flow direction of a liquid flow in said hydraulic means.

263. The apparatus according to example 260, wherein said reversing device comprises a mechanical reversing device.

264. The apparatus according to example 260, wherein said mechanical reversing device comprises a gearbox.

265. The apparatus according to example 260, wherein said reversing device comprises a switch.

266. The apparatus according to example 1, wherein said control device controls said constriction device to close the biliary duct, either at an upstream end or a downstream end of the wall portion, and controls said constriction device to constrict the remaining part of the wall portion to move the bile and/or gallstones in the biliary duct.

267. The apparatus according to example 266, wherein said control device controls said stimulation device to stimulate the wall portion as said constriction device constricts the remaining part of the wall portion.

268. The apparatus according to example 1, wherein said constriction device is adapted to constrict the wall portion to restrict but not stop the movement of bile and/or gallstones in the biliary duct, and said control device controls said stimulation device to stimulate the wall portion constricted by said constriction device to close the biliary duct, either at an upstream end or a downstream end of the wall portion, and simultaneously controls said constriction device to increase the constriction of the wall portion to move the bile and/or gallstones in the biliary duct.

269. The apparatus according to example 1, wherein said constriction device is adapted to constrict the wall portion to restrict or vary the movement of bile and/or gallstones in the biliary duct, and said control device controls said stimulation device to progressively stimulate the constricted wall portion, in the downstream or upstream direction of the biliary duct, to cause progressive contraction of the wall portion to move the bile and/or gallstones in the lumen.

270. The apparatus according to example 1, wherein said control device controls said constriction device to vary the constriction of different areas of the wall portion, such that the wall portion is progressively constricted in the downstream or upstream direction of the lumen to move the bile and/or gallstones in the biliary duct.

271. The apparatus according to example 270, wherein said constriction device comprises at least one elongated constriction element that extends along the wall portion, and said control device controls said elongated constriction element to progressively constrict the wall portion in the downstream or upstream direction of the biliary duct.

272. The apparatus according to example 270, wherein said control device controls said stimulation device to progressively stimulate the constricted wall portion to cause progressive contraction thereof in harmony with the progressive constriction of the wall portion performed by said constriction device.

273. The apparatus according to example 272, wherein said constriction device comprises at least one elongated constriction element, and said control device controls said elongated constriction element to progressively constrict the wall portion in the downstream or upstream direction of the biliary duct.

274. The apparatus according to example 273, wherein said elongated constriction element comprises contact surfaces dimensioned to contact a length of the wall portion, when said constriction device constricts the wall portion, and said stimulation device comprises a plurality of stimulation elements distributed along said contact surfaces, such that said stimulation elements stimulate the different areas of the wall portion along the length of the wall portion, when said control device controls said stimulation device to stimulate the wall portion.

275. The apparatus according to example 1, wherein said constriction device is adapted to constrict any one of a series of wall portions of the tissue wall to at least restrict the movement of bile and/or gallstones in the biliary duct.

276. The apparatus according to example 275, wherein said control device controls said constriction device to successively constrict the wall portions of the series of wall portions to move the bile and/or gallstones in the lumen in a peristaltic manner.

277. The apparatus according to example 276, wherein said constriction device comprises at least one constriction element moveable along the wall of the biliary duct to successively constrict the wall portions of the series of wall portions, and said control device controls said constriction device to cyclically move said constriction element along the wall portions of the series of wall portions.

278. The apparatus according to example 276, wherein said constriction device comprises a plurality of constriction elements, each of which is moveable along the wall of the biliary duct to successively constrict the wall portions of the series of wall portions, wherein said control device controls said constriction device to cyclically move said constriction elements one after the other along the wall portions of the series of wall portions.

279. The apparatus according to example 278, wherein said constriction device includes a rotor carrying said constriction elements, and said control device controls said rotor to rotate, such that each constriction element cyclically constricts the wall portions of the series of wall portions.

280. The apparatus according to example 279, wherein each constriction element comprises a roller for rolling on the wall of the biliary duct to constrict the latter.

281. The apparatus according to example 276, wherein said stimulation device stimulates the wall portion of the series of wall portions which is constricted by said constriction device, to close the biliary duct.

282. The apparatus according to example 277, wherein said stimulation device comprises at least one stimulation element positioned on said constriction element for stimulating the wall portion constricted by said constriction element to close the biliary duct.

283. The apparatus according to example 278, wherein said stimulation device comprises stimulation elements positioned on said constriction elements for stimulating the wall portions constricted by said constriction elements to close the biliary duct.

284. The apparatus according to example 1, wherein said constriction device comprises a first constriction element for constricting the wall portion at an upstream end thereof, a second constriction element for constricting the wall portion at a downstream end thereof, and a third constriction element for constricting the wall portion between the upstream and downstream ends thereof.

285. The apparatus according to example 284, wherein said control device controls said first, second and third constriction elements to constrict and release the wall portion independently of one another.

286. The apparatus according to example 285, wherein said control device controls said first or second constriction element to constrict the wall portion at the upstream or downstream end thereof to close the biliary duct, and controls said third constriction element to constrict the wall portion between the upstream and downstream ends thereof, whereby the bile and/or gallstones contained in the wall portion between the upstream and downstream ends thereof is moved downstream or upstream in the biliary duct.

287. The apparatus according to example 286, wherein said control device controls said stimulation device to stimulate the wall portion between the upstream and downstream ends thereof, when said third constriction element constricts the wall portion.

288. The apparatus according to example 285, wherein said control device controls said first constriction element to constrict the wall portion at the upstream end thereof to restrict the movement of bile and/or gallstones in the biliary duct and controls said stimulation device to stimulate the constricted wall portion at the upstream end to close the biliary duct.

289. The apparatus according to example 288, wherein said control device controls said third constriction element to constrict the wall portion between the upstream and downstream ends thereof to move bile and/or gallstones contained in the wall portion between the upstream and downstream ends thereof downstream in the biliary duct.

290. The apparatus according to example 289, wherein said control device controls said stimulation device to simultaneously stimulate the wall portion as the latter is constricted by said third constriction element.

291. The apparatus according to example 285, wherein said control device controls said second constriction element to constrict the wall portion at the downstream end thereof to restrict the movement of bile and/or gallstones in the biliary duct and controls said stimulation device to stimulate the constricted wall portion at the downstream end to close the biliary duct.

292. The apparatus according to example 291, wherein said control device controls said third constriction element to constrict the wall portion between the upstream and downstream ends thereof to move bile and/or gallstones contained in the wall portion between the upstream and downstream ends thereof upstream in the biliary duct.

293. The apparatus according to example 292, wherein said control device controls said stimulation device to simultaneously stimulate the wall portion as the latter is constricted by said third constriction element.

294. The apparatus according to example 137, wherein said constriction device comprises a plurality of separate constriction elements adapted to constrict any wall portions of a series of wall portions of the biliary duct's tissue wall, respectively.

295. The apparatus according to example 315, wherein said control device controls said constriction device to activate said constriction elements in random or in accordance with a predetermined sequence.

296. The apparatus according to example 315, wherein said control device controls said constriction device to activate said constriction elements one after the other, so that the wall portions of the series of wall portions are successively constricted along the biliary duct to move the bile and/or gallstones in the lumen of the patient's biliary duct.

297. The apparatus according to example 315, wherein said stimulation device comprises stimulation elements positioned on said constriction elements.

298. The apparatus according to example 318, wherein said control device controls said stimulation device to activate said stimulation elements to stimulate any wall portions of the series of wall portions constricted by said constriction elements.

299. The apparatus according to example 319, wherein said control device controls said constriction device to activate said constriction elements to constrict the wall portions of the series of wall portions without completely closing the biliary duct, and controls said stimulation device to activate said stimulation elements to stimulate the constricted wall portions one after the other, so that the wall portions of the series of wall portions are successively contracted along the biliary duct to move the bile and/or gallstones in the lumen of the patient's biliary duct.

300. The apparatus according to example 319, wherein said control device controls said constriction device to activate said constriction elements to constrict all of the wall portions of the series of wall portions, and controls said stimulation device to activate said stimulation elements to stimulate any constricted wall portions in random or in accordance with a predetermined sequence to close the biliary duct.

301. An apparatus for controlling the movement of bile and/or gallstones in the biliary duct of a patient suffering from gallstone trouble, comprising:

an implantable stimulation device for stimulating a portion of the tissue wall of the biliary duct of the patient, and
a control device for controlling said stimulation device to progressively stimulate the tissue wall portion, to cause progressive contraction of the tissue wall portion to influence the movement of bile and/or gallstones in the biliary duct in the direction towards the duodenum.

302. An apparatus according to example 301 where at least two stimulation devices are placed on various locations of the biliary duct, including but not limited to the heptic ducts, the common bile duct, the cystic ducts and their sphincters.

**Claims**

1. An apparatus for influencing a flow in the biliary duct, comprising:

an implantable stimulation device for stimulating muscle tissue of a wall portion of the biliary duct of the patient, comprising an electrical stimulation element configured to engage the muscle tissue; and
a control device for controlling said stimulation device to transmit electrical pulses to the muscle tissue causing contraction of the muscle tissue.

2. The apparatus according to claim 1, wherein the implantable stimulation device comprises a plurality of electrical stimulation elements adapted to be arranged at the wall portion, such that said electrical stimulation elements stimulate different areas of the wall portion.

3. The apparatus according to claim 2, wherein the control device is configured to control the stimulation device to cyclically propagate the stimulation of the different areas of the wall portion in accordance with a determined stimulation pattern.

4. The apparatus according to any one of claims 2 and 3, wherein the control device is configured to control the stimulation device to intermittently stimulate the areas of the wall portion with pulses that form pulse trains.

5. The apparatus according claim 4, wherein the control device is configured to control the stimulation device to vary the amplitudes of the pulses of the pulse trains, the duty cycle of the individual pulses of each pulse train, the width of each pulse of the pulse trains, the length of each pulse train, the repetition frequency of the pulses of the pulse trains, the repetition frequency of the pulse trains, the number of pulses of each pulse train, and/or the off time periods between the pulse trains.

6. The apparatus according to any one of the preceding claims, wherein the electrical elements are electrodes.

7. The apparatus according to any one of claims 2-6, wherein the electrical elements form an elongate pattern of electrical elements, wherein the electrical elements are adapted to be arranged at the tissue wall such that the elongate pattern of electrical elements extends lengthwise along the wall portion of the biliary duct, and the elements abut a respective area of the wall portion.

8. The apparatus according to claim 7, wherein the control device is configured to control the stimulation device to successively energize the electrical elements longitudinally along the elongate pattern of electrical elements in a direction opposite to, or in the same direction as that of, the flow in the biliary duct.

9. The apparatus according to any one of claims 7-8, wherein the control device is adapted to control the stimulation device to successively energize the electrical elements from a position substantially at the center of the wall portion towards both ends of the elongate pattern of electrical elements.

10. The apparatus according to claim 9, wherein the control device is adapted to control the stimulation device to energize the electrical elements, such that energized electrical elements form two waves of energized electrical elements that simultaneously advance from the center of the wall portion in two opposite directions towards both ends of the elongate pattern of electrical elements.

11. The apparatus according to any one of claims 2-6, wherein the electrical elements are adapted to be arranged at least in part around the biliary duct.

12. The apparatus according to claim 11, wherein electrical elements are adapted to be arranged in two paths extending on mutual sides of the biliary duct, preferably substantially transverse to the flow direction in the lumen of the biliary duct.

13. The apparatus according to any of claims 2-12, wherein the stimulation device further comprises a structure for holding the electrical elements in a fixed orientation.

14. The apparatus according to any one of the preceding claims, further comprising at least one implantable sensor for directly or indirectly sense at least one of a physical parameter of the patient, a functional parameter of the apparatus, or a functional parameter of a medical implant in the patient, wherein the control device is configured to control the stimulation device in response to signals from the sensor.

15. The apparatus according to claim 14, wherein the control device further comprises an implantable internal control unit configured to control the stimulation device in response to signals from the sensor.

Fig.1A

Fig.1B

Fig.1C

CSD

BD

Fig.1D

Fig.1E

CSD

BD

CSDE1    CSDE2    CSDE3    Fig. 1F

Fig. 1G

Fig. 1H

EP 3 821 848 A1

Fig. 1I

Fig. 1K

Fig. 1L

EP 3 821 848 A1

Fig.3

Fig.2

Fig.4

Fig. 5A

Fig. 5B

Fig. 5C

Fig. 6A

Fig. 6B

Fig. 6C

Fig. 7A

Fig. 7B

Fig.8A

Fig.8B

Fig.9A

Fig.9B

Fig.10A

Fig.10B

Fig. 11B

4

Fig. 11A

19

21b

20b

21a

20a

Fig. 12A

Fig. 12B

4

30  28  26

8

29

27  7

Fig. 13A

30  28

8

29

Fig. 13B

Fig. 13C

Fig.14

Fig. 15

Fig. 16

Fig. 17

Fig. 20

Fig. 18

Fig. 27

Fig. 19

Fig. 28

Fig. 29

Fig. 21

Fig. 22

Fig. 23

67

64

7

8

XXV                    XXV

Fig. 24

7                        7

64                      64

Fig. 25

Fig. 26

Fig. 30A

Fig. 30B

Fig. 31A

Fig. 31B

Fig. 31C

Fig. 31D

Fig. 32

Fig. 33A

Fig. 33B

97

101

103

100

102

98

99

Fig. 34

101　　100

103

98　　102

Fig. 35A

101

103

100　　98　　102

Fig. 35B

Fig. 36 A

Fig. 36 B

Fig. 36 C

Fig. 36 D

Fig. 36 E

110

112

111

Fig. 37

109    111A    114    110

113

Fig. 38

109  111A    110

113    115

Fig. 39

109  111A    116    110

113

117    118    Fig. 40

109  111A    119    110

121

113

111B    120    Fig. 41

Fig. 42

Fig. 43

Fig. 44

Fig. 45

Fig. 46

Fig. 47

Fig. 48

Fig. 49

Fig. 50

Fig. 51

EP 3 821 848 A1

Fig.52

Fig. 53A

Fig. 53B

Fig. 53C

Fig. 54A

Fig. 54B

Fig. 55A

Fig. 55B

Fig. 55 C

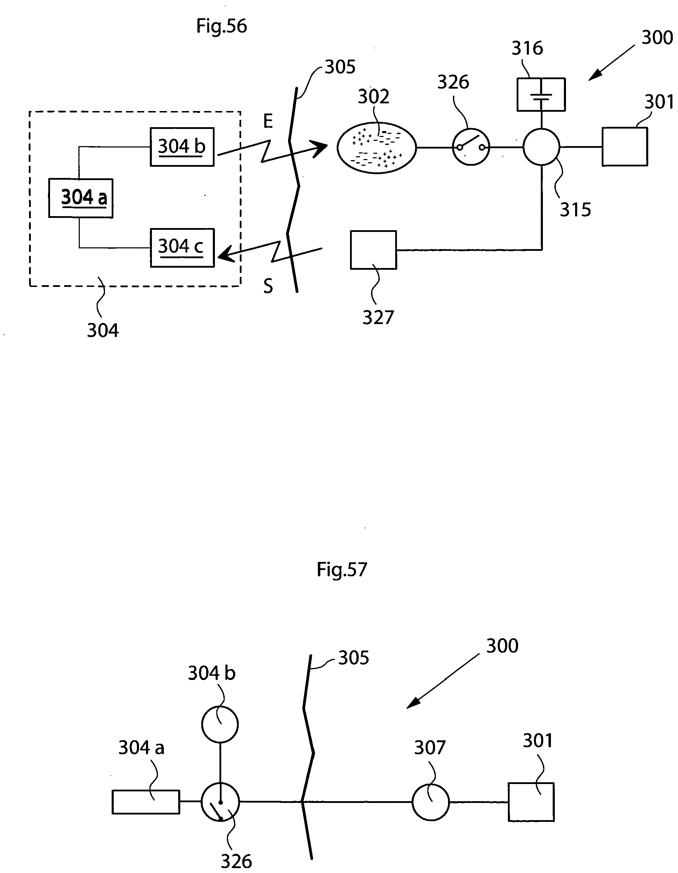

Fig.56

Fig.57

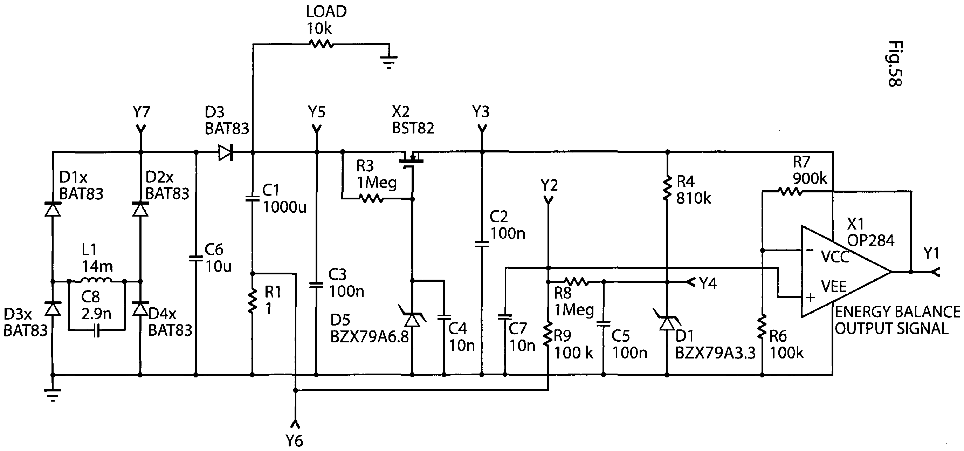

Fig.58

EP 3 821 848 A1

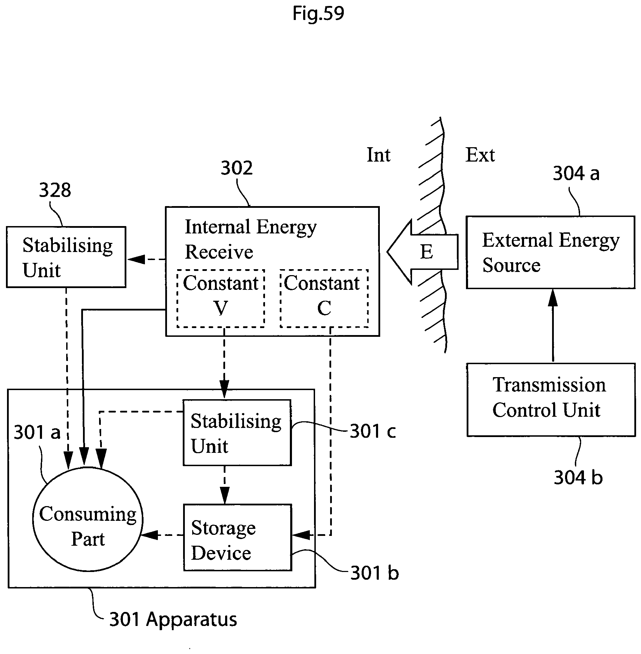

Fig.59

Europäisches
Patentamt

European
Patent Office

Office européen
des brevets

# EUROPEAN SEARCH REPORT

Application Number

EP 20 21 1627

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/106338 A1 (ERRICO JOSEPH P [US]) 10 May 2007 (2007-05-10) | 1-6,11, 13-15 | INV. A61F2/00 |
| Y | * paragraphs [0028], [0030] - [0036], [0064] - [0067], [0079], [0081] - [0084]; figures 2-5 * | 7,8 | A61B17/12 A61F7/02 A61N1/05 A61N1/36 |
| Y | US 2003/055465 A1 (BEN-HAIM SHLOMO [IL] ET AL) 20 March 2003 (2003-03-20) | 7,8 | |
| A | * paragraphs [0071] - [0073], [0251], [0252]; figures 1-4 * | 1-6,9-15 | |
| Y | US 6 238 423 B1 (BARDY GUST H [US]) 29 May 2001 (2001-05-29) | 7,8 | |
| A | * column 11, line 8 - column 12, line 17; figures 1-7 * | 1-6,9-15 | |

TECHNICAL FIELDS
SEARCHED (IPC)

A61F
A61B
A61N

The present search report has been drawn up for all claims

| Place of search | Date of completion of the search | Examiner |
|---|---|---|
| The Hague | 12 April 2021 | Jucker, Chava |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 1627

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2021

| Patent document cited in search report | | | Publication date | Patent family member(s) | | | Publication date |
|---|---|---|---|---|---|---|---|
| US 2007106338 | A1 | | 10-05-2007 | US | 2007106338 | A1 | 10-05-2007 |
| | | | | US | 2010249873 | A1 | 30-09-2010 |
| US 2003055465 | A1 | | 20-03-2003 | AT | 353689 | T | 15-03-2007 |
| | | | | AT | 426430 | T | 15-04-2009 |
| | | | | AU | 3458197 | A | 10-02-1999 |
| | | | | CA | 2296632 | A1 | 28-01-1999 |
| | | | | DE | 69737367 | T2 | 31-10-2007 |
| | | | | EP | 0996482 | A1 | 03-05-2000 |
| | | | | EP | 1779890 | A2 | 02-05-2007 |
| | | | | ES | 2283020 | T3 | 16-10-2007 |
| | | | | JP | 4102545 | B2 | 18-06-2008 |
| | | | | JP | 2001513338 | A | 04-09-2001 |
| | | | | KR | 20010021797 | A | 15-03-2001 |
| | | | | US | 6571127 | B1 | 27-05-2003 |
| | | | | US | 2003055465 | A1 | 20-03-2003 |
| | | | | US | 2003055466 | A1 | 20-03-2003 |
| | | | | US | 2003055467 | A1 | 20-03-2003 |
| | | | | US | 2007185540 | A1 | 09-08-2007 |
| | | | | US | 2008051849 | A1 | 28-02-2008 |
| | | | | US | 2008058889 | A1 | 06-03-2008 |
| | | | | US | 2008058891 | A1 | 06-03-2008 |
| | | | | US | 2014330333 | A1 | 06-11-2014 |
| | | | | WO | 9903533 | A1 | 28-01-1999 |
| | | | | ZA | 976341 | B | 03-02-1998 |
| US 6238423 | B1 | | 29-05-2001 | AU | 5911798 | A | 03-08-1998 |
| | | | | US | 6026326 | A | 15-02-2000 |
| | | | | US | 6238423 | B1 | 29-05-2001 |
| | | | | WO | 9830280 | A1 | 16-07-1998 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4942668 A

- US 5900909 A